(19) Europäisches Patentamt
European Patent Office
Office européen des brevets

(11) **EP 3 283 124 B1**

(12) **EUROPEAN PATENT SPECIFICATION**

(45) Date of publication and mention of the grant of the patent:
**24.04.2024 Bulletin 2024/17**

(21) Application number: **16718838.2**

(22) Date of filing: **15.04.2016**

(51) International Patent Classification (IPC):
***A61K 47/61*** *(2017.01)* ***C07K 14/745*** *(2006.01)*
***A61P 19/04*** *(2006.01)*

(52) Cooperative Patent Classification (CPC):
**A61K 31/727; A61K 47/64; A61K 31/737; A61K 38/14; A61K 38/16; A61K 47/61; A61K 47/62; A61P 1/00; A61P 3/06; A61P 3/10; A61P 7/02; A61P 9/00; A61P 9/10; A61P 9/12; A61P 11/00;** (Cont.)

(86) International application number:
**PCT/US2016/027953**

(87) International publication number:
**WO 2016/168743 (20.10.2016 Gazette 2016/42)**

(54) **BIOCONJUGATES AND USES THEREOF**

BIOKONJUGATE UND VERWENDUNGEN DAVON

BIOCONJUGUÉS ET UTILISATIONS DE CEUX-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **17.04.2015 US 201562149428 P**
**13.10.2015 US 201562241057 P**
**21.10.2015 US 201562244665 P**

(43) Date of publication of application:
**21.02.2018 Bulletin 2018/08**

(73) Proprietor: **Symic Holdings (USA), Inc.**
**San Francisco, CA 94164 (US)**

(72) Inventors:
- **PRESTWICH, Glenn**
  **Emeryville, CA 94608 (US)**
- **PADERI, John, Eric**
  **Emeryville, CA 94608 (US)**
- **CHEN, Julia**
  **Emeryville, CA 94608 (US)**
- **BARTLETT II, Rush, Lloyd**
  **Emeryville, CA 94608 (US)**

(74) Representative: **Beck Greener LLP**
**Fulwood House**
**12 Fulwood Place**
**London WC1V 6HR (GB)**

(56) References cited:
**WO-A1-90/06767** **WO-A1-2007/005491**
**WO-A1-2009/046530** **WO-A1-2010/129547**
**WO-A1-2011/163492** **WO-A1-2014/144969**
**WO-A2-2004/045542** **WO-A2-2012/112767**
**WO-A2-2012/162534**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)
**A61P 13/12; A61P 17/02; A61P 19/00; A61P 19/02; A61P 19/04; A61P 19/08; A61P 27/02; A61P 27/06; A61P 29/00; A61P 31/04; A61P 31/12; A61P 37/02; A61P 43/00**

## Description

Provided herein are bioconjugates comprising a glycan and from 1 to about 50 peptide(s) bound thereto, wherein the peptide(s) comprise a collagen-binding unit, or hyaluronic acid-binding unit, compositions containing the same, and uses thereof.

## Background

In tissues, cells are surrounded by an extracellular matrix (ECM) containing various macromolecules, such as bioconjugates, collagen, hyaluronic acid, laminin, fibronectin, etc. In mammals, bioconjugates are a major component of the extracellular matrix, where they form large complexes, both to other bioconjugates, to hyaluronic acid, and to fibrous matrix proteins (such as collagen). As mammals age and in some disease states, the extracellular matrix in certain areas of the body (e.g., in synovial joints, the vitreous humor, the spinal discs, the skin, etc.) can degrade, causing undesirable symptoms, such as various forms of arthritis, loss of vision, and the like. In addition, some tissue injuries, such as vascular injury, corneal injury and dermal wounds, result in the exposure of the extracellular matrix and /or components thereof, including collagen and hyaluronic acid.

Previously, bioconjugates were synthesized via oxidation chemistry, which cleaved one or more of the saccharide rings within the glycan backbone in order to provide aldehyde functional groups used to conjugate the peptides.

WO2014/144969 discloses a synthetic glycan comprising a glycan, collagen binding peptides and hyaluronic acid binding peptides, wherein the glycan can be dextran,chondroitin, chondroitin sulfate, dermatan, dermatan sulfate, heparan, heparin, keratin, keratan sulfate, or hyaluronic acid. The example bioconjugates of the said document are made by an oxidative method - whereby the glycan is oxidised before peptide coupling.

## Summary

The present disclosure provides bioconjugates comprising a glycan selected from the group consisting of chondroitin sulfate, dermatan sulfate, heparin or a derivative thereof and at least one peptide(s) comprising a collagen-binding unit or a hyaluronic acid-binding unit, covalently bound thereto via a -C(O)-NH-NH-C(O)- (i.e. a hydrazide-carbonyl) linkage. The bioconjugates described herein are structurally different from those known in the art in that the peptides are bound to the glycan via a hydrazide-carbonyl linkage, where a carbonyl group of the hydrazide-carbonyl is an exocyclic carbonyl group present on the glycan. In the bioconjugates disclosed herein, the carbonyl group of the hydrazide-carbonyl is pendant on a saccharide ring within the glycan, such as, but not limited to, D-glucuronate or L-iduronate. It is contemplated that the beneficial effects exhibited by the bioconjugates as disclosed herein (such as increased binding affinity) is at least partially due to the glycan not containing oxidatively cleaved saccharide rings. Another contemplated beneficial effect is improved stability of bioconjugates that do not contain oxidatively cleaved saccharide rings. Accordingly, in certain embodiments, the bioconjugate comprises a glycan, which is a non-oxidized glycan and/or a glycan in which peptides are not conjugated by functional groups created from cleaved saccharides. In certain embodiments, the glycan is a chemically modified glycan derivative, such as a partially N-desulfated glycan derivative, partially O-desulfated glycan derivative, partially O-carboxymethylated glycan derivative, or any combination thereof.

In certain embodiments, the hydrazide-carbonyl linkage is between a terminal hydrazide group on the peptides and a carbonyl group on the glycan. In other embodiments, the hydrazide-carbonyl linkage is between a terminal carbonyl group on the peptides and a hydrazide group on the glycan.

In one embodiment, the present disclosure is directed to a bioconjugate comprising a glycan and from 1 to 50 peptide(s), wherein the peptide(s) comprise a collagen-binding unit and/or a hyaluronic acid-binding unit and are bound to the glycan via a hydrazide-carbonyl linkage.

In one embodiment, the present disclosure is directed to a bioconjugate comprising a glycan and from 1 to 50 peptide(s) comprising a collagen-binding unit and from 1 to 50 peptide(s) comprising a hyaluronic acid-binding unit, and wherein the peptides are bound to the glycan via a hydrazide-carbonyl linkage.

Also provided are compositions comprising the bioconjugates described herein, and their use in methods of treatment. In one aspect, provided are compositions comprising the bioconjugates as described herein, where the number peptides bound to the glycan varies. For example, the composition can comprise bioconjugates where the number of peptides bound thereto is calculated as an average, such as from about 5 to about 20 peptides per glycan.

Also provided herein is a composition comprising one or more bioconjugates selected from the group consisting of a) a bioconjugate comprising a glycan and at least one peptide comprising a hyaluronic acid-binding unit; b) a bioconjugate comprising a glycan and at least one peptide comprising a collagen-binding unit, and combinations thereof.

In certain embodiments, in particular those comprising different peptides having than one type of binding unit (e.g., a bioconjugate comprising peptides having a collagen-binding unit and peptides having a hyaluronic acid-binding

unit), only one may be bound to the glycan via a hydrazide-carbonyl linkage.

Also provided are pharmaceutical compositions comprising the bioconjugates described herein or compositions containing the same and one or more diluent or carrier (such as saline).

The biological function of peptide-functionalized polymers described herein can be tuned by variation of the glycan backbone and/or the peptides attached thereto. For example, the bioconjugates described herein can be used for treating and/or preventing diseases, such as those associated with vascular injury and/or inflammation.

The bioconjugates, and compositions comprising the same, as described herein can be used to treat and/or prevent coronary artery disease and/or peripheral artery disease in a patient in need thereof. Bypass grafts are used as one form of treatment of arterial blockage in both coronary artery disease (CAD) and peripheral artery disease (PAD). Approximately 500,000 coronary artery bypass graft (CABG) procedures and over 70,000 peripheral bypass graft procedures are performed annually in the US. Most commonly, an autologous vessel graft is harvested, often from the saphenous vein. The bioconjugate as described herein can be used as a vein graft preservation solution for patients with cardiovascular disease undergoing surgical bypass with autologous vein grafts.

Also provided herein is a bioconjugate as described herein, or a composition comprising the same for use in a method for treating a blood vessel in a patient prior to, during, and/or after a vascular injury or intervention, comprising applying an effective amount of the bioconjugate, or the composition, to the blood vessel.

Also provided herein is a bioconjugate as described herein, or a composition comprising the same for use in a method for treating stenosis or occlusion within a lumen (e.g., the femoropopliteal artery) in a patient in need thereof, comprising applying a solution to the internal wall of the lumen before, during and/or after a balloon angioplasty, wherein the solution comprises an effective amount of the bioconjugate or the composition.

Also provided is a bioconjugate as described herein, or a composition comprising the same for use in a method for treating arthritis, where the treating can include reducing one or more symptoms associated with arthritis. Various symptoms are known in the art to be associated with arthritis, including but not limited to pain, stiffness, tenderness, inflammation, swelling, redness, warmth, and decreased mobility. In various embodiments, the arthritis is osteoarthritis or rheumatoid arthritis.

Also provided herein is a method for making a bioconjugate comprising a glycan and from 1 to 50 peptide(s), said method comprising contacting the glycan selected from the group consisting of chondroitin sulfate or a derivative thereof, dermatan sulfate or a derivative thereof, and heparin or a derivative thereof with a sufficient amount of peptide, optionally in the presence of an activating agent, wherein the peptide comprises a hydrazide group, under coupling reaction conditions to provide the bioconjugate, wherein the peptide(s) comprise a collagen-binding unit, or a hyaluronic acid-binding unit, and are bound to the glycan via a hydrazide-carbonyl linkage between a terminal hydrazide group on the peptides and a carbonyl group on the glycan, wherein the glycan does not contain oxidatively cleaved saccharide units. In certain embodiments, the method comprises contacting the glycan with an activating agent to provide an activated glycan having at least one activated carboxylic acid moiety. In other embodiments, the hydrazide-carbonyl linkage is between a terminal carbonyl group on the peptides and a hydrazide group on the glycan.

### Brief Description of the Drawings

Certain aspects of the present disclosure can be viewed by the accompanying figures. Included are the following:

> **FIGS. 1A-1D** shows the hyaluronic acid-binding affinity for (1A) biotin-labeled chondroitin sulfate without peptide (control); (1B) bioconjugate having GAH peptide bound to CS via oxidative saccharide ring-opening chemistry and BMPH linker; (1C) bioconjugate having GAH peptide bound to CS via a hydrazide-carbonyl linkage; and (1D) bioconjugate having STM peptide bound to CS via a hydrazide-carbonyl linkage (see Example 5).
>
> **FIG. 2** shows the stability of eDS-SILY (bioconjugate as described herein) versus oxDS-SILY (bioconjugate synthesized utilizing oxidized glycan) over time (See Example 11).

### Detailed Description

It is to be understood that this disclosure is not limited to particular embodiments described, as such may, of course, vary. It is also to be understood that the terminology used herein is for the purpose of describing particular embodiments only, and is not intended to be limiting, since the scope of the present disclosure will be limited only by the appended claims.

### 1. Definitions

Unless defined otherwise, all technical and scientific terms used herein have the same meaning as commonly

understood by one of ordinary skill in the art to which this disclosure belongs. It must be noted that as used herein and in the appended claims, the singular forms "a", "an", and "the" include plural referents unless the context clearly dictates otherwise. Thus, for example, reference to "a peptide" includes a plurality of peptides.

As used herein the following terms and abbreviations have the following meanings.

| | |
|---|---|
| °C | Degrees Celsius |
| μg | Microgram |
| AVF | Arteriovenous fistula |
| BMPH | N-β-maleimidopropionic acid hydrazide |
| CS | Chondroitin sulfate |
| cps | Centipoise |
| DS | Dermatan sulfate |
| DCC | N,N'-dicyclohexylcarbodiimide |
| DIC | N,N'-diisopropylcarbodiimide |
| EDC | 1-ethyl-3-(3-dimethylaminopropyl)carbodiimide |
| BMC | N-t-butyl-N-methylcarbodiimide |
| BEC | N-t-butyl-N-ethylcarbodiimide |
| BDDC | 1,3-bis(2,2-dimethyl-1,3-dioxolan-4-ylmethyl)carbodiimide |
| HFA | hexafluoroacetone |
| CDI | Carbonyldiimidazole |
| HOBt | Hydroxybenzotriazole |
| PyBOP | Benzotriazol-1-yl-oxytripyrrolidinophosphonium hexafluorophosphate |
| HOAt | 1-Hydroxy-7-azabenzotriazole |
| TBTU | O-(Benzotriazol-1-yl)-N,N,N',N'-tetramethyluronium tetrafluoroborate. |
| HOSu | N-hydroxysuccinimide |
| IIDQ | 2-Isobutoxy-1-isobutoxycarbonyl-1,2-dihydroquinoline |
| EEDQ | Ethyl-1,2-dihydro-2-ethoxy-l-quinolinecarboxylate |
| ICAM | Intercellular adhesion molecule |
| VCAM | Vascular cell adhesion molecule |
| Hep | Heparin |
| HA | Hyaluronic acid |
| DNA | Deoxyribonucleic acid |
| EDTA | Ethylenediaminetetraacetic Acid |
| ELISA | Enzyme-Linked Immunosorbent Assay |
| FGF | Fibroblast Growth Factor |
| GAH | GAHWQFNALTVR (SEQ ID NO: 58) |
| HEPES | 2-[4-(2-hydroxyethyl)piperazin-1-yl]ethanesulfonic acid |
| HLB | Hydrophile/Lipophile/Balance |
| ITC | Isothermal Titration Calorimeters |
| kDa | KiloDalton |
| g | Gram |

(continued)

| | |
|---|---|
| GAG | Glycosaminoglycan |
| MES | 2-ethanesulfonic acid |
| mg | Milligram |
| ESRD | End-stage renal disease |
| Da | Daltons |
| μM | Micromolar |
| M | Molar |
| $K_d$ | Dissociation constant |
| vWF | von Willebrand factor |
| MMP | Matrix metalloproteinase enzymes |
| N | Normal |
| MW | Molecular weight |
| CVs | Column volumes |
| min | minutes |
| TMP | Trimethyl phosphate |
| psi | Pounds per square inch |
| μL | Microliters |
| PF-4 | Platelet factor 4 |
| BSA | Bovine serum albumin |
| nm | Nanometers |
| DG | Deionized water |
| PEGDA | Polyethylene glycol diacrylate |
| mL | Milliliter |
| MOPS | 3-(N-morpholino)propanesulfonic acid |
| mV | Millivolt |
| PBS | Phosphate buffered saline |
| PIPES | Piperazine-N,N'-bis(2-ethanesulfonic acid) |
| SILY | RRANAALKAGELYKSILY (SEQ ID NO: 1) |
| SPR | Surface Plasmon Resonance |
| STM | STMMSRSHKTRSHHV (SEQ ID NO: 59) |
| TAPS | 3-[[1,3-dihydroxy-2-(hydroxymethyl)propan-2-yl]amino]propane-1-sulfonic acid |
| TES | 2-[[1,3-dihydroxy-2-(hydroxymethyl)propan-2-yl]amino]ethanesulfonic acid |
| Tris | 2-Amino-2-hydroxymethyl-propane-1,3-diol |
| w/w | Weight/Weight |
| w/v | Weight/Volume |

As used herein, the term "comprising" or "comprises" is intended to mean that the compositions and methods include the recited elements, but not excluding others. "Consisting essentially of" when used to define compositions and methods, shall mean excluding other elements of any essential significance to the combination for the stated purpose. Thus, a composition consisting essentially of the elements as defined herein would not exclude other materials or steps

that do not materially affect the basic and novel characteristic(s) claimed. "Consisting of" shall mean excluding more than trace elements of other ingredients and substantial method steps. Embodiments defined by each of these transition terms are within the scope of this disclosure.

The term "about" when used before a numerical designation, e.g., temperature, time, amount, and concentration, including range, indicates approximations which may vary by (+) or (-) 10%, 5% or 1%.

As used herein, the terms "bioconjugate", "peptidoglycan", and "proteoglycan", and "synthetic proteoglycan" are used interchangeably and refer to a synthetic conjugate that comprises glycan and one or more peptides covalently bonded thereto. The glycan portion can be made synthetically or derived from animal sources. The peptides are covalently bound to the glycan via a hydrazide-carbonyl linkage (i.e., -C(O)-NH-NH-C(O)-). In certain embodiments, the hydrazide-carbonyl linkage is between a terminal hydrazide group on the peptides and a carbonyl group on the glycan. In other embodiments, the hydrazide-carbonyl linkage is between a terminal carbonyl group on the peptides and a hydrazide group on the glycan. In some embodiments, the term bioconjugate includes peptidoglycan.

As used herein, the term "glycan" refers to a compound having a large number of monosaccharides linked glycosidically selected from chondroitin sulfate, dermatan sulfate, or heparin or derivatives thereof. In certain embodiments, the glycan is a glycosaminoglycan (GAG), which comprise 2-aminosugars linked in an alternating fashion with uronic acids, and include polymers such as heparin, heparan sulfate, chondroitin, , and dermatan. Accordingly, examples of glycans which can be used in the embodiments described herein include chondroitin sulfate (CS), dermatan sulfate (DS), , and heparin (Hep including derivatives thereof. In another embodiment the molecular weight of the glycan is varied to tailor the effects of the bioconjugate (see e.g., Radek, K. A., et al., Wound Repair Regen., 2009, 17: 118-126; and Taylor, K. R., et al., J. Biol. Chem., 2005, 280:5300-5306). In one embodiment, the glycan is degraded by oxidation and alkaline elimination (see e.g., Fransson, L. A., et al., Eur. J. Biochem., 1980, 106 :59-69) to afford degraded glycan having a lower molecular weight (e.g., from about 10 kDa to about 50 kDa). In some embodiments, the glycan is unmodified. The glycan does not contain oxidatively cleaved saccharide rings and thus does not, and has not, contain(ed) aldehyde functional groups. In certain embodiments, the glycan is derivatized.

As used herein, the term "derivatized glycan" is intended to include derivatives of glycans. For example, a derivatized glycan can include one or more chemical derivizations, such as, but not limited to partially N-desulfated derivatives, partially O-desulfated derivatives, and/or partially O-carboxymethylated derivatives. For example, as used herein, the term "heparin" is intended to include heparin and derivatives thereof, such as, but not limited to partially N- and/or partially O-desulfated heparin derivatives, partially O-carboxymethylated heparin derivatives, or a combination thereof. In certain embodiments, the heparin is non-oxidized heparin (i.e., does not contain oxidatively cleaved saccharide rings) and does not contain aldehyde functional groups.

As used herein, the terms "bonded" and "covalently bonded" can be used interchangeably and refer to the sharing of one or more pairs of electrons by two atoms.

In certain embodiments, the peptide sequences are bonded to the glycan via a spacer. As used herein, the term "spacer" is intended to refer to an optional portion of the bioconjugate which links the binding unit or peptide to the hydrazide-carbonyl bond. In any of the embodiments described herein, any one or more of the peptides may have a spacer sequence comprising from one to about five amino acids. It is contemplated that any amino acid, natural or unnatural, can be used in the spacer sequence, provided that the spacer sequence does not significantly interfere with the intended binding of the peptide. Exemplary spacers include, but are not limited to, short sequences comprising from one to five glycine units (e.g., G, GG, GGG, GGGG, or GGGGG), optionally comprising cysteine (e.g., GC, GCG, GSGC, or GGC) and/or serine (e.g., GSG, SGG, or GSGSG), or from one to five arginine units (e.g., R, RR, RRR, etc.). The spacer can also comprise non-amino acid moieties, such as polyethylene glycol (PEG), 6-aminohexanoic acid, succinic acid, or combinations thereof, with or without an additional amino acid spacer. In certain embodiments, the peptide sequences described herein further comprise a GSG-$NHNH_2$ moiety. Typically, the GSG-$NHNH_2$ moiety is bound to either the C- or N-terminus.

In one embodiment, the bioconjugates of the disclosure bind, either directly or indirectly to collagen. The terms "binding" or "bind" as used herein are meant to include interactions between molecules that may be detected using, for example, a hybridization assay, surface plasmon resonance, ELISA, competitive binding assays, isothermal titration calorimetry, phage display, affinity chromatography, rheology or immunohistochemistry. The terms are also meant to include "binding" interactions between molecules. Binding may be "direct" or "indirect." "Direct" binding comprises direct physical contact between molecules. "Indirect" binding between molecules comprises the molecules having direct physical contact with one or more molecules simultaneously. This binding can result in the formation of a "complex" comprising the interacting molecules. A "complex" refers to the binding of two or more molecules held together by covalent or non-covalent bonds, interactions or forces.

As used herein, the terms "peptide" and "peptide sequence" are intended to refer to a linear or branched chain of amino acids linked by peptide (or amide) bonds. In one embodiment, the peptide comprises from about 3 to about 120 amino acids, or from about 3 to about 110 amino acids, or from about 3 to about 100 amino acids, or from about 3 to about 90 amino acids, or from about 3 to about 80 amino acids, or from about 3 to about 70 amino acids, or from

about 3 to about 60 amino acids, or from about 3 to about 50 amino acids, or from about 3 to about 40 amino acids, or from about 5 to about 120 amino acids, or from about 5 to about 100 amino acids, or from about 5 to about 90 amino acids, or from about 5 to about 80 amino acids, or from about 5 to about 70 amino acids, or from about 5 to about 60 amino acids, or from about 5 to about 50 amino acids, or from about 5 to about 40 amino acids, or from about 5 to about 30 amino acids, or from about 5 to about 20 amino acids, or from about 5 to about 10 amino acids.

In various embodiments described herein, the peptides (or binding units) can be modified by the inclusion of one or more conservative amino acid substitutions. As is well known to those skilled in the art, altering any non-critical amino acid of a peptide by conservative substitution should not significantly alter the activity of that peptide because the side-chain of the replacement amino acid should be able to form similar bonds and contacts to the side chain of the amino acid which has been replaced. Non-conservative substitutions may too be possible, provided that they do not substantially affect the binding activity of the peptide.

As used herein, the term "sequence identity" refers to a level of amino acid residue or nucleotide identity between two peptides or between two nucleic acid molecules. When a position in the compared sequence is occupied by the same base or amino acid, then the molecules are identical at that position. A peptide (or a polypeptide or peptide region) has a certain percentage (for example, at least about 60%, or at least about 65%, or at least about 70%, or at least about 75%, or at least about 80%, or at least about 83%, or at least about 85%, or at least about 90%, or at least about 95%, or at least about 98% or at least about 99%) of "sequence identity" to another sequence means that, when aligned, that percentage of bases (or amino acids) are the same in comparing the two sequences. It is noted that, for any sequence ("reference sequence") disclosed in this application, sequences having at least about 60%, or at least about 65%, or at least about 70%, or at least about 75%, or at least about 80%, or at least about 83%, or at least about 85%, or at least about 90%, or at least about 95%, or at least about 98% or at least about 99% sequence identity to the reference sequence are also within the disclosure. Likewise, the present disclosure also includes sequences that have one, two, three, four, or five substitution, deletion or addition of amino acid residues or nucleotides as compared to the reference sequences. In certain embodiments, in any one or more of the sequences specified herein, the sequence may be modified by having one, two, or three amino addition, deletion and/or substitution each therefrom.

As used herein, the term "extracellular matrix" refers to the extracellular part of tissue that provides structural and biochemical support to the surrounding cells.

As used herein, the term "composition" refers to a preparation suitable for administration to an intended patient for therapeutic purposes that contains at least one pharmaceutically active ingredient, including any solid form thereof. The composition may include at least one pharmaceutically acceptable component to provide an improved formulation of the compound, such as a suitable carrier. In certain embodiments, the composition is formulated as a film, gel, patch, or liquid solution. As used herein, the term "topically" refers to administering a composition non-systemically to the surface of a tissue and/or organ (internal or, in some cases, external) to be treated, for local effect.

As used herein, the term "pharmaceutically acceptable" indicates that the indicated material does not have properties that would cause a reasonably prudent medical practitioner to avoid administration to a patient, taking into consideration the amount used and/or the disease or conditions to be treated and the respective route of administration. Typical pharmaceutically acceptable materials are essentially sterile.

As used herein, the term "pharmaceutically acceptable carrier" refers to pharmaceutically acceptable materials, compositions or vehicles, such as a liquid or solid filler, diluent, excipient, solvent or encapsulating material, involved in carrying or transporting any supplement or composition, or component thereof, from one organ, or portion of the body, to another organ, or portion of the body, or to deliver an agent to the internal surface of a vein.

As used herein, the term "formulated" or "formulation" refers to the process in which different chemical substances, including one or more pharmaceutically active ingredients, are combined to produce a dosage form. In certain embodiments, two or more pharmaceutically active ingredients can be coformulated into a single dosage form or combined dosage unit, or formulated separately and subsequently combined into a combined dosage unit. A sustained release formulation is a formulation which is designed to slowly release a therapeutic agent in the body over an extended period of time, whereas an immediate release formulation is a formulation which is designed to quickly release a therapeutic agent in the body over a shortened period of time.

As used herein, the term "delivery" refers to routes, approaches, formulations, technologies, and systems for transporting a pharmaceutical composition in the body as needed to safely achieve its desired therapeutic effect. The route of delivery can be any suitable route, including but not limited to, intravascular, intravenous, intraarterial, intramuscular, cutaneous, subcutaneous, percutaneous, intradermal, and intraepidermal routes.

As used herein, the term "solution" refers to solutions, suspensions, emulsions, drops, ointments, liquid wash, sprays, and liposomes, which are well known in the art. In some embodiments, the liquid solution contains an aqueous pH buffering agent which resists changes in pH when small quantities of acid or base are added. In certain embodiments, the liquid solution contains a lubricity enhancing agent.

As used herein, the term "polymer," "polymer matrix" or "polymeric agent" refers to a biocompatible polymeric material. The polymeric material described herein may comprise, for example, sugars (such as mannitol), peptides,

protein, laminin, collagen, hyaluronic acid, ionic and non-ionic water soluble polymers; acrylic acid polymers; hydrophilic polymers such as polyethylene oxides, polyoxyethylene-polyoxypropylene copolymers, and polyvinylalcohol; cellulosic polymers and cellulosic polymer derivatives such as hydroxypropyl cellulose, hydroxyethyl cellulose, hydroxypropyl methylcellulose, hydroxypropyl methylcellulose phthalate, methyl cellulose, carboxymethyl cellulose, and etherified cellulose; poly(lactic acid), poly(glycolic acid), copolymers of lactic and glycolic acids, or other polymeric agents, both natural and synthetic.

In certain embodiments, the polymeric matrix is absorbable, such as, for example collagen, polyglycolic acid, polylactic acid, polydioxanone, and caprolactone. As used herein, the term "absorbable" refers to the ability of a material to be absorbed into the body. In other embodiments, the polymer is non-absorbable, such as, for example polypropylene, polyester or nylon.

As used herein, the term "pH buffering agent" refers to an aqueous buffer solution which resists changes in pH when small quantities of acid or base are added to it. pH Buffering solutions typically comprise a mixture of weak acid and its conjugate base, or vice versa. For example, pH buffering solutions may comprise phosphates such as sodium phosphate, sodium dihydrogen phosphate, sodium dihydrogen phosphate dihydrate, disodium hydrogen phosphate, disodium hydrogen phosphate dodecahydrate, potassium phosphate, potassium dihydrogen phosphate and dipotassium hydrogen phosphate; boric acid and borates such as, sodium borate and potassium borate; citric acid and citrates such as sodium citrate and disodium citrate; acetates such as sodium acetate and potassium acetate; carbonates such as sodium carbonate and sodium hydrogen carbonate, etc. pH Adjusting agents can include, for example, acids such as hydrochloric acid, lactic acid, citric acid, phosphoric acid and acetic acid, and alkaline bases such as sodium hydroxide, potassium hydroxide, sodium carbonate and sodium hydrogen carbonate, etc. In some embodiments, the pH buffering agent is a phosphate buffered saline (PBS) solution (i.e., containing sodium phosphate, sodium chloride and in some formulations, potassium chloride and potassium phosphate).

As used herein, the term "treating" refers to preventing, curing, reversing, attenuating, alleviating, minimizing, inhibiting, suppressing and/or halting one or more clinical symptoms of a disease or disorder prior to, during, and/or after a vascular injury or intervention.

As used herein, the term "concurrently" refers to simultaneous (i.e., in conjunction) administration. In one embodiment, the administration is coadministration such that two or more pharmaceutically active ingredients, including any solid form thereof, are delivered together at one time.

As used herein, the term "sequentially" refers to separate (i.e., at different times) administration. In one embodiment, the administration is staggered such that two or more pharmaceutically active ingredients, including any solid form thereof, are delivered separately at different times.

***Collagen-Binding Peptides***

"Collagen-binding peptides" are peptides comprising 1 to about 120 amino acids having one or more collagen-binding units (or sequences). As used herein, the term "collagen-binding unit" is intended to refer to an amino acid sequence within a peptide which binds to collagen. "Collagen-binding" indicates an interaction with collagen that could include hydrophobic, ionic (charge), and/or Van der Waals interactions, such that the compound binds or interacts favorably with collagen. This binding (or interaction) is intended to be differentiated from covalent bonds and nonspecific interactions with common functional groups, such that the peptide would interact with any species containing that functional group to which the peptide binds on the collagen. Peptides can be tested and assessed for binding to collagen using any method known in the art. See, e.g., Li, Y., et al., Current Opinion in Chemical Biology, 2013, 17: 968-975, Helmes, B.A., et al., J. Am. Chem. Soc. 2009, 131, 11683-11685, and Petsalaki, E., et al., PLoS Comput Biol, 2009, 5(3): e1000335. In one embodiment, the peptide, or the collagen-binding unit of the peptide, binds to collagen with a dissociation constant ($K_d$) of less than about 1 mM, or less than about 900 μM, or less than about 800 μM, or less than about 700 μM, or less than about 600 μM, or less than about 500 μM, or less than about 400 μM, or less than about 300 μM, or less than about 200 μM, or less than about 100 μM.

The peptide can have amino acid homology with a portion of a protein normally or not normally involved in collagen fibrillogenesis. In some embodiments, these peptides have homology or sequence identity to the amino acid sequence of a small leucine-rich bioconjugate, a platelet receptor sequence, or a protein that regulates collagen fibrillogenesis. In various embodiments, the peptide comprises an amino acid sequence selected from RRANAALKAGELYKSILY (SEQ ID NO: 1), GELYKSILY (SEQ ID NO: 2), RRANAALKAGELYKCILY (SEQ ID NO: 3), GELYKCILY (SEQ ID NO: 4), RLDGNEIKR (SEQ ID NO: 5), AHEEISTTNEGVM (SEQ ID NO: 6), NGVFKYRPRYFLYKHAYFYPPLKRFPVQ (SEQ ID NO: 7), CQDSETRTFY (SEQ ID NO: 8), TKKTLRT (SEQ ID NO: 9), GLRSKSKKFRRPDIQYPDATDEDITSHM (SEQ ID NO: 10), SQNPVQP (SEQ ID NO: 11), SYIRIADTNIT (SEQ ID NO: 12), KELNLVYT (SEQ ID NO: 13), GSIT (SEQ ID NO: 14), GSITTIDVPWNV (SEQ ID NO: 15), GQLYKSILY (SEQ ID NO: 16), RRANAALKAGQLYKSILY (SEQ ID NO: 17), or a sequence having at least about 80% sequence identity, or at least about 83% sequence identity, or at least about 85% sequence identity, or at least about 90% sequence identity, or at least about 95% sequence identity,

or at least about 98% sequence identity thereto, provided the sequence is capable of binding to collagen.

In certain embodiments, the peptide comprises an amino acid sequence that has at least about 80%, or at least about 83%, or at least about 85%, or at least about 90%, or at least about 95%, or at least about 98%, or at least about 100% sequence identity with the collagen-binding domain(s) of the von Willebrand factor (vWF) or a platelet collagen receptor as described in Chiang, T.M., et al. J. Biol. Chem., 2002, 277: 34896-34901, Huizinga, E.G. et al., Structure, 1997, 5: 1147-1156, Romijn, R.A., et al., J. Biol. Chem., 2003, 278: 15035-15039, and Chiang, et al., Cardio. & Haemato. Disorders-Drug Targets, 2007, 7: 71-75. A non-limiting example is WREPSFCALS (SEQ ID NO: 18), derived from vWF.

Various methods for screening peptide sequences for collagen-binding affinity (or a collagen-binding domain/unit) are routine in the art. Other peptide sequences shown to have collagen-binding affinity (or a collagen-binding unit) which can be used in the bioconjugates and methods disclosed herein include but are not limited to, βAWHCTTKFPHHYCLYBip (SEQ ID NO: 19), βAHKCPWLYTTHYCFTBip (SEQ ID NO: 20), βAHKCPWLYTHYCFT (SEQ ID NO: 21), etc., where Bip is biphenylalanine and βA is beta-alanine (see, Abd-Elgaliel, W.R., et al., Biopolymers, 2013, 100(2), 167-173), GROGER (SEQ ID NO: 22), GMOGER (SEQ ID NO: 23), GLOGEN (SEQ ID NO: 24), GLOGER (SEQ ID NO: 25), GLKGEN (SEQ ID NO: 26), GFOGERGVEGPOGPA (SEQ ID NO: 27), etc., where O is 4-hydroxyproline (see, Raynal, N., et al., J. Biol. Chem., 2006, 281(7), 3821-3831), HVWMQAPGGGK (SEQ ID NO: 28) (see, Helms, B.A., et al., J. Am. Chem. Soc. 2009, 131, 11683-11685), WREPSFCALS (SEQ ID NO: 18) (see, Takagi, J., et al., Biochemistry, 1992, 31, 8530-8534), WYRGRL (SEQ ID NO: 29), etc. (see, Rothenfluh D.A., et al., Nat Mater. 2008, 7(3), 248-54), WTCSGDEYTWHC (SEQ ID NO: 30), WTCVGDHKTWKC (SEQ ID NO: 31), QWHCTTRFPHHYCLYG (SEQ ID NO: 32), etc. (see, U.S. 2007/0293656), STWTWNGSAWTWNEGGK (SEQ ID NO: 33), STWTWNGTNWTRNDGGK (SEQ ID NO: 34), etc. (see, WO/2014/059530), CVWLWEQC (SEQ ID NO: 35) cyclic CVWLWENC (SEQ ID NO: 36), cyclic CVWLWEQC (SEQ ID NO: 35), (see, Depraetere H., et al., Blood. 1998, 92, 4207-4211, and Duncan R., Nat Rev Drug Discov, 2003, 2(5), 347-360), CMTSPWRC (SEQ ID NO: 37), etc. (see, Vanhoorelbeke, K., et al., J. Biol. Chem., 2003, 278, 37815-37821), CPGRVMHGLHLGDDEGPC (SEQ ID NO: 38) (see, Muzzard, J., et al., PLoS one. 4 (e 5585) I-10), KLWLLPK (SEQ ID NO: 39) (see, Chan, J. M., et al., Proc Natl Acad Sci U.S.A., 2010, 107, 2213- 2218), and CQDSETRTFY (SEQ ID NO: 8), etc. (see, U.S. 2013/0243700), H-V-F/W-Q/ M-Q-P/A-P/K (Helms, B.A., et al., J. Am. Chem. Soc., 2009, 131(33), 11683-11685).

Additional peptide sequences shown to have collagen-binding affinity (or a collagen-binding unit) which can be used in the bioconjugates and methods disclosed herein include but are not limited to, LSELRLHEN (SEQ ID NO: 40), LTELHLDNN (SEQ ID NO: 41), LSELRLHNN (SEQ ID NO: 42), LSELRLHAN (SEQ ID NO: 43), and LRELHLNNN (SEQ ID NO: 44) (see, Fredrico, S., Angew. Chem. Int. Ed. 2015, 37, 10980-10984).

In certain embodiments, the peptides include one or more sequences selected from the group consisting of RVMHGLHLGDDE (SEQ ID NO: 45), D-amino acid EDDGLHLGHMVR (SEQ ID NO: 46), RVMHGLHLGNNQ (SEQ ID NO: 47), D-amino acid QNNGLHLGHMVR (SEQ ID NO: 48), RVMHGLHLGNNQ (SEQ ID NO: 47), GQLYKSILYGSG-4K2K (SEQ ID NO: 49) (a 4-branch peptide), GSGQLYKSILY (SEQ ID NO: 50), GSGGQLYKSILY (SEQ ID NO: 51), KQLNLVYT (SEQ ID NO: 52), KELNVYT (SEQ ID NO: 53), CVWLWQQC (SEQ ID NO: 54), WREPSFSALS (SEQ ID NO: 55), GHRPLDKKREEAPSLRPAPPPISGGGYR (SEQ ID NO: 56), and GHRPLNKKRQQAPSLRPAPPPISGGGYR (SEQ ID NO: 57).

Similarly for a collagen-binding peptide, a peptide derived from a phage display library selected for collagen can be generated. The peptide can be synthesized and evaluated for binding to collagen by any of the techniques such as SPR, ELISA, ITC, affinity chromatography, or others known in the art. An example could be a biotin modified peptide sequence (e.g., SIL $Y_{biotin}$) that is incubated on a microplate containing immobilized collagen. A dose response binding curve can be generated using a streptavidin-chromophore to determine the ability of the peptide to bind to collagen.

In one embodiment, the peptides comprise one or more collagen-binding units which binds any one or more of collagen type I, II, III, IV, V, VI, VII, VIII, IX, X, XI, XII, XIII, or XIV. In one embodiment, the peptide binds to type I collagen with a dissociation constant ($K_d$) of less than about 1 mM, or less than about 900 μM, or less than about 800 μM, or less than about 700 μM, or less than about 600 μM, or less than about 500 μM, or less than about 400 μM, or less than about 300 μM, or less than about 200 μM, or less than about 100 μM. In one embodiment, the peptide binds to type II collagen with a dissociation constant ($K_d$) of less than about 1 mM, or less than about 900 μM, or less than about 800 μM, or less than about 700 μM, or less than about 600 μM, or less than about 500 μM, or less than about 400 μM, or less than about 300 μM, or less than about 200 μM, or less than about 100 μM. In one embodiment, the peptide binds to type III collagen with a dissociation constant ($K_d$) of less than about 1 mM, or less than about 900 μM, or less than about 800 μM, or less than about 700 μM, or less than about 600 μM, or less than about 500 μM, or less than about 400 μM, or less than about 300 μM, or less than about 200 μM, or less than about 100 μM. In one embodiment, the peptide binds to type IV collagen with a dissociation constant ($K_d$) of less than about 1 mM, or less than about 900 μM, or less than about 800 μM, or less than about 700 μM, or less than about 600 μM, or less than about 500 μM, or less than about 400 μM, or less than about 300 μM, or less than about 200 μM, or less than about 100 μM.

### *Hyaluronic Acid-Binding Peptides*

"Hyaluronic acid-binding peptides" are peptides comprising 1 to about 120 amino acids having one or more collagen-binding units (or sequences). As used herein, "hyaluronic acid-binding unit" is intended to refer to an amino acid sequence within a peptide which binds to hyaluronic acid. "Hyaluronic acid-binding" indicates an interaction with hyaluronic acid that could include hydrophobic, ionic (charge), and/or Van der Waals interactions, such that the compound binds or interacts favorably with hyaluronic acid. This binding (or interaction) is intended to be differentiated from covalent bonds and nonspecific interactions with common functional groups, such that the hyaluronic acid-binding peptide would interact with any species containing that functional group to which the peptide binds on the hyaluronic acid. See, e.g., Becerra, S.P., et al. J. Biol. Chem., 2008, 283: 33310-33320. In one embodiment, the peptide, or the hyaluronic acid-binding unit, binds to hyaluronic acid with a dissociation constant ($K_d$) of less than about 1 mM, or less than about 900 μM, or less than about 800 μM, or less than about 700 μM, or less than about 600 μM, or less than about 500 μM, or less than about 400 μM, or less than about 300 μM, or less than about 200 μM, or less than about 100 μM.

In certain embodiments, the hyaluronic acid-binding unit of the synthetic bioconjugate can comprise an amino acid sequence derived from hyaluronan-mediated motility receptor (RHAMM) (exemplary sequences include, but are not limited to, NP_001136028, NP_001136029, NP_036616, and NP_036617).

In the various embodiments described herein, the hyaluronic acid-binding peptide component of the synthetic bioconjugate can comprise an amino acid sequence selected from GAHWQFNALTVR (SEQ ID NO: 58), STMMSRSHKTRSHHV (SEQ ID NO: 59), TMTRPHFHKRQLVLS (SEQ ID NO: 60), STMMSRSHKTRSCHH (SEQ ID NO: 61), STMMSRSHKTRSHH (SEQ ID NO: 62), GDRRRRRMWHRQ (SEQ ID NO: 63), GKBLGGKHRRSR (SEQ ID NO: 64), RGTHHAQKRRS (SEQ ID NO: 65), RRHKSGHIQGSK (SEQ ID NO: 66), SRMHGRVRGRHE (SEQ ID NO: 67), RRRAGLTAGRPR (SEQ ID NO: 68), RYGGHRTSRKWV (SEQ ID NO: 69), RSARYGHRRGVG (SEQ ID NO: 70), GLRGNRRVFARP (SEQ ID NO: 71), SRGQRGRLGKTR (SEQ ID NO: 72), DRRGRSSLPKLAGPVEFPDRKIKGRR (SEQ ID NO: 73), AGPVEFPDRKIKGRR (SEQ ID NO: 74), RMRRKGRVKHWG (SEQ ID NO: 75), RGGARGRHKTGR (SEQ ID NO: 76), TGARQRGLQGGWGPRHLRGKDQPPGR (SEQ ID NO: 77), RQRRRDLTRVEG (SEQ ID NO: 78), STKDHNRGRRNVGPVSRSTLRDPIRR (SEQ ID NO: 79), RRIGHQVGGRRN (SEQ ID NO: 80), RLESRAAGQRRA (SEQ ID NO: 81), GGPRRHLGRRGH (SEQ ID NO: 82), VSKRGHRRTAHE (SEQ ID NO: 83), RGTRSGSTR (SEQ ID NO: 84), RRRKKIQGRSKR (SEQ ID NO: 85), RKSYGKYQGR (SEQ ID NO: 86), KNGRYSISR (SEQ ID NO: 87), RRRCGQKKK (SEQ ID NO: 88), KQKIKHVVKLK (SEQ ID NO: 89), KLKSQLVKRK (SEQ ID NO: 90), RYPISRPRKR (SEQ ID NO: 91), KVGKSPPVR (SEQ ID NO: 92), KGRYSISR (SEQ ID NO: 93), RRRCGQKK (SEQ ID NO: 94), KTFGKMKPR (SEQ ID NO: 95), RIKWSRVSK (SEQ ID NO: 96) and KRTMRPTRR (SEQ ID NO: 97), or a sequence having at least about 80% sequence identity, or at least about 83% sequence identity, or at least about 85% sequence identity, or at least about 90% sequence identity, or at least about 95% sequence identity, or at least about 98% sequence identity thereto, provided the sequence is capable of binding to hyaluronic acid.

Additional peptides that can be included as the peptide component of the hyaluronic acid-binding synthetic bioconjugate include peptides which have an Arg-Arg (R-R) motif, such as one or more peptides selected from RRASRSRGQVGL (SEQ ID NO: 98), GRGTHHAQKRRS (SEQ ID NO: 99), QPVRRLGTPVVG (SEQ ID NO: 100), ARRAEGKTRMLQ (SEQ ID NO: 101), PKVRGRRHQASG (SEQ ID NO: 102), SDRHRRRREADG (SEQ ID NO: 103), NQRVRRVKHPPG (SEQ ID NO: 104), RERRERHAVARHGPGLERDARNLARR (SEQ ID NO: 105), TVRPGGKRGGQVGPPAGVLHGRRARS (SEQ ID NO: 106), NVRSRRGHRMNS (SEQ ID NO: 107), DRRRGRTRNIGN (SEQ ID NO: 108), KTAGHGRRWSRN (SEQ ID NO: 109), AKRGEGRREWPR (SEQ ID NO: 110), GGDRRKAHKLQA (SEQ ID NO: 111), RRGGRKWGSFEG (SEQ ID NO: 112), and RQRRRDLTRVEG (SEQ ID NO: 78) (see, e.g., Amemiya et al., Biochem. Biophys. Acta, 2005, 1724, 94-99,); or a sequence having at least about 80% sequence identity, or at least about 83% sequence identity, or at least about 85% sequence identity, or at least about 90% sequence identity, or at least about 95% sequence identity, or at least about 98% sequence identity thereto, provided the sequence is capable of binding to hyaluronic acid. In another embodiment, the peptide is selected from RDGTRYVQKGEYR (SEQ ID NO: 113), HREARSGKYK (SEQ ID NO: 114), PDKKHKLYGV (SEQ ID NO: 115), and WDKERSRYDV (SEQ ID NO: 116) (see, e.g., Yang, B., et al, EMBO Journal, 1994, 13, 286-296, and Goetinck, P.F. et al, J. Cell. Biol, 1987, 105, 2403-2408,); or a sequence having at least about 80% sequence identity, or at least about 83% sequence identity, or at least about 85% sequence identity, or at least about 90% sequence identity, or at least about 95% sequence identity, or at least about 98% sequence identity thereto, provided the sequence is capable of binding to hyaluronic acid.

Peptides may also be selected by phage display, utilizing positive selection for binding to hyaluronic acid. A hyaluronic acid-binding peptide may be determined by its interaction with hyaluronic acid, and measured by any of the techniques used to evaluate molecular interactions (such as surface plasmon resonance, ELISA, competitive binding assays, isothermal titration calorimetry, affinity chromatography, rheology and/or immunohistochemistry). Peptides that are considered "hyaluronic acid-binding" may interact with hyaluronic acid or hyaluronic acid-containing tissues such that the interaction is not attributed to known chemically reactive groups. The interaction may be due to hydrophobic and charge interactions resulting from the amino acid residues in the peptide. The interaction may be measured by

immobilizing hyaluronic acid on a microplate and incubating with hyaluronic acid-binding peptides followed by detection techniques such as biotin-avidin with the use of a chromophore. The interaction may also be measured by mechanical influence on hyaluronic acid-containing fluids, gels, or tissues that have been incubated with the hyaluronic acid-binding peptide or with a synthetic bioconjugate containing an hyaluronic acid-binding peptide or peptides.

For identifying a peptide, a peptide selected from phage display, or one that is identified from a hyaluronic acid-binding motif in a protein, can be synthesized and evaluated for its interaction with hyaluronic acid. For example, a B-X7-B sequence could be synthesized with a biotin modification at the N-terminus and incubated on a hyaluronic acid coated microplate. A dose response binding curve can be generated to determine the ability of the peptide to bind to hyaluronic acid. In one embodiment, the peptide comprises from about 3 to about 120 amino acids, or from about 3 to about 110 amino acids, or from about 3 to about 100 amino acids, or from about 3 to about 90 amino acids, or from about 3 to about 80 amino acids, or from about 3 to about 70 amino acids, or from about 3 to about 60 amino acids, or from about 3 to about 50 amino acids, or from about 3 to about 40 amino acids, or from about 5 to about 120 amino acids, or from about 5 to about 100 amino acids, or from about 5 to about 90 amino acids, or from about 5 to about 80 amino acids, or from about 5 to about 70 amino acids, or from about 5 to about 60 amino acids, or from about 5 to about 50 amino acids, or from about 5 to about 40 amino acids, or from about 5 to about 30 amino acids, or from about 5 to about 20 amino acids, or from about 5 to about 10 amino acids.

In certain embodiments, any sequence described herein may be modified such that any one or more amino acids (e.g., 1, 2, 3, 4 or 5 amino acids) are added, deleted or substituted therefrom. In some embodiments, the sequence is modified such that any one or more amino acids is replaced by alanine. In some embodiments, the sequence is modified such that any one or more l-amino acid is replaced the corresponding d-amino acid scan. In some embodiments, the sequence is modified such that any one or more valine is replaced by leucine, any one or more glutamic acid is replaced by glutamine, any one or more aspartic acid is replaced by asparagine, and/or any one or more arginine is replaced by glutamine.

In any of the embodiments described herein, the peptide having a collagen-binding unit, or a hyaluronic acid-binding unit, , comprises any amino acid sequence described in the preceding paragraphs or an amino acid sequence having at least about 80%, or at least about 83%, or at least about 85%, or at least about 90%, or at least about 95%, or at least about 98%, or at least about 100% homology to any of these amino acid sequences. In various embodiments, the peptide components of the synthetic bioconjugates described herein can be modified by the inclusion of one or more conservative amino acid substitutions. As is well-known to those skilled in the art, altering any non-critical amino acid of a peptide by conservative substitution should not significantly alter the activity of that peptide because the side-chain of the replacement amino acid should be able to form similar bonds and contacts to the side chain of the amino acid which has been replaced.

As is well-known in the art, a "conservative substitution" of an amino acid or a "conservative substitution variant" of a peptide refers to an amino acid substitution which maintains: 1) the secondary structure of the peptide; 2) the charge or hydrophobicity of the amino acid; and 3) the bulkiness of the side chain or any one or more of these characteristics. Illustratively, the well-known terminologies "hydrophilic residues" relate to serine or threonine. "Hydrophobic residues" refer to leucine, isoleucine, phenylalanine, valine or alanine, or the like. "Positively charged residues" relate to lysine, arginine, ornithine, or histidine. "Negatively charged residues" refer to aspartic acid or glutamic acid. Residues having "bulky side chains" refer to phenylalanine, tryptophan or tyrosine, or the like. A list of illustrative conservative amino acid substitutions is given in **Table 1.**

**Table 1**

| For Amino Acid | Replace With |
|---|---|
| Alanine | D-Ala, Gly, Aib, β-Ala, L-Cys, D-Cys |
| Arginine | D-Arg, Lys, D-Lys, Orn, D-Orn |
| Asparagine | D-Asn, Asp, D-Asp, Glu, D-Glu, Gln, D-Gln |
| Aspartic Acid | D-Asp, D-Asn, Asn, Glu, D-Glu, Gln, D-Gln |
| Cysteine | D-Cys, S-Me-Cys, Met, D-Met, Thr, D-Thr, L-Ser, D-Ser |
| Glutamine | D-Gln, Asn, D-Asn, Glu, D-Glu, Asp, D-Asp |
| Glutamic Acid | D-Glu, D-Asp, Asp, Asn, D-Asn, Gln, D-Gln |
| Glycine | Ala, D-Ala, Pro, D-Pro, Aib, β-Ala |
| Isoleucine | D-Ile, Val, D-Val, Leu, D-Leu, Met, D-Met |
| Leucine | Val, D-Val, Met, D-Met, D-Ile, D-Leu, Ile |

(continued)

| For Amino Acid | Replace With |
|---|---|
| Lysine | D-Lys, Arg, D-Arg, Orn, D-Orn |
| Methionine | D-Met, S-Me-Cys, Ile, D-Ile, Leu, D-Leu, Val, D-Val |
| Phenylalanine | D-Phe, Tyr, D-Tyr, His, D-His, Trp, D-Trp |
| Proline | D-Pro |
| Serine | D-Ser, Thr, D-Thr, allo-Thr, L-Cys, D-Cys |
| Threonine | D-Thr, Ser, D-Ser, allo-Thr, Met, D-Met, Val, D-Val |
| Tyrosine | D-Tyr, Phe, D-Phe, His, D-His, Trp, D-Trp |
| Valine | D-Val, Leu, D-Leu, Ile, D-Ile, Met, D-Met |

In some embodiments, the peptide sequences are modified to replace one or more glutamic acid residue(s) with glutamine and/or one or more aspartic acid residue(s) with asparagine.

## 2. Bioconjugates

Provided herein are bioconjugates comprising a glycan and from 1 to 50 peptide(s) comprising a collagen-binding unit and/or a hyaluronic acid-binding unit, covalently bound thereto via a -C(O)-NH-NH-C(O)- (i.e. a hydrazide-carbonyl) linkage.

Previously, peptides were bound to glycans, such as dermatan sulfate, by utilizing oxidation chemistry to cleave one or more of the saccharide ring within the glycan backbone in order to provide aldehyde binding sites on the glycan. The aldehyde binding sites were then used to conjugate the peptides (e.g., via a -C(O)-NH-N=C bond).

The bioconjugates described herein are structurally different from those known in the art in that the peptides are bound to the glycan via a hydrazide-carbonyl linkage, where a carbonyl group of the hydrazide-carbonyl is an exocyclic carbonyl group present on the glycan. The exocyclic carbonyl group may be present on the native glycan, or alternatively, the glycan can be modified to include such a functional group. Such methods are further detailed below. It is contemplated that the beneficial effects exhibited by the bioconjugates as disclosed herein (such as increased binding affinity) is at least partially due to the glycan not containing oxidatively cleaved saccharide rings.

In certain embodiments, the bioconjugate can comprise a polymer backbone (e.g., a biocompatible polymer other than glycan), comprised of any single or combination of monomeric units, provided there are at least one, and in some instances, between 1 and about 50, suitable functional groups present thereon, such that the peptide(s) as described herein can be covalently bound thereto. The polymer can be linear, branched, or can contain side chains (e.g., other than the 1 to 50 peptides). The polymers can be neutral, cationic, anionic, or Zwitterionic. In certain embodiments, the polymer is a glycopolymer. The polymer can be a copolymer, including a block copolymer of the formula A-B-A, for example. Methods for providing such polymers are known in the art, and include for example, living polymerizations. In one embodiment, the polymer is a poly(ethylene glycol) (PEG). In another embodiment, the polymer is not a poly(ethylene glycol) (PEG). In certain embodiments, the polymer is not a glycan or a nanoparticle. In certain embodiments, the polymer is a glycan.

In certain embodiments of the bioconjugates described herein, the glycan can be chondroitin sulfate , dermatan sulfate, or, heparinIn one embodiment, the glycan is dermatan sulfate. In one embodiment, the glycan is not dermatan sulfate. In another embodiment, the glycan is chondroitin sulfate. In another embodiment, the glycan is heparin. Various molecular weights for the heparin can be used in the bioconjugates described herein, such as from a single disaccharide unit of about 650-700 Da, to a glycan of about 50 kDa. In some embodiments, the heparin is from about 10 to about 20 kDa. In some embodiments, the heparin is up to about 15, or about 16, or about 17, or about 18, or about 19, or about 20 kDa.

In one embodiment, the bioconjugate comprises a peptide having a collagen-binding unit which binds to one or more of collagen type I, II, III, IV, V, VI, VII, VIII, IX, X, XI, XII, XIII, or XIV. In one embodiment, the collagen-binding unit promotes or inhibits fibrillogenesis upon binding to collagen. In one embodiment, the collagen-binding unit does not promote or inhibit fibrillogenesis upon binding to collagen. In some embodiments, the peptide binds to type I collagen. In other embodiments, the peptide binds to type IV collagen. In certain embodiments, one or more peptide(s) having a specified binding affinity for collagen can be used in the bioconjugates described herein. For example, the synthetic bioconjugates can comprise at least one peptide which has binding affinity to type I collagen and at least one peptide which has binding affinity to type IV collagen. In another embodiment, the peptides have binding affinity to type I collagen.

In another embodiment, the peptides have binding affinity to type IV collagen. In certain embodiments, the peptides have binding affinity to type II collagen. In certain embodiments, the peptides have binding affinity to type III collagen. In certain embodiments, the peptide binds to more than one type of collagen, where the relative affinity to each collagen type may vary. In one embodiment, the collagen-binding unit binds to collagen with a dissociation constant ($K_d$) of less than about 1 mM, or less than about 900 μM, or less than about 800 μM, or less than about 700 μM, or less than about 600 μM, or less than about 500 μM, or less than about 400 μM, or less than about 300 μM, or less than about 200 μM, or less than about 100 μM.

Further, the bioconjugates described herein may comprise peptides with more than one binding unit, where the binding unit can be the same or different. For example, in certain embodiments, the peptide comprises two or more collagen-binding units, where the collagen-binding units are the same. In another embodiment, the peptide comprises two or more collagen-binding units, where the collagen-binding units are different.

In certain embodiments, the bioconjugate comprises one or more peptides comprising an amino acid sequence of formula (I) or (II):

$$(X)_n\text{-YKS-}(X)_m \qquad \text{(I)}$$

$$(X)_n\text{-YKC-}(X)_m \qquad \text{(II)}$$

wherein

n is from 0 to 50;

m is from 0 to 50;

and each X is independently selected from a natural or an unnatural amino acid. In certain embodiments, each X is independently selected from the group consisting of alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, and valine.

In certain embodiments, the bioconjugate comprises one or more peptides comprising an amino acid sequence selected from the group consisting of YKSILY (SEQ ID NO: 179), LYKSILY (SEQ ID NO: 180), ELYKSILY (SEQ ID NO: 181), GELYKSILY (SEQ ID NO: 2), AGELYKSILY (SEQ ID NO: 182), KAGELYKSILY (SEQ ID NO: 183), LKAGELYKSILY (SEQ ID NO: 184), ALKAGELYKSILY (SEQ ID NO: 185), AALKAGELYKSILY (SEQ ID NO: 186), NAALKAGELYKSILY (SEQ ID NO: 187), ANAALKAGELYKSILY (SEQ ID NO: 188), RANAALKAGELYKSILY (SEQ ID NO: 189), RRANAALKAGELYKSILY (SEQ ID NO: 1), QLYKSILY (SEQ ID NO: 190), GQLYKSILY (SEQ ID NO: 16), AGQLYKSILY (SEQ ID NO: 191), KAGQLYKSILY (SEQ ID NO: 192), LKAGQLYKSILY (SEQ ID NO: 193), ALKAGQLYKSILY (SEQ ID NO: 194), AALKAGQLYKSILY (SEQ ID NO: 195), NAALKAGQLYKSILY (SEQ ID NO: 196), ANAALKAGQLYKSILY (SEQ ID NO: 197), RANAALKAGQLYKSILY (SEQ ID NO: 198), and RRANAALKAGQLYKSILY (SEQ ID NO: 17), or a sequence having at least about 80% sequence identity, or at least about 83% sequence identity, or at least about 85% sequence identity, or at least about 90% sequence identity, or at least about 95% sequence identity, or at least about 98% sequence identity thereto, provided that the sequence comprises at least one YKS sequence.

In certain embodiments, the bioconjugate comprises one or more peptides comprising an amino acid sequence selected from the group consisting of YKCILY (SEQ ID NO: 199), LYKCILY (SEQ ID NO: 200), ELYKCILY (SEQ ID NO: 201), GELYKCILY (SEQ ID NO: 4), AGELYKCILY (SEQ ID NO: 202), KAGELYKCILY (SEQ ID NO: 203), LKAGEL YKCIL Y (SEQ ID NO: 204), ALKAGELYKCILY (SEQ ID NO: 205), AALKAGELYKCILY (SEQ ID NO: 206), NAALKAGELYKCILY (SEQ ID NO: 207), ANAALKAGELYKCILY (SEQ ID NO: 208), RANAALKAGELYKCILY (SEQ ID NO: 209), RRANAALKAGELYKCILY (SEQ ID NO: 3), QLYKCILY (SEQ ID NO: 210), GQLYKCILY (SEQ ID NO: 211), AGQL YKCIL Y (SEQ ID NO: 212), KAGQL YKCIL Y (SEQ ID NO: 213), LKAGQL YKCIL Y (SEQ ID NO: 214), ALKAGQL YKCIL Y (SEQ ID NO: 215), AALKAGQLYKCILY (SEQ ID NO: 216), NAALKAGQL YKCIL Y (SEQ ID NO: 217), ANAALKAGQLYKCILY (SEQ ID NO: 218), RANAALKAGQLYKCILY (SEQ ID NO: 219), and RRANAALKAGQLYKCILY (SEQ ID NO: 220), or a sequence having at least about 80% sequence identity, or at least about 83% sequence identity, or at least about 85% sequence identity, or at least about 90% sequence identity, or at least about 95% sequence identity, or at least about 98% sequence identity thereto, provided that the sequence comprises at least one YKC sequence.

In certain embodiments, the bioconjugate comprises one or more peptides comprising an amino acid sequence selected from the group consisting of LYKS (SEQ ID NO: 221), ELYKS (SEQ ID NO: 222), GELYKS (SEQ ID NO: 223), AGELYKS (SEQ ID NO: 224), KAGELYKS (SEQ ID NO: 225), LKAGELYKS (SEQ ID NO: 226), ALKAGELYKS (SEQ ID NO: 227), AALKAGELYKS (SEQ ID NO: 228), NAALKAGELYKS (SEQ ID NO: 229), ANAALKAGELYKS (SEQ ID

NO: 230), RANAALKAGELYKS (SEQ ID NO: 231), and RRANAALKAGELYKS (SEQ ID NO: 232), or a sequence having at least about 80% sequence identity, or at least about 83% sequence identity, or at least about 85% sequence identity, or at least about 90% sequence identity, or at least about 95% sequence identity, or at least about 98% sequence identity thereto, provided that the sequence comprises at least one YKS sequence.

In certain embodiments, the bioconjugate comprises one or more peptides comprising an amino acid sequence selected from the group consisting of LYKC (SEQ ID NO: 233), ELYKC (SEQ ID NO: 234), GELYKC (SEQ ID NO: 235), AGELYKC (SEQ ID NO: 236), KAGELYKC (SEQ ID NO: 237), LKAGELYKC (SEQ ID NO: 238), ALKAGELYKC (SEQ ID NO: 239), AALKAGELYKC (SEQ ID NO: 240), NAALKAGELYKC (SEQ ID NO: 241), ANAALKAGELYKC (SEQ ID NO: 242), RANAALKAGELYKC (SEQ ID NO: 243), and RRANAALKAGELYKC (SEQ ID NO: 244), or a sequence having at least about 80% sequence identity, or at least about 83% sequence identity, or at least about 85% sequence identity, or at least about 90% sequence identity, or at least about 95% sequence identity, or at least about 98% sequence identity thereto, provided that the sequence comprises at least one YKC sequence.

In certain embodiments, the bioconjugate comprises one or more peptides comprising an amino acid sequence selected from the group consisting of A YKS (SEQ ID NO: 245), RAYKS (SEQ ID NO: 246), LRAYKS (SEQ ID NO: 247), NLRAYKS (SEQ ID NO: 248), LNLRAYKS (SEQ ID NO: 249), RLNLRAYKS (SEQ ID NO: 250), ARLNLRAYKS (SEQ ID NO: 251), LARLNLRAYKS (SEQ ID NO: 252), ILARLNLRAYKS (SEQ ID NO: 253), AILARLNLRAYKS (SEQ ID NO: 254), EAILARLNLRAYKS (SEQ ID NO: 255), AEAILARLNLRAYKS (SEQ ID NO: 256), KAEAILARLNLRAYKS (SEQ ID NO: 257), GKAEAILARLNLRAYKS (SEQ ID NO: 258), and YGKAEAILARLNLRAYKS (SEQ ID NO: 259), or a sequence having at least about 80% sequence identity, or at least about 83% sequence identity, or at least about 85% sequence identity, or at least about 90% sequence identity, or at least about 95% sequence identity, or at least about 98% sequence identity thereto, provided that the sequence comprises at least one YKS sequence.

In certain embodiments, the bioconjugate comprises one or more peptides comprising an amino acid sequence selected from the group consisting of AYKC (SEQ ID NO: 260), RAYKC (SEQ ID NO: 261), LRAYKC (SEQ ID NO: 262), NLRAYKC (SEQ ID NO: 263), LNLRAYKC (SEQ ID NO: 264), RLNLRAYKC (SEQ ID NO: 265), ARLNLRAYKC (SEQ ID NO: 266), LARLNLRAYKC (SEQ ID NO: 267), ILARLNLRAYKC (SEQ ID NO: 268), AILARLNLRAYKC (SEQ ID NO: 269), EAILARLNLRAYKC (SEQ ID NO: 270), AE AILARLNLRAYKC (SEQ ID NO: 271), KAE AILARLNLRAYKC (SEQ ID NO: 272), GKAEAILARLNLRAYKC (SEQ ID NO: 273), and YGKAEAILARLNLRAYKC (SEQ ID NO: 274), or a sequence having at least about 80% sequence identity, or at least about 83% sequence identity, or at least about 85% sequence identity, or at least about 90% sequence identity, or at least about 95% sequence identity, or at least about 98% sequence identity thereto, provided that the sequence comprises at least one YKC sequence.

In certain embodiments, the bioconjugate comprises one or more peptides comprising the amino acid sequence GAHWQFNALTVR (SEQ ID NO: 58), or a sequence having at least about 80% sequence identity, or at least about 83% sequence identity, or at least about 85% sequence identity, or at least about 90% sequence identity, or at least about 95% sequence identity, or at least about 98% sequence identity thereto, provided that the sequence binds to hyaluronic acid with a dissociation constant ($K_d$) of less than about 1 mM.

In certain embodiments, the bioconjugate comprises one or more peptides comprising the amino acid sequence STMMSRSHKTRSHHV (SEQ ID NO: 59), or a sequence having at least about 80% sequence identity, or at least about 83% sequence identity, or at least about 85% sequence identity, or at least about 90% sequence identity, or at least about 95% sequence identity, or at least about 98% sequence identity thereto, provided that the sequence binds to hyaluronic acid with a dissociation constant ($K_d$) of less than about 1 mM.

Accordingly, in some embodiments, the bioconjugate comprises one or more peptides comprising an amino acid sequence of formula (IA) or (IIA):

$(X)_n$-YKS-$(X)_m$-GSG (IA)

$(X)_n$-YKC-$(X)_m$-GSG (IIA)

wherein

n is from 0 to 50;

m is from 0 to 50;

and each X is independently selected from a natural or an unnatural amino acid. In some embodiments, each X is independently selected from the group consisting of alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, and valine. In some embodiments, each X is independently selected from the group consisting of alanine, arginine, asparagine, glutamic acid, glycine, isoleucine, leucine, lysine, and tyrosine.

In some embodiments, n in any of formulas I, II, IA and IIA is from 0 to about 50, or from 0 to about 40, or from 0 to about 30, or from 0 to about 20, or from 0 to about 15, or from 0 to about 10, or from 0 to about 5. In some embodiments, n is from 1 to about 40, or from 1 to about 30, or from 1 to about 20, or from 1 to about 15, or from 1 to about 10, or from 1 to about 5. In some embodiments, n is from 0 to about 50, or from 0 to about 40, or from 0 to about 30, or from 0 to about 20, from 0 to about 15, or from 0 to about 10, or from 0 to about 5. In some embodiments, n is 0, 1, 2, 3, 4, 5, 6, 7, 8, 9, 10, 11, 12, 13, 14, 15, 16, 17, 18, 19 or 20.

In some embodiments, m in any of formulas I, II, IA and IIA is from 0 to about 50, or from 0 to about 40, or from 0 to about 30, or from 0 to about 20, or from 0 to about 15, or from 0 to about 10, or from 0 to about 5. In some embodiments, m is from 1 to about 50, or from 1 to about 40, or from 1 to about 30, or from 1 to about 20, or from 1 to about 15, or from 1 to about 10, or from 1 to about 5. In some embodiments, m is from 0 to about 50, or from 0 to about 40, or from 0 to about 30, or from 0 to about 20, from 0 to about 15, or from 0 to about 10, or from 0 to about 5. In some embodiments, m is 0, 1, 2, 3, 4, 5, 6, 7, 8, 9, 10, 11, 12, 13, 14, 15, 16, 17, 18, 19 or 20.

In one embodiment, the bioconjugate comprises one or more peptides comprising up to about 40 amino acids. Accordingly, in certain embodiments, the sum of n and m in any of formulas I, II, IA and IIA is about 120, or about 110, or about 100, or about 90, or about 80, or about 70, or about 60, or about 50, or about 40, or about 30, or about 25, or about 20, or about 15, or about 10, or about 5, or about 3, or about 2.

In certain embodiments, the bioconjugate comprises one or more peptides comprising an amino acid sequence selected from the group consisting of YKSILYGSG (SEQ ID NO: 275), LYKSILYGSG (SEQ ID NO: 276), ELYKSILYGSG (SEQ ID NO: 277), GELYKSILYGSG (SEQ ID NO: 278), AGELYKSII,YGSG (SEQ ID NO: 279), KAGEL YKSIL YGSG (SEQ ID NO: 280), LKAGELYKSILYGSG (SEQ ID NO: 281), ALKAGELYKSILYGSG (SEQ ID NO: 282), AALKAGEL YKSIL YGSG (SEQ ID NO: 283), NAALKAGEL YKSIL YGSG (SEQ ID NO: 284), ANAALKAGEL YKSIL YGSG (SEQ ID NO: 285), RANAALKAGEL YKSIL YGSG (SEQ ID NO: 286), and RRANAALKAGEL YKSIL YGSG (SEQ ID NO: 287), or a sequence having at least about 80% sequence identity, or at least about 83% sequence identity, or at least about 85% sequence identity, or at least about 90% sequence identity, or at least about 95% sequence identity, or at least about 98% sequence identity thereto, provided that the sequence comprises at least one YKS sequence.

In certain embodiments, the bioconjugate comprises one or more peptides comprising an amino acid sequence selected from the group consisting of YKCILYGSG (SEQ ID NO: 288), LYKCII,YGSG (SEQ ID NO: 289), ELYKCILYGSG (SEQ ID NO: 290), GELYKCILYGSG (SEQ ID NO: 291), AGELYKCILYGSG (SEQ ID NO: 292), KAGELYKCILYGSG (SEQ ID NO: 293), LKAGELYKCILYGSG (SEQ ID NO: 294), ALKAGELYKCILYGSG (SEQ ID NO: 295), AALKAGELYKCII,YGSG (SEQ ID NO: 296), NAALKAGELYKCILYGSG (SEQ ID NO: 297), ANAALKAGELYKCII,YGSG (SEQ ID NO: 298), RANAALKAGELYKCILYGSG (SEQ ID NO: 299), and RRANAALKAGELYKCILYGSG (SEQ ID NO: 300), or a sequence having at least about 80% sequence identity, or at least about 83% sequence identity, or at least about 85% sequence identity, or at least about 90% sequence identity, or at least about 95% sequence identity, or at least about 98% sequence identity thereto, provided that the sequence comprises at least one YKC sequence.

In certain embodiments, the bioconjugate comprises one or more peptides comprising an amino acid sequence selected from the group consisting of YKSGSG (SEQ ID NO: 301), LYKSGSG (SEQ ID NO: 302), ELYKSGSG (SEQ ID NO: 303), GELYKSGSG (SEQ ID NO: 304), AGELYKSGSG (SEQ ID NO: 305), KAGELYKSGSG (SEQ ID NO: 306), LKAGELYKSGSG (SEQ ID NO: 307), ALKAGELYKSGSG (SEQ ID NO: 308), AALKAGELYKSGSG (SEQ ID NO: 309), NAALKAGELYKSGSG (SEQ ID NO: 310), ANAALKAGELYKSGSG (SEQ ID NO: 311), RANAALKAGELYKSGSG (SEQ ID NO: 312), and RRANAALKAGELYKSGSG (SEQ ID NO: 313), or a sequence having at least about 80% sequence identity, or at least about 83% sequence identity, or at least about 85% sequence identity, or at least about 90% sequence identity, or at least about 95% sequence identity, or at least about 98% sequence identity thereto, provided that the sequence comprises at least one YKS sequence.

In certain embodiments, the bioconjugate comprises one or more peptides comprising an amino acid sequence selected from the group consisting of YKCGSG (SEQ ID NO: 314), LYKCGSG (SEQ ID NO: 315), ELYKCGSG (SEQ ID NO: 316), GELYKCGSG (SEQ ID NO: 317), AGELYKCGSG (SEQ ID NO: 318), KAGELYKCGSG (SEQ ID NO: 319), LKAGELYKCGSG (SEQ ID NO: 320), ALKAGELYKCGSG (SEQ ID NO: 321), AALKAGELYKCGSG (SEQ ID NO: 322), NAALKAGELYKCGSG (SEQ ID NO: 323), ANAALKAGELYKCGSG (SEQ ID NO: 324), RANAALKAGELYKCGSG (SEQ ID NO: 325), and RRANAALKAGELYKCGSG (SEQ ID NO: 326), or a sequence having at least about 80% sequence identity, or at least about 83% sequence identity, or at least about 85% sequence identity, or at least about 90% sequence identity, or at least about 95% sequence identity, or at least about 98% sequence identity thereto, provided that the sequence comprises at least one YKC sequence.

In certain embodiments, the bioconjugate comprises one or more peptides comprising an amino acid sequence selected from the group consisting of AYKSGSG (SEQ ID NO: 327), RAYKSGSG (SEQ ID NO: 328), LRAYKSGSG (SEQ ID NO: 329), NLRAYKSGSG (SEQ ID NO: 330), LNLRAYKSGSG (SEQ ID NO: 331), RLNLRAYKSGSG (SEQ ID NO: 332), ARLNLRAYKSGSG (SEQ ID NO: 333), LARLNLRAYKSGSG (SEQ ID NO: 334), ILARLNLRAYKSGSG (SEQ ID NO: 335), AILARLNLRAYKSGSG (SEQ ID NO: 336), EAILARLNLRAYKSGSG (SEQ ID NO: 337), AEAILARLNLRAYKSGSG (SEQ ID NO: 338), KAEAILARLNLRAYKSGSG (SEQ ID NO: 339), GKAEAILARLNLRAYKSGSG (SEQ ID NO: 340), and YGKAEAILARLNLRAYKSGSG (SEQ ID NO: 341), or a sequence having at least about 80% sequence identity, or at least about 83% sequence identity, or at least about 85% sequence identity, or at least about 90% sequence identity, or at least about 95% sequence identity, or at least about 98% sequence identity thereto, provided that the sequence comprises at least one YKS sequence.

In certain embodiments, the bioconjugate comprises one or more peptides comprising an amino acid sequence selected from the group consisting of AYKCGSG (SEQ ID NO: 342), RAYKCGSG (SEQ ID NO: 343), LRAYKCGSG (SEQ ID NO: 344), NLRAYKCGSG (SEQ ID NO: 345), LNLRAYKCGSG (SEQ ID NO: 346), RLNLRAYKCGSG (SEQ ID NO: 347), ARLNLRAYKCGSG (SEQ ID NO: 348), LARLNLRAYKCGSG (SEQ ID NO: 349), ILARLNLRAYKCGSG (SEQ ID NO: 350), AILARLNLRAYKCGSG (SEQ ID NO: 351), EAILARLNLRAYKCGSG (SEQ ID NO: 352), AEAILARLNLRAYKCGSG (SEQ ID NO: 353), KAEAILARLNLRAYKCGSG (SEQ ID NO: 354), GKAEAILARLNLRAYKCGSG (SEQ ID NO: 355), and YGKAEAILARLNLRAYKCGSG (SEQ ID NO: 356), or a sequence having at least about 80% sequence identity, or at least about 83% sequence identity, or at least about 85% sequence identity, or at least about 90% sequence identity, or at least about 95% sequence identity, or at least about 98% sequence identity thereto, provided that the sequence comprises at least one YKC sequence.

In certain embodiments, the bioconjugate comprises one or more peptides comprising the amino acid sequence GAHWQFNALTVRGSG (SEQ ID NO: 357), or a sequence having at least about 80% sequence identity, or at least about 83% sequence identity, or at least about 85% sequence identity, or at least about 90% sequence identity, or at least about 95% sequence identity, or at least about 98% sequence identity thereto, provided that the sequence binds to hyaluronic acid with a dissociation constant ($K_d$) of less than about 1 mM.

In certain embodiments, the bioconjugate comprises one or more peptides comprising the amino acid sequence STMMSRSHKTRSHHVGSG (SEQ ID NO: 358), or a sequence having at least about 80% sequence identity, or at least about 83% sequence identity, or at least about 85% sequence identity, or at least about 90% sequence identity, or at least about 95% sequence identity, or at least about 98% sequence identity thereto, provided that the sequence binds to hyaluronic acid with a dissociation constant ($K_d$) of less than about 1 mM.

In certain embodiments, one or more peptide(s) having a specified binding affinity for hyaluronic acid can be used in the bioconjugates described herein. Further, the peptides as used herein may comprise more than one hyaluronic acid-binding unit, where the binding units can be the same or different.

In addition, in certain embodiments, the bioconjugate comprises one or more peptides comprising at least one hyaluronic acid-binding unit and at least one collagen-binding unit. In one embodiment, the bioconjugate comprises one or more peptides comprising at least one hyaluronic acid-binding unit and at least one collagen-binding unit, where the collagen-binding unit binds to one or more of collagen type I, II, III, IV, V, VI, VII, VIII, IX, X, XI, XII, XIII, or XIV.

Depending on the desired properties of the bioconjugate, the total number of peptides bonded to the glycan can be varied. In certain embodiments, the total number of peptides present in the bioconjugate is from about 1 to about 50, or from about 1 to about 40, or from about 1 to about 30, or from about 1 to about 25, or from about 2 to about 30, or from about 2 to about 25, or from about 3 to about 25, or from about 4 to about 25, or from about 5 to about 25, or from about 5 to about 30, or from about 1 to about 25, or from about 2 to about 25, or from about 11 to about 14, or from about 1 to about 8, or from about 1 to about 5, or about 1, or about 2, or about 3, or about 4, or about 5, or about 6, or about 7, or about 8 peptides. In some embodiments, the bioconjugate comprises from about 10 to about 40 peptides. In other embodiments, the bioconjugate comprises from about 5 to about 30 peptides. In certain embodiments, the bioconjugate comprises less than about 20 peptides. In certain embodiments, the bioconjugate comprises less than about 18 peptides. In various embodiments, the bioconjugate comprises from about 4 to about 18 peptides. In certain embodiments, the bioconjugate comprises less than about 15 peptides. In certain embodiments, the bioconjugate comprises less than about 10 peptides. In certain embodiments, the bioconjugate comprises less than about 30 peptides. In certain embodiments, the bioconjugate comprises about 25 peptides. In certain embodiments, the bioconjugate comprises from about 5 to about 40, or from about 10 to about 40, or from about 5 to about 20, or from about 4 to about 18, or about 10, or about 11, or about 18, or about 20 peptides, or about 25 peptides, or about 30 peptides, or about 40 peptides, or about 50 peptides.

The peptides as described herein further comprise a hydrazide moiety for conjugation to the peptide. The hydrazide group can be bound to the peptide(s) at any suitable point of attachment, such as for example, the C-terminus, the N-terminus or via a side chain on an amino acid. For example, when a peptide is bound to the glycan via a side chain of an amino acid of the peptide, the side chain is glutamic acid or aspartic acid. The hydrazide can be formed between a hydrazine ($-NHNH_2$) bound to a carbonyl group present on an amino acid in the peptide sequence (e.g., a C-terminal carbonyl group).

In certain embodiments, the hydrazide group is bonded to the peptide(s) via a spacer. The spacer can be any appropriately functionalized linear or branched moiety, and typically has between about 4 and about 100 atoms. In one embodiment, the spacer comprises one or more, or from 1 to 10, or from 1 to 5, or from 1 to 3, amino acids. The amino acids can be any amino acid, and are in some instances non-polar amino acids, such as alanine, cysteine, glycine, isoleucine, leucine, methionine, phenylalanine, proline, tryptophan, tyrosine and valine. In certain embodiments, the

amino acids are selected from the group consisting of glycine, alanine, arginine and serine. In one embodiment, the spacer is selected from the group consisting of glycine, glycine-glycine, glycine-serine-glycine, arginine-arginine, arginine-glycine-serine-glycine and lysine-glycine-serine-glycine. The spacer can also comprise non-amino acid moieties, such as polyethylene glycol (PEG), 6-aminohexanoic acid, succinic acid, or combinations thereof, with or without an additional amino acid spacer(s). In certain embodiments, the peptide sequences described herein further comprise a GSG-$NHNH_2$ moiety. Typically, the GSG-$NHNH_2$ moiety is bound to either the C- or N-terminus.

In certain embodiments, the spacer comprises more than one binding site (may be linear or branched) such that more than one peptide sequence can be bound thereto, thus creating a branched construct. In addition, since the peptide can be bound to the glycan via a terminal or non-terminal amino acid moiety, the peptide will be branched when bound to the glycan via a non-terminal amino acid moiety. The binding sites on the spacer can be the same or different, and can be any suitable binding site, such as an amine or carboxylic acid moiety, such that a desired peptide sequence can be bound thereto (e.g. via an amide bond). Thus in certain embodiments, the spacer contains one or more lysine, glutamic acid or aspartic acid residues. Such constructs can provide peptides having more than one collagen- and/or hyaluronic acid-binding unit of the formula $P_nL$, where P is a collagen- and/or hyaluronic acid-binding unit, L is a spacer and n is an integer from 2 to about 10, or from 2 to 8, or from 2 to 6, or from 2 to 5, or from 2 to 4, or 2, or 3, or 4, or 5, or 6, or 7, or 8, or 9, or 10. For example, the spacer L can be an amino acid sequence such as KGSG (SEQ ID NO: 359), KKGSG (SEQ ID NO: 360), or KKKGSG (SEQ ID NO: 361), etc., providing 2, 3, or 4 binding sites, respectively.

In certain embodiments, spacers, or a combination thereof, can be used to create further branching. For example, the spacer may comprise one or more amino acids which contain a side chain capable of linking additional peptides or collagen-binding units. Exemplary amino acids for including in such spacers include, but are not limited to, lysine, glutamic acid, aspartic acid, etc. In certain embodiments, the spacer comprises from 2 to 6 amino acids, or 3 or 4 amino acids. In certain embodiments, the spacer comprises one or more amino acid sequences of the formula KXX, where each X is independently a natural or unnatural amino acid. Specific examples of spacers which can be used alone or in combination to make branched constructs include, but are not limited to, KRR, KKK, $(K)_n$GSG, and $(KRR)_n$-KGSG, where n is 0 to 5, or 1, 2, 3, 4, or 5. In one embodiment, the spacer is or GSGKRRGSG (SEQ ID NO: 362).

A schematic of these spacers bound to peptides is shown in the table below.

| Spacer | Number of peptides (i.e., binding sites) | Structure of Spacer |
|---|---|---|
| KGSG | 2 | 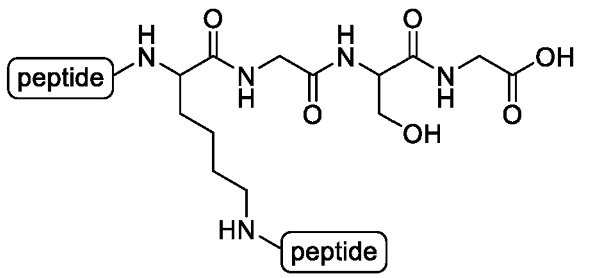 |
| KKGSG | 3 | 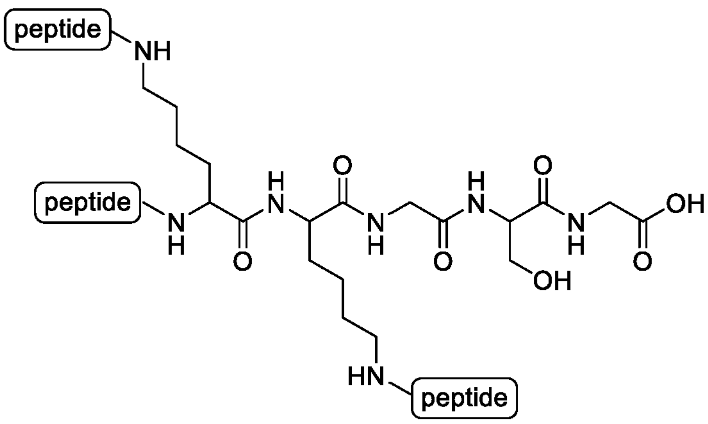 |

(continued)

| Spacer | Number of peptides (i.e., binding sites) | Structure of Spacer |
|---|---|---|
| KKKGSG | 4 | 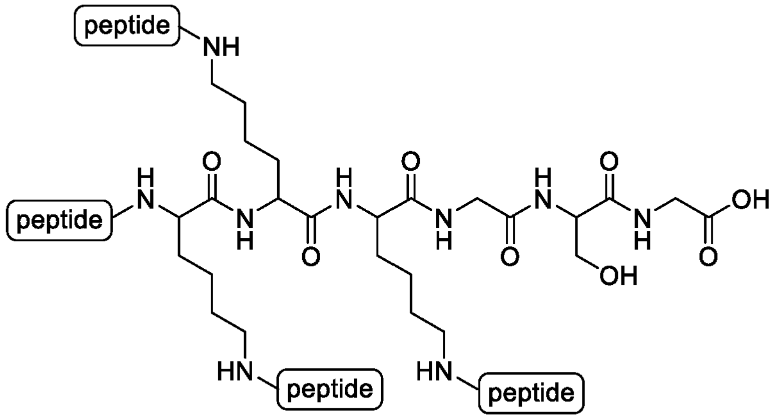 |
| $K_2$KGSG | 4 | 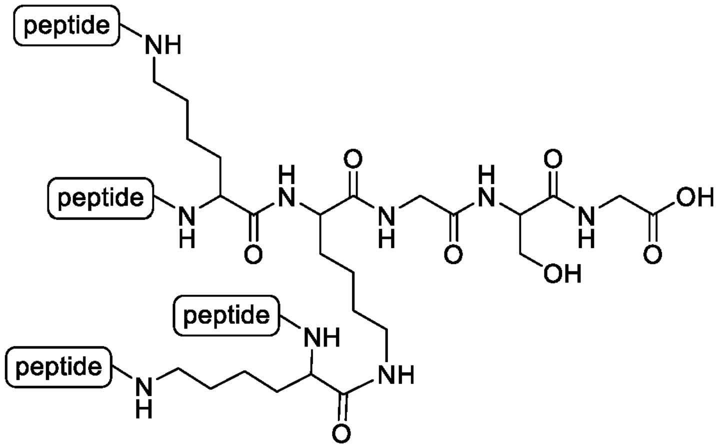 |

Exemplary collagen-binding constructs include, but are not limited to, $(GELYKSILYGSG)_2K$ (SEQ ID NO: 363), $(GELYKSILYGSG)_2KGSG$ (SEQ ID NO: 364), $(GELYKSILYGSG)_3KK$ (SEQ ID NO: 365), $(GELYKSILYGSG)_3KKGSG$ (SEQ ID NO: 366), $(GELYKSILYGSG)_4KKK$ (SEQ ID NO: 367), $(GELYKSILYGSG)_4KKKGSG$ (SEQ ID NO: 368), $(GQLYKSILYGSG)_2K$ (SEQ ID NO: 369), $(GQLYKSILYGSG)_2KGSG$ (SEQ ID NO: 370), $(GQLYKSILYGSG)_3KK$ (SEQ ID NO: 371), $(GQLYKSILYGSG)_3KKGSG$ (SEQ ID NO: 372), $(GQLYKSILYGSG)_4K_2K$ (SEQ ID NO: 373), $(GQLYKSILYGSG)_4K_2KGSG$ (SEQ ID NO: 374), $(GQLYKSILYGSG)_4KKK$ (SEQ ID NO: 375), $(GQLYKSILYGSG)_4KKKGSG$ (SEQ ID NO: 376), $(GQLYKSILYGSG)_4$-$(KRR)_2$-K (SEQ ID NO: 377), and $(GQLYKSILYGSG)_4$-$(KRR)_2$-KGSG (SEQ ID NO: 378).

In certain embodiments, the hydrazide group is bonded to the peptide(s) N-terminus. In certain embodiments, the hydrazide group is bonded to the peptide(s) C-terminus. In certain embodiments, the hydrazide group is bonded to a side chain of an amino acid in the peptide(s), such as a glutamic acid and/or aspartic acid. In certain embodiments, the hydrazide group is bonded to the peptide(s) C-terminus, via a spacer. The spacer can be bound to the peptide via any suitable bond. In some embodiments, the spacer is bound to the peptide via an amide bond.

In one embodiment, the hydrazide group is bonded to the C-terminus via a spacer comprising one or more amino acids selected from the group consisting of glycine, alanine, arginine and serine. In one embodiment, the spacer is selected from the group consisting of glycine, glycine-glycine, and glycine-serine-glycine. In various embodiments, the peptide comprises an amino acid spacer, such as glycine-serine-glycine (GSG), KRRGSG (SEQ ID NO: 384), or GSGKRRGSG (SEQ ID NO: 362).

In any of the embodiments described herein, the number of peptides per glycan is an average, where certain bioconjugates in a composition may have more peptides per glycan and certain bioconjugates have less peptides per glycan. Accordingly, in certain embodiments, the number of peptides as described herein is an average in a composition of bioconjugates. For example, in certain embodiments, the bioconjugates are a composition where the average number of peptides per glycan is about 5. In other embodiments, the average number of peptides per glycan is about 6, or about

7, or about 8, or about 9, or about 10, or about 11, or about 12, or about 13, or about 14, or about 15, or about 16, or about 17, or about 18, or about 19, or about 20, or about 25, or about 30.

In certain embodiments, the number of peptides per glycan may be described as a "percent (%) functionalization" based on the percent of disaccharide units which are functionalized with peptide on the glycan backbone. For example, the total number of available disaccharide units present on the glycan can be calculated by dividing the molecular weight (or the average molecular weight) of a single disaccharide unit (e.g., about 550-800 Da, or from about 650-750 Da) by the molecular weight of the glycan (e.g., about 25 kDa up to about 70 kDa, or even about 100 kDa). In embodiments where the glycan does not contain conventional "disaccharide units" (e.g., alginic acid), the total number of available disaccharide units present on the glycan to be used in the calculations presented herein, can be calculated by dividing the molecular weight (or the average molecular weight) of a single saccharide unit by the molecular weight of the glycan, and multiplying by 2.

In some embodiments, the number of available disaccharide units present on the glycan is from about 10 to about 80, or from about 10 to about 70, or from about 15 to about 70, or from about 20 to about 70, or from about 30 to about 70, or from about 35 to about 70, or from about 40 to about 70, or from about 10 to about 75, or from about 15 to about 75, or from about 20 to about 75, or from about 30 to about 75, or from about 35 to about 75, or from about 40 to about 75, or from about 10 to about 50, or from about 20 to about 50, or from about 25 to about 50, or from about 10 to about 35, or from about 15 to about 35, or from about 20 to about 35, or from about 10 to about 30, or from about 15 to about 30, or from about 20 to about 30, or about 15, or about 20, or about 25, or about 30, or about 35, or about 40, or about 45, or about 50, or about 55, or about 60, or about 65, or about 70.

Therefore, in certain embodiments, the glycan comprises from about 1 to about 50, or from about 5 to about 30% functionalization, or about 25% functionalization, wherein the percent (%) functionalization is determined by a percent of disaccharide units on the glycan which are functionalized with peptide. In some embodiments, the percent (%) functionalization of the glycan is from about 1% to about 50%, or from about 3% to about 40%, or from about 5% to about 30%, or from about 10% to about 20%, or about 1%, or about 2%, or about 5%, or about 10%, or about 15%, or about 20%, or about 25%, or about 30%, or about 35%, or about 40%, or about 45%, or about 50%, or about 55%, or about 60%, or about 65%, or about 70%, or about 75%, or about 80%, or about 85%, or about 90%, or about 95%, or about 100%.

In certain embodiments, provided is a composition comprising a bioconjugate as described herein and peptide, where the peptide is closely associated (e.g., via ionic bonding) to the bioconjugate. In certain embodiments, a bioconjugate aggregate may be formed thereby. It is contemplated that the bioconjugate aggregate (comprising bioconjugate and non-covalently bound peptide) may comprise from 1% to 200% functionalization (determined by a percent of disaccharide units on the glycan which are functionalized with peptide). In some embodiments, the percent (%) functionalization of the bioconjugate is from about 1% to about 50%, or from about 3% to about 40%, or from about 5% to about 30%, or from about 10% to about 20%, or about 1%, or about 2%, or about 5%, or about 10%, or about 15%, or about 20%, or about 25%, or about 30%, or about 35%, or about 40%, or about 45%, or about 50%, or about 55%, or about 60%, or about 65%, or about 70%, or about 75%, or about 80%, or about 85%, or about 90%, or about 95%, or about 100%.

It is contemplated that any glycan can be utilized in the various embodiments described herein, selected from chondroitin sulfate (CS), dermatan sulfate (DS), and heparin (Hep). The glycan can be naturally occurring or chemically derivatized, such as, but not limited to, partially N-desulfated derivatives, partially O-desulfated derivatives, and/or partially O-carboxymethylated derivatives.

In some embodiments, the glycan is unmodified. The glycan does not contain oxidatively cleaved saccharide rings and thus does not contain aldehyde functional groups. It is contemplated that the beneficial effects exhibited by the bioconjugates as disclosed herein may be at least partially be attributed to the glycan not containing oxidatively cleaved saccharide rings. For example, the bioconjugates as disclosed herein having a hyaluronic acid-binding unit exhibit an increased binding affinity when compared to hyaluronic acid-binding bioconjugates known in the art (See, e.g., Example 5).

Such a linkage can result from coupling a hydrazide group on the peptide and a carbonyl group (e.g., a carboxylic acid group, or activated derivative thereof) on the glycan, or alternatively, a hydrazide group on the glycan and a carbonyl group (e.g., a carboxylic acid group, or activated derivative thereof) on the peptide. In certain embodiments, the hydrazide-carbonyl linkage is between a terminal hydrazide group on the peptide(s) and a carbonyl group on the glycan.

In one embodiment, the glycan is heparin, where the heparin may include heparin derivatives, such as, but not limited to partially N- and/or partially O-desulfated heparin derivatives, partially O-carboxymethylated heparin derivatives, or a combination thereof. In certain embodiments, the heparin is non-oxidized heparin (i.e., does not contain oxidatively cleaved saccharide rings) and does not contain aldehyde functional groups. Heparin derivatives and/or methods for providing heparin derivatives, such as partially N-desulfated heparin and/or partially O-desulfated heparin (i.e., 2-O and/or 6-O-desulfated heparin) are known in the art (see, e.g., Kariya et al., J. Biol. Chem., 2000, 275:25949-5958; Lapierre, et al. Glycobiology, 1996, 6(3):355-366). It is also contemplated that partially O-carboxymethylated heparin (or any glycan) derivatives, such as those which could be prepared according to Prestwich, et al. (US 2012/0142907;

US 2010/0330143), can be used to provide the bioconjugates disclosed herein.

In certain embodiments, the molecular weight of the glycan is varied to tailor the effects of the bioconjugate (see e.g., Radek, K. A., et al., Wound Repair Regen., 2009, 17: 118-126; and Taylor, K. R., et al., J. Biol. Chem., 2005, 280:5300-5306). In another embodiment, the glycan is degraded by oxidation and alkaline elimination (see e.g., Fransson, L. A., et al., Eur. J. Biochem., 1980, 106 :59-69) to afford degraded glycan having a lower molecular weight (e.g., from about 10 kDa to about 50 kDa).

In one embodiment, the glycan is dermatan sulfate (DS). The biological functions of DS is extensive, and includes the binding and activation of growth factors FGF-2, FGF-7, and FGF-10, which promote endothelial cell and keratinocyte proliferation and migration. In some embodiments, the weight range of the dermatan sulfate is from about 10 kDa to about 70 kDa. In one embodiment, the molecular weight of the dermatan sulfate is about 46 kDa. In another embodiment, the dermatan sulfate is degraded by oxidation and alkaline elimination (see e.g., Fransson, L. A., et al., Eur. J. Biochem., 1980, 106:59-69) to afford degraded dermatan sulfate having a low molecular weight (e.g., about 10 kDa).

In another embodiment, the glycan is heparin. Various molecular weights for the heparin can be used in the bioconjugates described herein, such as from a single disaccharide unit of about 650-700 Da to about 50 kDa. In some embodiments, the heparin is from about 10 to about 20 kDa. In some embodiments, the heparin is up to about 15, or about 16, or about 17, or about 18, or about 19, or about 20 kDa. In certain embodiments, the heparin may be oxidized under conditions that do not cleave one or more of the saccharide rings (see, e.g., Wang, et al. Biomacromolecules 2013, 14(7):2427-2432).

In one embodiment, the bioconjugate comprises a glycan and from about 5 to about 10, or about 7, peptides, wherein the peptides comprise at least one sequence of GELYKSILY (SEQ ID NO: 2) or GELYKSILYGSG (SEQ ID NO: 278), and are bound to the glycan via a hydrazide-carbonyl linkage. In certain embodiments, the hydrazide-carbonyl linkage is between a terminal hydrazide group on the peptides and a carbonyl group on the glycan. In one embodiment, the glycan is heparin. The heparin does not contain oxidatively cleaved saccharide rings and thus does not contain aldehyde functional groups.

In one embodiment, the bioconjugate comprises a glycan and about 20 peptides, wherein the peptides comprise at least one sequence of GELYKSILY (SEQ ID NO: 2) or GELYKSILYGSG (SEQ ID NO: 278), and are bound to the glycan via a hydrazide-carbonyl linkage. In certain embodiments, the hydrazide-carbonyl linkage is between a terminal hydrazide group on the peptides and a carbonyl group on the glycan. In one embodiment, the glycan is dermatan sulfate. , The dermatan sulfate does not contain oxidatively cleaved saccharide rings and thus does not contain aldehyde functional groups.

In one embodiment, the bioconjugate comprises a glycan and from about 5 to about 10, or about 7, peptides, wherein the peptides comprise at least one sequence of GQLYKSILY (SEQ ID NO: 16) or GQLYKSILYGSG (SEQ ID NO: 387), and are bound to the glycan via a hydrazide-carbonyl linkage. In certain embodiments, the hydrazide-carbonyl linkage is between a terminal hydrazide group on the peptides and a carbonyl group on the glycan. In one embodiment, the glycan is heparin. The heparin does not contain oxidatively cleaved saccharide rings and thus does not contain aldehyde functional groups.

In one embodiment, the bioconjugate comprises a glycan and about 20 peptides, wherein the peptides comprise at least one sequence of GQLYKSILY (SEQ ID NO: 16) or GQLYKSILYGSG (SEQ ID NO: 387), and are bound to the glycan via a hydrazide-carbonyl linkage. In certain embodiments, the hydrazide-carbonyl linkage is between a terminal hydrazide group on the peptides and a carbonyl group on the glycan. In one embodiment, the glycan is dermatan sulfate. The dermatan sulfate does not contain oxidatively cleaved saccharide rings and thus does not contain aldehyde functional groups.

In one embodiment, the bioconjugate comprises a glycan and from about 5 to about 10, or about 7, peptides, wherein the peptides comprise at least one sequence of RRANAALKAGELYKSILY (SEQ ID NO: 1) or RRANAALKAGEL YKSIL YGSG (SEQ ID NO: 287), and are bound to the glycan via a hydrazide-carbonyl linkage. In certain embodiments, the hydrazide-carbonyl linkage is between a terminal hydrazide group on the peptides and a carbonyl group on the glycan. In one embodiment, the glycan is heparin. The heparin does not contain oxidatively cleaved saccharide rings and thus does not contain aldehyde functional groups.

In one embodiment, the bioconjugate comprises a glycan and about 20 peptides, wherein the peptides comprise at least one sequence of RRANAALKAGELYKSILY (SEQ ID NO: 1) or RRANAALKAGEL YKSIL YGSG (SEQ ID NO: 287), and are bound to the glycan via a hydrazide-carbonyl linkage. In certain embodiments, the hydrazide-carbonyl linkage is between a terminal hydrazide group on the peptides and a carbonyl group on the glycan. In one embodiment, the glycan is dermatan sulfate. The dermatan sulfate does not contain oxidatively cleaved saccharide rings and thus does not contain aldehyde functional groups.

In one embodiment, the bioconjugate is not a bioconjugate comprising dermatan sulfate and about 1 to about 50 peptides comprising at least one sequence of RRANAALKAGELYKSILY (SEQ ID NO: 1), RRANAALKAGEL YKSIL YGC (SEQ ID NO: 388) or RRANAALKAGEL YKSIL YGSG (SEQ ID NO: 287), where the peptides are bound to the

glycan via a hydrazide-carbonyl linkage.

In one embodiment, the bioconjugate comprises a glycan and from about 5 to about 10, or about 7, peptides, wherein the peptides comprise at least one sequence of RRANAALKAGQLYKSILY (SEQ ID NO: 17) or RRANAALKAGQLYKSILYGSG (SEQ ID NO: 389), and are bound to the glycan via a hydrazide-carbonyl linkage. In certain embodiments, the hydrazide-carbonyl linkage is between a terminal hydrazide group on the peptides and a carbonyl group on the glycan. In one embodiment, the glycan is heparin. The heparin does not contain oxidatively cleaved saccharide rings and thus does not contain aldehyde functional groups.

In one embodiment, the bioconjugate comprises a glycan and about 20 peptides, wherein the peptides comprise at least one sequence of RRANAALKAGQLYKSILY (SEQ ID NO: 17) or RRANAALKAGQLYKSILYGSG (SEQ ID NO: 389), and are bound to the glycan via a hydrazide-carbonyl linkage. In certain embodiments, the hydrazide-carbonyl linkage is between a terminal hydrazide group on the peptides and a carbonyl group on the glycan. In one embodiment, the glycan is dermatan sulfate. The dermatan sulfate does not contain oxidatively cleaved saccharide rings and thus does not contain aldehyde functional groups.

In one embodiment, the bioconjugate comprises a glycan and from about 1 to about 5, or about 1 to 2, peptides, wherein the peptides comprise $(GQLYKSILY)_4$-$(KRR)_2$-K (SEQ ID NO: 390), $(GQLYKSILYGSG)_4$-$(KRR)_2$-KGSG (SEQ ID NO: 378) or $(GQLYKSILY)_4$-$(KRR)_2$-KGSG (SEQ ID NO: 391), and are bound to the glycan via a hydrazide-carbonyl linkage. In certain embodiments, the hydrazide-carbonyl linkage is between a terminal hydrazide group on the peptides and a carbonyl group on the glycan. In one embodiment, the glycan is heparin. ITthe heparin does not contain oxidatively cleaved saccharide rings and thus does not contain aldehyde functional groups.

In one embodiment, the bioconjugate comprises a glycan and about 5 to about 10 peptides, wherein the peptides comprise at least one sequence of GAHWQFNALTVR (SEQ ID NO: 58) or GAHWQFNALTVRGSG (SEQ ID NO: 357), and are bound to the glycan via a hydrazide-carbonyl linkage. In certain embodiments, the hydrazide-carbonyl linkage is between a terminal hydrazide group on the peptides and a carbonyl group on the glycan. In one embodiment, the glycan is chondroitin sulfate. The chondroitin sulfate does not contain oxidatively cleaved saccharide rings and thus does not contain aldehyde functional groups.

In one embodiment, the bioconjugate comprises a glycan and about 5 to about 10 peptides, wherein the peptides comprise at least one sequence of STMMSRSHKTRSHHV (SEQ ID NO: 59) or STMMSRSHKTRSHHVGSG (SEQ ID NO: 358), and are bound to the glycan via a hydrazide-carbonyl linkage. In certain embodiments, the hydrazide-carbonyl linkage is between a terminal hydrazide group on the peptides and a carbonyl group on the glycan. In one embodiment, the glycan is chondroitin sulfate. The chondroitin sulfate does not contain oxidatively cleaved saccharide rings and thus does not contain aldehyde functional groups.

In one embodiment, the bioconjugate comprises a glycan and about 1 to about 20 peptides, wherein the peptides comprise at least one sequence of GAHWQFNALTVR (SEQ ID NO: 58) or GAHWQFNALTVRGSG (SEQ ID NO: 357), and at least one sequence of WYRGRL (SEQ ID NO: 29) or WYRGRLGSG (SEQ ID NO: 392), and are bound to the glycan via a hydrazide-carbonyl linkage. In one embodiment, the glycan is chondroitin sulfate. The chondroitin sulfate does not contain oxidatively cleaved saccharide rings and thus does not contain aldehyde functional groups.

In one embodiment, the bioconjugate comprises a glycan and about 1 to about 20 peptides, wherein the peptides comprise at least one sequence of GAHWQFNALTVR (SEQ ID NO: 58) or GAHWQFNALTVRGSG (SEQ ID NO: 357), and at least one sequence of RRANAALKAGELYKSILY (SEQ ID NO: 1) or RRANAALKAGEL YKSIL YGSG (SEQ ID NO: 287), and are bound to the glycan via a hydrazide-carbonyl linkage. In one embodiment, the glycan is chondroitin sulfate. The chondroitin sulfate does not contain oxidatively cleaved saccharide rings and thus does not contain aldehyde functional groups.

### 3. Synthesis of Bioconjugates

The peptides as used herein may be purchased from a commercial source or partially or fully synthesized using methods well known in the art (e.g., chemical and/or biotechnological methods). In certain embodiments, the peptides are synthesized according to solid phase peptide synthesis protocols that are well known in the art. In another embodiment, the peptide is synthesized on a solid support according to the well-known Fmoc protocol, cleaved from the support with trifluoroacetic acid and purified by chromatography according to methods known to persons skilled in the art. In other embodiments, the peptide is synthesized utilizing the methods of biotechnology that are well known to persons skilled in the art. In one embodiment, a DNA sequence that encodes the amino acid sequence information for the desired peptide is ligated by recombinant DNA techniques known to persons skilled in the art into an expression plasmid (for example, a plasmid that incorporates an affinity tag for affinity purification of the peptide), the plasmid is transfected into a host organism for expression, and the peptide is then isolated from the host organism or the growth medium, e.g., by affinity purification. Recombinant DNA technology methods are described in Sambrook et al., "Molecular Cloning: A Laboratory Manual", 3rd Edition, Cold Spring Harbor Laboratory Press, (2001), and are well-known to the skilled artisan.

As shown in **Scheme 1,** the peptides as described herein can be covalently bound to the glycan (e.g., heparin)

**1A** through a carboxylic acid moiety to provide a bioconjugate **1B** as disclosed herein. As is typical in peptide coupling reactions, an activating agent may be used to facilitate the reaction. Suitable coupling agents (or activating agents) are known in the art and include for example, carbodiimides (e.g., N,N'-dicyclohexylcarbodiimide (DCC), N,N'-dicyclopentylcarbodiimide, N,N'-diisopropylcarbodiimide (DIC), 1-ethyl-3-(3-dimethylaminopropyl)carbodiimide (EDC), N-t-butyl-N-methylcarbodiimide (BMC), N-t-butyl-N-ethylcarbodiimide (BEC), 1,3-bis(2,2-dimethyl-1,3-dioxolan-4-ylmethyl)carbodiimide (BDDC), etc.), anhydrides (e.g., symmetric, mixed, or cyclic anhydrides), activated esters (e.g., phenyl activated ester derivatives, p-hydroxamic activated ester, hexafluoroacetone (HFA), etc.), acylazoles (acylimidazoles using CDI, acylbenzotriazoles, etc.), acyl azides, acid halides, phosphonium salts (HOBt, PyBOP, HOAt, etc.), aminium/uronium salts (e.g., tetramethyl aminium salts, bispyrrolidino aminium salts, bispiperidino aminium salts, imidazolium uronium salts, pyrimidinium uronium salts, uronium salts derived from N,N,N'-trimethyl-N'-phenylurea, morpholino-based aminium/uronium coupling reagents, antimoniate uronium salts, etc.), organophosphorus reagents (e.g., phosphinic and phosphoric acid derivatives), organosulfur reagents (e.g., sulfonic acid derivatives), triazine coupling reagents (e.g., 2-chloro-4,6-dimethoxy-1,3,5-triazine, 4-(4,6-dimethoxy-1,3,5-triazin-2-yl)-4 methylmorpholinium chloride, 4-(4,6-dimethoxy-1,3,5-triazin-2-yl)-4 methylmorpholinium tetrafluoroborate, etc.), pyridinium coupling reagents (e.g., Mukaiyama's reagent, pyridinium tetrafluoroborate coupling reagents, etc.), polymer-supported reagents (e.g., polymer-bound carbodiimide, polymer-bound TBTU, polymer-bound 2,4,6-trichloro-1,3,5-triazine, polymer-bound HOBt, polymer-bound HOSu, polymer-bound IIDQ, polymer-bound EEDQ, etc.), and the like (see, e.g., El-Faham, et al. Chem. Rev., 2011, 111(11): 6557-6602; Han, et al. Tetrahedron, 2004, 60:2447-2467).

In one embodiment, the peptide coupling reaction proceeds via an activated glycan intermediate by reacting a carboxylic acid moiety of the glycan with a coupling agent (e.g., a carbodiimide reagent, such as but not limited to, N,N'-dicyclohexylcarbodiimide (DCC), N,N'-diisopropylcarbodiimide (DIC), 1-ethyl-3-(3-dimethylaminopropyl)carbodiimide (EDC), etc.) to form an O-acylisourea intermediate. Such carbodiimide chemistry is well known in the art and suitable coupling agents can be purchased from commercial sources. Contacting the O-acylisourea intermediate with the desired peptide yields the bioconjugate. The glycan can be contacted with activating agent prior to, or in the presence of, the peptide. In some embodiments, the reaction is carried out in the presence of N-hydroxysuccinimide (NHS) or derivatives thereof. In certain embodiments, the peptide sequence can comprise a reactive moiety (e.g., a hydrazide functional group) to aid in the coupling reaction with the glycan, or O-acylisourea intermediate thereof. In some embodiments, the peptide sequence includes one or more amino acid residues that act as a spacer between the binding unit and the terminal amino acid (e.g., a terminating glycine) or reactive moiety (i.e., hydrazide functional group). For example, a serine-glycine (SG), glycine-serine-glycine (GSG) or glycine-serine-glycine-serine-glycine (GSGSG) spacer may be added to provide an attachment point for the glycan. In addition, in certain instances where one or more amino acids in the peptides contain reactive functional groups (e.g., carboxylic acid side chains), standard protecting group chemistry may be used to protect one or more side chains facilitate the coupling reaction. In addition, non-amino acid spacers may also be employed alone, or in combination with amino acid spacers (e.g., aminohexanoic acid)

**Scheme 1.** *Synthesis of Bioconjugates*

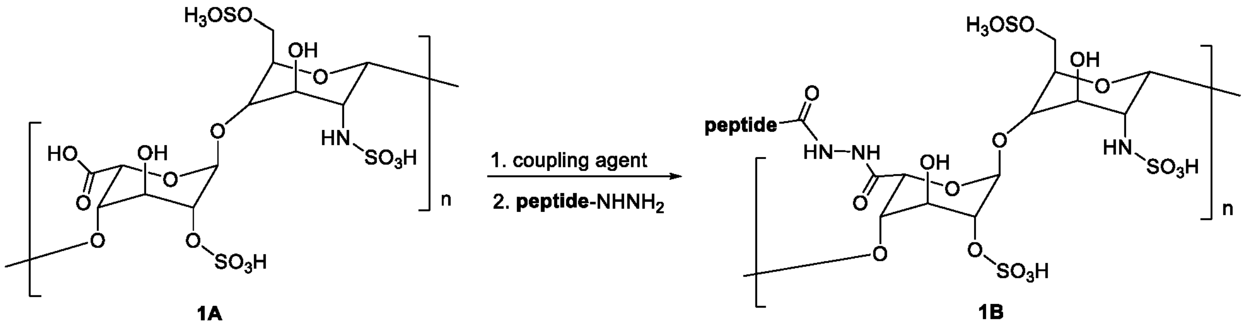


In certain embodiments, the bioconjugates are derived from modified glycan derivatives (e.g., heparin) **(Scheme** 2). Various glycan derivatives suitable for use in the bioconjugates described herein are known in the art, such as partially N-desulfated heparin and partially O-desulfated heparin (i.e., 2-O and/or 6-O-desulfated heparin, see, e.g., Kariya et al., J. Biol. Chem., 2000, 275:25949-5958; Lapierre, et al. Glycobiology, 1996, 6(3):355-366). Exemplary methods are shown below in **Scheme 2.** As shown in **Scheme 2,** glycan (e.g., heparin) **1A** can be reacted with a suitable desulfating agent, such as for example, a base (e.g., NaOH) or a silylating reagent (e.g., N,O-bis(trimethylsilyl)acetamide (BTSA), N-methyl-N-(trimethylsilyl)trifluoro acetamide (MTSTFA), etc.) to provide one or more desulfated glycan derivative(s) **2A.** As is apparent to one of skill in the art, the glycan derivative **2A** can be tailored depending on the reagents and

reaction conditions employed, such that partial, complete or a mixture of desulfated glycan derivative(s) **2A** can be obtained. The desulfated glycan derivative(s) **2A** can then be reacted with peptide, optionally in the presence of a coupling agent, as described above for **Scheme 1,** under typical peptide coupling reaction conditions to provide bioconjugate **2B.** In addition, as shown in **Scheme 2,** glycan derivatives having at least one hydroxyl group (e.g., 6-O-desulfated heparin) can be converted to an O-carboxymethylated glycan derivative(s) (e.g., 6-O- carboxymethylated heparin) **2C** (see, e.g., Prestwich, et al. in US 2012/0142907 and US 2010/0330143). Reaction of **2C** with peptide, optionally in the presence of a coupling agent as described above for **Scheme 1** under typical peptide coupling reaction conditions can provide bioconjugates **2D** and/or **2E.**

**Scheme 2.** *Alternative Synthesis of Bioconjugates*

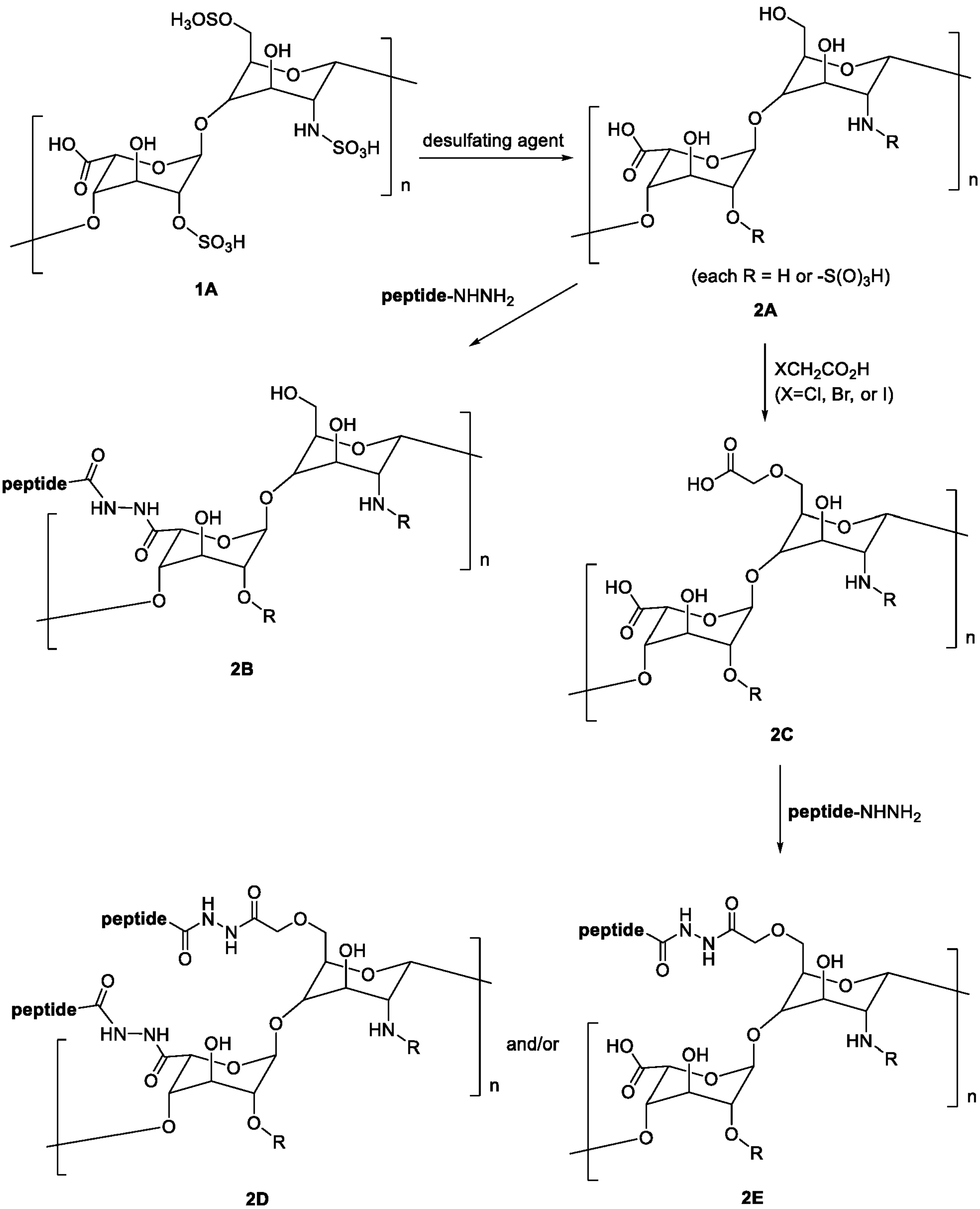


1A, 2A, 2B, 2C, 2D, 2E

In contrast to Schemes 1 and 2, Scheme 3 shows the synthesis of bioconjugates known in the art. As shown in **Scheme 1,** the glycan (e.g., chondroitin sulfate "CS") is oxidized using a periodate reagent, such as sodium periodate, to provide aldehyde functional groups on the glycan (e.g., "ox-CS") for covalently bonding the peptides to the glycan. The peptides are then covalently bonded to the glycan (e.g., chondroitin sulfate "CS") by reacting an aldehyde function of the oxidized glycan (e.g., "ox-CS") with N-[β-maleimidopropionic acid]hydrazide (BMPH) to form a glycan intermediate

(e.g., "BMPH-CS") and further reacting the glycan intermediate with peptides containing at least one free thiol group (i.e., -SH group) to yield the synthetic peptidoglycan.

**Scheme 3.** *Synthesis of CS-BMPH-Peptide$_n$*

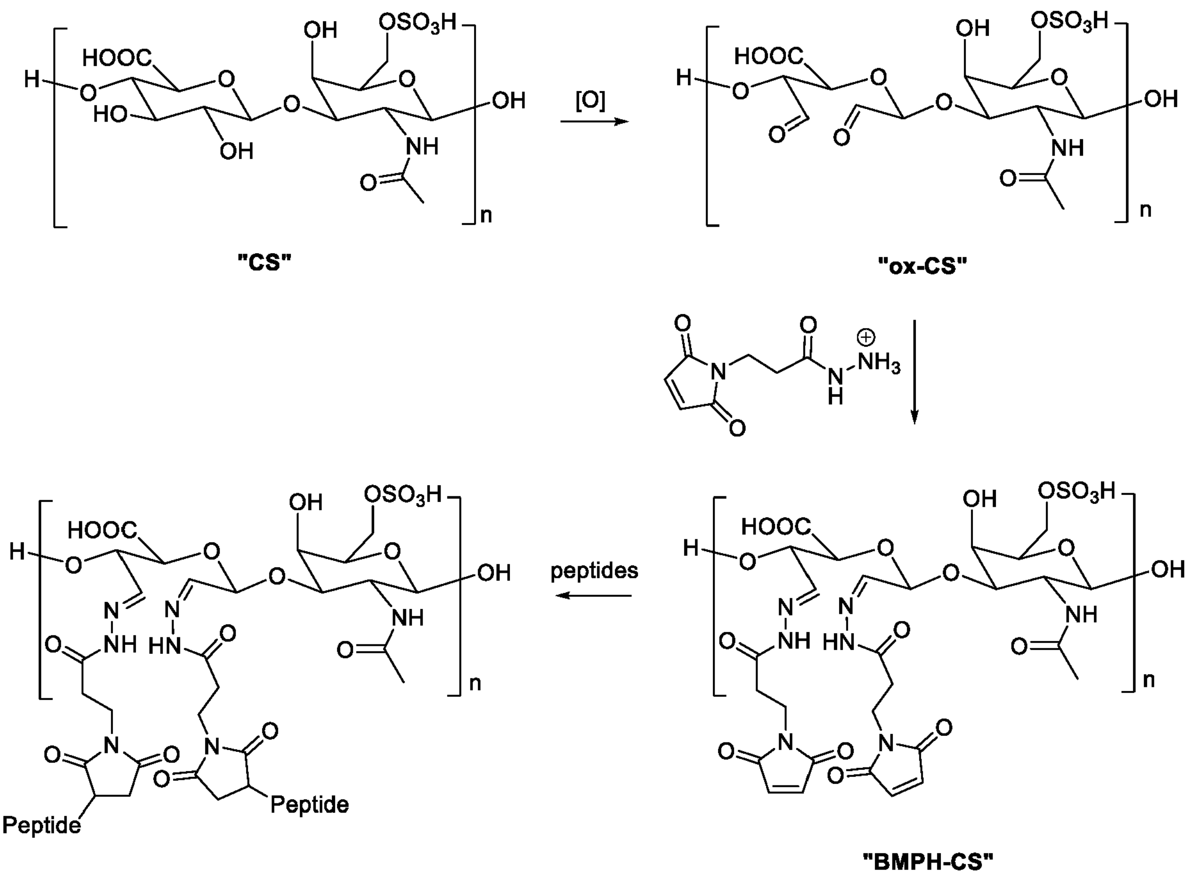


**4. Methods of Use**

**4.1 Methods of Using Collagen-Binding Bioconjugates**

The use of a collagen-binding bioconjugate in a method for inhibiting activation of platelets is described, the method comprising the step of providing a collagen-binding synthetic bioconjugate for contacting collagen wherein the collagen-binding synthetic bioconjugate binds to the collagen and wherein activation of the platelets is inhibited. The use of a collagen-binding bioconjugate in a method for inhibiting adhesion of platelets to collagen is described, the method comprising the step of providing a collagen-binding synthetic bioconjugate for contacting collagen wherein the collagen-binding synthetic bioconjugate binds to the collagen, and wherein adhesion of the platelets to collagen is inhibited. Other methods of using the collagen-binding bioconjugates are discussed below, but do not form part of the invention.

***a. Coronary Artery Disease (CAD) and Peripheral Artery Disease (PAD)***

Methods and associated compositions for improving the success rate and/or reducing failure of a surgical bypass procedure are described. Bypass grafts are used as one form of treatment of arterial blockage in both coronary artery disease (CAD) and peripheral artery disease (PAD). Approximately 500,000 coronary artery bypass graft (CABG) procedures and over 70,000 peripheral bypass graft procedures are performed annually in the US. Most commonly, an autologous vessel graft is harvested, often from the saphenous vein.

Despite the prevalence of surgical bypass with autologous vein grafts to restore blood flow, there are a large number of vein graft failures (VGF) in both CAD and PAD. In the periphery alone, vein graft failure rates reach levels of 50% failure within 5 years. While 5% to 10% of vein grafts fail shortly after implantation due to technical factors and

acute thrombosis, mid-term failure (3 to 24 months) may occur in another 20% to 30% of cases and can lead to costly surveillance, reintervention procedures and amputation. The 12-month incidence of vein graft failure in CLI patients (n = 1219) was 29% during a two-decade experience at the Brigham and Women's Hospital. The consequences of vein graft failure are often severe for the patient, including recurrent ischemic symptoms, debilitating surgery and limb loss. To date, pharmacotherapies and technical innovations have had little impact on reducing vein graft failure.

It is contemplated that injuries to the fragile endothelial layer of vein graft conduits, whether caused by vein graft harvesting, preservation media, excessive manipulation in preparation for bypass, or ischemia and reperfusion injury, result in a platelet mediated inflammatory response within the vessel wall after implantation. Such endothelial injuries and ECM-platelet activation cascade can result in early VGF via acute inflammation and thrombosis, or delayed VGF via neointimal hyperplasia. Limiting the exposure of the vein graft sub-endothelial matrix to circulating platelets after implantation, therefore, can help reduce acute vessel wall inflammation, improve re-epithelialization and limit excessive neointimal hyperplasia that may lead to vessel occlusion and VGF. The bioconjugate as described herein can be used as a vein graft preservation solution for patients with cardiovascular disease undergoing surgical bypass with autologous vein grafts. The bioconjugates, and compositions comprising the same, as described herein can be used to treat and/or prevent coronary artery disease and/or peripheral artery disease in a patient in need thereof.

Aa collagen-binding bioconjugate can be used in in a method for preparing a vascular graft (e.g., a vein graft) by contacting the internal wall of a section of a blood vessel with a solution that contains a synthetic bioconjugate of the disclosure. One way of implementing the contact is to soak the section in the solution. Conditions for this contact can vary but can be readily determined, depending on the concentration of the synthetic bioconjugate and the characteristics of the blood vessel, such that there is a suitable amount of the synthetic bioconjugate bound to the internal wall. The vascular graft prepared with such a method is also within the scope of the present disclosure.

Once the graft is prepared, it can be implanted to a patient in need thereof. The surgical bypass procedure can be readily carried out by a medical professional. Once implanted, the synthetic bioconjugate bound to the internal wall of the grant can help reduce acute vessel wall inflammation, improve re-epithelialization of the graft and limit excessive neointimal hyperplasia of the graft, resulting in reduced graft failure.

When the graft has been treated with a synthetic bioconjugate as described above, during or following the bypass procedure, a solution of the synthetic bioconjugate can be injected into the lumen of the graft such that the synthetic bioconjugate will bind to the internal wall of the graft. The injection may be done before blood flow is restored or started through the graft. Alternatively, the injection may be done shortly after (e.g., within 10 minutes, within 5 minutes, or within 1 minute) the blood flow is restored or started.

Th method is effective in inhibiting negative remodeling of the blood vessel. Coronary artery disease, also known as ischemic or coronary heart disease, occurs when part of the smooth, elastic lining inside a coronary artery (the arteries that supply blood to the heart muscle) develops atherosclerosis, effectively restricting blood flow to the heart. Peripheral arterial disease, also known as atherosclerosis or hardening of the arteries, is a disorder that occurs in the arteries of the circulatory system. Negative remodeling includes the physiologic or pathologic response of a blood vessel to a stimulus resulting in a reduction of vessel diameter and lumen diameter. Such a stimulus could be provided by, for example, a change in blood flow or an angioplasty procedure. The injection of the bioconjugates described herein, and compositions comprising the same, leads to an increase of vessel diameter by about any of 10%, 20%, 30%, 40%, 60%, 70%, 80%, 95%, or more, compared to the diameter of a vessel of without the injection. Negative remodeling can be quantified, for example, angiographically as the percent diameter stenosis at the lesion site (or disease site). Another method of determining the degree of remodeling involves measuring in-lesion external elastic lamina area using intravascular ultrasound (IVUS). IVUS is a technique that can image the external elastic lamina as well as the vascular lumen. The negative remodeling may be associated with a vascular interventional procedure, such as angioplasty, stenting, or atherectomy. The bioconjugates, and compositions comprising the same, as described herein can therefore be injected before, during and/or after the vascular interventional procedure. A method of treating stenosis, or occlusion within the femoropopliteal artery, in a patient in need thereof is described, comprising applying a solution to the internal wall of a lumen before, during and/or after a balloon angioplasty, wherein the solution comprises an effective amount of a bioconjugate as described herein or a composition comprising the same.

A method of inhibiting negative remodeling in a blood vessel (e.g., artery) in an individual in need thereof is described, comprising injecting into the blood vessel wall or tissue surrounding the blood vessel wall an effective amount of a bioconjugate as described herein or a composition comprising the same. The bioconjugate or composition may be injected at or adjacent to a site of potential or actual negative remodeling (such as no more than about 2, 1, or 0.5 cm away from the site). The nanoparticle composition may be injected remotely from a site of potential or actual negative remodeling (for example at least about any of 1, 2, 3, 4, 5, 6, 7, 8, 9, or 10 cm away from the site). The injection may be via a catheter with a needle. The site may be a coronary artery or a peripheral artery. The artery may be selected from the group consisting of renal artery, cerebral artery, pulmonary artery, and artery in the leg. The artery may be a balloon injured artery. Further examples, include, but are not limited to, abdominal aorta, anterior tibial artery, arch of aorta, arcuate artery, axillary artery, brachial artery, carotid artery, celiac artery, circumflex fibular artery, common hepatic

artery, common iliac artery, deep femoral artery, deep palmar arterial arch, dorsal digital artery, dorsal metatarsal artery, external carotid artery, external iliac artery, facial artery, femoral artery, inferior mesenteric artery, internal iliac artery, intestinal artery, lateral inferior genicular artery, lateral superior genicular artery, palmar digital artery, peroneal artery, popliteal artery, posterior tibial artery, profunda femoris artery, pulmonary artery, radial artery, renal artery, splenic artery, subclavian artery, superficial palmar arterial arch, superior mesenteric artery, superior ulnar collateral artery, and/or ulnar artery. The artery may be part of the coronary vasculature.

The bioconjugate used in the methods described above comprises heparin and from about 5 to about 10, or about 7, peptides, wherein the peptides comprise at least one sequence of RRANAALKAGELYKSILY (SEQ ID NO: 1) or RRANAALKAGEL YKSIL YGSG (SEQ ID NO: 287), and are bound to the heparin via a hydrazide-carbonyl linkage.

***b. Vascular Treatments***

The bioconjugates and compositions described herein can be used to treat a blood vessel in a patient prior to, during, and/or after a vascular injury or intervention. The vascular intervention can include, but is not limited to, angioplasty with and without stents, graft vessels, atherectomy and vascular access dysfunction, or other surgical procedure.

As described herein, a bioconjugate, or composition thereof, may be administered to a patient in need of treatment to inhibit platelet activation, such as that involved in thrombosis, platelet binding to exposed collagen of the denuded endothelium, inflammation resulting from denuding the endothelium, intimal hyperplasia, or vasospasm.

The bioconjugate can be administered intravenously or into muscle, for example. Other suitable routes for parenteral administration include intravascular, intravenous, intraarterial, intramuscular, cutaneous, subcutaneous, percutaneous, intradermal, and intraepidermal delivery. Suitable means for parenteral administration include needle (including microneedle) injectors, infusion techniques, and catheter-based delivery. The catheter-based delivery can include delivering the bioconjugate as a coating on a balloon, through a porous balloon, or as a coating on a stent. The bioconjugate can be delivered systemically (i.e., not delivered directly to the target vessel, but delivered by parenteral administration).

These bioconjugates locally bind to exposed collagen through physical peptide-collagen interactions. When bound to collagen, the bioconjugate has a number of functions including 1) acting as a barrier to platelet attachment/activation, 2) protecting collagen from degradation by inhibiting MMP access, and 3) sequestering growth factors FGF-2, FGF-7, and FGF-10, thus promoting endothelial and epithelial cell proliferation and migration.

The bioconjugates can compete for platelet binding sites on collagen and prevent platelet binding and activation. The glycan backbone of the bioconjugate can be negatively charged and bind water molecules, creating a hydrophilic barrier over the collagen surface that prevents platelet and protein adhesion. By masking the exposed collagen, rather than inhibiting normal platelet function, the bioconjugate can provide a local treatment that addresses the initial steps in the cascade to inflammation and intimal hyperplasia.

The present disclosure provides a new approach to address the unmet need of vascular access dysfunction in patients receiving hemodialysis. The approach entails generation of a luminal vessel coating designed from a bioconjugate as described herein. In arteriovenous fistula (AVF), for example, the neointimal hyperplasia mostly occurs in the venous portion of the AVF. While the initial mechanisms of intimal hyperplasia are similar in arteries and veins, there are differences in the resulting lesions. Venous neointimal hyperplasia tends to be a more aggressive lesion than arterial intimal hyperplasia in the setting of peripheral vascular disease and have poorer response to angioplasty. The ability of the disclosed bioconjugate to prevent platelet binding and intimal hyperplasia in an arterial injury is contemplated to contribute to its ability to reduce or prevent neointimal hyperplasia.

Thus, the present disclosure describes a method for improving maturation of an arteriovenous fistula (AVF) in a patient in need of hemodialysis, or alternatively for improving patency, enlarging inner diameter of the veins, reducing stenosis, reducing neointimal hyperplasia, reducing hemodynamic stress, reducing endothelial or smooth muscle cell injury, reducing vascular access dysfunction, or reducing coagulation or inflammation at the AVF. The method entails applying a solution to the internal wall of a lumen of an AVF; and restoring or initiating blood flow in the AVF, wherein the solution is a bioconjugate of the present disclosure, or the solution comprises an effective amount of a bioconjugate of the present disclosure.

A localized treatment is disclosed using a synthetic polymeric luminal coating, which binds specifically to exposed collagen, where the coating can block platelet adhesion to the vessel wall and thus inhibit the initiating events in thrombosis and intimal hyperplasia. Additionally, the coating can promote rapid re-endothelialization of the vessel wall, resulting in faster healing. It is contemplated that the application of the disclosed bioconjugate to native AV fistulas during the creation will result in fistulas with significantly less stenosis and larger diameters.

For a newly created AVF before blood flow is initiated, the solution is applied less than about 10 minutes before the blood flow is initiated. The solution may be applied less than about 20, 15, 10, 9, 8, 7, 6, 5, 4, 3, or 2 minutes, or 60, 45, 30, 20, 10 or 5 seconds before the blood flow is initiated. The solution may be applied at least 1 minute or at least 2, 3, 4, 5 minutes before the blood flow is initiated. The solution may be applied at least 1 minute or at least 2, 3, 4, 5 minutes after blood flow is restored. The blood flow may be initiated, then stopped to allow for delivery of the solution.

The solution may be applied to the vessel prior to creation of an anastomosis, during the creation of an anastomosis, or after. The solution may be applied to the vessel prior to creation of an anastomosis, during the creation of an anastomosis, and after.

The solution may be flushed through the AVF, e.g., with a needle, catheter or other drug-delivery device. The method further entails closing the AVF after the AVF is flushed with the solution. In addition to the application of the solution as described above, or alternatively, the solution is injected into an enclosed lumen generated by clamping the proximal and vein and artery of an established AVF.

The solution may be applied within about 5 minutes (or alternatively within 10, 9, 8, 7, 6, 4, 3, or 2 minutes) following vein dilation or rubbing of the vein portion of the AVF, which is used to enlarge the internal diameter of the vein. Applying the solution to the mechanically dilated or rubbed surface of the vein interior can reduce loss of the bioconjugate on the surface during rubbing.

It is further contemplated that the disclosed compositions and methods can be used for establishing a vascular access in a patient which method can entail applying a solution of the disclosure to a wall of a blood vessel in a vascular access; and restoring or initiating blood flow in the vascular access. The wall may be an internal wall of the blood vessel, but it can also be the external wall of any blood vessel.

The vascular access may be an arteriovenous fistula (AVF), an arteriovenous graft (AVG), or a durable vascular access used for parenteral nutrition, chemotherapy, or plasmapheresis. It is contemplated that the solution reduces exposure of the wall to platelets. The wall may comprises a cell or tissue exposed to blood flow due to injury or a surgical procedure. It is shown that application of the solution improves patency, improves survival, improves blood flow, enlarges vascular inner diameter, or reduces stenosis in the vascular access, such as AVF and AVG.

The present disclosure describes a method for improving maturation of an arteriovenous fistula (AVF) in a patient in need of hemodialysis, or alternatively for improving patency, enlarging inner diameter of the veins, reducing stenosis, reducing neointimal hyperplasia, reducing hemodynamic stress, reducing endothelial or smooth muscle cell injury, reducing vascular access dysfunction or reducing coagulation or inflammation at the AVF. The method may entail applying a solution to the internal wall of a lumen of an AVF; and restoring or initiating blood flow in the AVF, wherein the solution comprises an effective amount of a bioconjugate of the present disclosure.

It is contemplated that the bioconjugates may be administered to the interior of the patient's vessel percutaneously or intravenously. The percutaneous or intravenous delivery allows for treatment of a patient post-surgical fistula creation. The bioconjugates may be delivered for treatment of the vessel, maintenance of the vessel, or prevention of the failure of the fistula.

The methods may further include carrying out one or more maintenance applications, such as balloon-assisted maturation, balloon angioplasty, atherectomy, or declotting procedures. Still, , prophylactic delivery at the time of hemodialysis, especially following the procedure when especially high flow rates damage the endothelium and an injection in the graft or fistula is contemplated to be beneficial for maintenance and prevention of stenosis.

The bioconjugate used in the methods described above comprises heparin and from about 5 to about 10, or about 7, peptides, wherein the peptides comprise at least one sequence of RRANAALKAGELYKSILY (SEQ ID NO: 1) or RRANAALKAGELYKSILYGSG (SEQ ID NO: 287), and are bound to the heparin via a hydrazide-carbonyl linkage. The heparin may be unfractionated heparin (UFH) or low molecular weight heparin (LMWH).

The bioconjugate used in the methods described above comprises heparin and from about 5 to about 20% functionalization with peptides, wherein the peptides comprise at least one sequence of RRANAALKAGELYKSILY (SEQ ID NO: 1) or RRANAALKAGELYKSILYGSG (SEQ ID NO: 287), and are bound to the heparin via a hydrazide-carbonyl linkage.

### *c. **Vascular Intervention***

Vascular intervention, such as percutaneous coronary intervention, can be carried out by any conventional procedure prior to, during, or after administration of the collagen-binding synthetic bioconjugate. Examples of vascular intervention procedures contemplated for use in conjunction with the the bioconjugates include stenting, atherectomy, and angioplasty, such as balloon angioplasty. The vascular intervention procedure can be one which involves temporarily occluding the vessel (e.g., balloon angioplasty), or it can be one which does not involve temporarily occluding the vessel (e.g., non-balloon angioplasty procedures, stenting procedures that do not involve balloon angioplasty, etc.). Illustrative modes of delivery can include a catheter, parenteral administration, a coating on a ballon, through a porous ballon, a coated stent, and any combinations thereof or any other known methods of delivery of drugs during a vascular intervention procedure.

In another illustrative embodiment, during a vascular intervention procedure, any of these bioconjugates with conservative amino acid substitutions can inhibit platelet binding to exposed collagen of the denuded endothelium, platelet activation, thrombosis, inflammation resulting from denuding the endothelium, intimal hyperplasia, and/or vasospasm, or can stimulate endothelial cell proliferation or can bind to collagen in a denuded vessel. In another illustrative

embodiment, during a vascular intervention procedure, any of the bioconjugates with conservative amino acid substitutions described in this paragraph can inhibit platelet binding to exposed collagen of the denuded endothelium, platelet activation, intimal hyperplasia, and/or vasospasm, or can bind to collagen in a denuded vessel.

A collagen-binding synthetic bioconjugate may be administered to a patient (e.g., a patient in need of treatment to inhibit platelet activation, such as that involved in thrombosis, platelet binding to exposed collagen of the denuded endothelium, thrombosis, inflammation resulting from denuding the endothelium, intimal hyperplasia, or vasospasm). In various embodiments, the collagen-binding synthetic bioconjugate can be administered intravenously or into muscle, for example. Suitable routes for parenteral administration include intravascular, intravenous, intraarterial, intramuscular, cutaneous, subcutaneous, percutaneous, intradermal, and intraepidermal delivery. Suitable means for parenteral administration include needle (including microneedle) injectors, infusion techniques, and catheter-based delivery. In an illustrative embodiment, pharmaceutical formulations for use with collagen-binding synthetic bioconjugates for parenteral administration or catheter-based delivery comprising: a) a pharmaceutically active amount of the collagen-binding synthetic bioconjugate; b) a pharmaceutically acceptable pH buffering agent to provide a pH in the range of about pH 4.5 to about pH 9; c) an ionic strength modifying agent in the concentration range of about 0 to about 300 millimolar; and d) water soluble viscosity modifying agent in the concentration range of about 0.25% to about 10% total formula weight or any individual component a), b), c), or d) or any combinations of a), b), c) and d) are provided.

As described herein, the collagen-binding synthetic bioconjugate can be administered intravascularly into the patient (e.g., into an artery or vein) in any suitable way. The collagen-binding synthetic bioconjugate can be administered into a vessel of a patient prior to, during, or after vascular intervention. Vascular interventions, such as percutaneous coronary intervention (PCI), can include, for example, stenting, atherectomy, grafting, and angioplasty, such as balloon angioplasty. Illustratively, the vascular intervention can be one which involves temporarily occluding an artery, such as a coronary artery or a vein (e.g., balloon angioplasty), or it can be one which does not involve temporarily occluding an artery or a vein (e.g., non- balloon angioplasty procedures, stenting procedures that do not involve balloon angioplasty, etc.). Illustrative modes of delivery can include a catheter, parenteral administration, a coating on a balloon, through a porous balloon, a coated stent, and any combinations thereof or any other known methods of delivery of drugs during a vascular intervention procedure. The target vessel can include a coronary artery, e.g., any blood vessel which supplies blood to the heart tissue of a patient, including native coronary arteries as well as those which have been grafted into the patient, for example, in an earlier coronary artery bypass procedure. Tthe target vessel into which the collagen-binding synthetic bioconjugate is to be administered and on which the vascular intervention procedure is to be performed may contain a blockage, such as a stenosis or some other form of complete or partial blockage which causes reduced blood flow through the vessel. Thus, the collagen-binding synthetic bioconjugate can be delivered to the vessel via a catheter (e.g., a dilatation catheter, an over-the-wire angioplasty balloon catheter, an infusion catheter, a rapid exchange or monorail catheter, or any other catheter device known in the art) which is percutaneously inserted into the patient and which is threaded through the patient's blood vessels to the target vessel. Various catheter-based devices are known in the art, including those described in U.S. Patent No. 7,300,454.Where a catheter is used, the catheter used to deliver the collagen-binding synthetic bioconjugate can be the same catheter through which the vascular intervention is to be performed, or it can be a different catheter (e.g., a different catheter which is percutaneously inserted into the patient via the same or a different cutaneous incision and/or which is threaded through the patient's blood vessels to the target vessel via the same or a different route). The collagen-binding synthetic bioconjugate can be injected directly into the target vessel. The collagen-binding synthetic bioconjugate can be delivered systemically (i.e., not delivered directly to the target vessel, but delivered by parenteral administration without catheter-based delivery).

In the case where the vessel contains a blockage (e.g., a stenosis), administration can be carried out by delivering the collagen-binding synthetic bioconjugate directly to the target vessel at the site of the blockage or distal to the blockage or both. The collagen-binding synthetic bioconjugate can be delivered to one or more sites proximal to the blockage. Illustratively, the catheter tip can be maintained stationary while the collagen-binding synthetic bioconjugate is being delivered, or the catheter tip can be moved while the collagen-binding synthetic bioconjugate is being delivered (e.g., in a proximal direction from a position that is initially distal to the blockage, to or through the blockage, or to a position which is proximal to the blockage).

As indicated above, the collagen-binding synthetic bioconjugate can be administered directly into the patient's vessel at a time prior to vascular intervention, e.g., percutaneous coronary intervention. For example, delivery of the collagen-binding synthetic bioconjugate can be carried out just prior to vascular intervention (e.g., within about 1 hour, such as within about 30 minutes, within about 15 minutes, and/or within about 5 minutes prior to vascular intervention). Optionally, delivery of the collagen-binding synthetic bioconjugate directly to the target vessel can be continued during all or part of the vascular intervention procedure and/or subsequent to completion of such procedure, or delivery of the collagen-binding synthetic bioconjugate directly to the target vessel can be stopped prior to the commencement of the vascular intervention procedure and not subsequently recommenced. Delivery of the collagen-binding synthetic bioconjugate can be continuous or it can be effected through a single or multiple administrations. Prior to, during, and/or after the collagen-binding synthetic bioconjugate is administered to the target vessel, the same collagen-binding synthetic

bioconjugate or one or more different collagen-binding synthetic bioconjugates can be administered.

The bioconjugate used in the methods described above comprises heparin and from about 5 to about 10, or about 7, peptides, wherein the peptides comprise at least one sequence of RRANAALKAGELYKSILY (SEQ ID NO: 1) or RRANAALKAGELYKSILYGSG (SEQ ID NO: 287), and are bound to the heparin via a hydrazide-carbonyl linkage.

### *d. Endothelial Dysfunction*

The present disclosuredescribes compositions and their use in methods for treating a patient suffering from a disease associated with endothelial dysfunction. The compositions include a synthetic collagen binding bioconjugate of the present disclosure.

It is discovered herein that collagen binding bioconjugates can reduce the inflammatory impact of endothelial dysfunction or injury, in both acute and chronic diseases. It is contemplated that such bioconjugates inhibit or reduce platelet binding to the dysfunctional endothelium and thus reduce platelet-mediated inflammation. Inflammation can be activated through platelet processes such as platelet-platelet binding, platelet-leukocyte binding, facilitation of leukocyte diapedesis, or simply release from platelets of local and regional cytokines.

Further, it is discovered that collagen binding bioconjugates decrease pro-inflammatory cytokine secretion and the expression of E-selectin and P-selectin in the exposed endothelial cells. Moreover, these bioconjugates can increase endothelial cell proliferation and migration, attenuate IL-6 secretion and the production of vascular injury markers, even in the presence of platelet-derived growth factor (PDGF). It is contemplated that some or all of these effects brought about by the administration of collagen binding bioconjugates contribute to the reduction of inflammatory at dysfunctional endothelium.

A method for preventing or reducing inflammation at a vascular site suffering from endothelial dysfunction is described. The method entails administering to the site a pharmaceutical composition that includes a synthetic collagen binding bioconjugate of the present disclosure.

The term "endothelial dysfunction" is also referred to as "endothelial cell (EC) dysfunction," "dysfunctional endothelium," or "dysfunctional endothelial cells." Endothelial dysfunction can be determined with unmasking or exposure of ICAM and VCAM receptors or selectin receptors on the cell surface of an endothelial cell. P-selectin and E-selectin are examples of selectin receptors exposed which are transiently expressed on the cell surface due to damage and inflammation, and chronically expressed in dysfunctional endothelium.

The endothelial dysfunction may be characterized with permeated endothelial lining or damaged endothelial cells. The endothelial dysfunction may be characterized by loss of glycocalyx. The endothelial dysfunction may be characterized by a selectin protein expressed on the surface of endothelial cells and exposed to circulation. The site may suffer from inflammation.

The vascular site may not be denuded by physical means and is not undergoing to recovering from a vascular intervention procedure. Non-limiting examples of vascular intervention procedures include percutaneous coronary intervention (PCI).

A "dysfunctional endothelial cell" or "endothelial cell (EC) dysfunction" means the unmasking or exposure of ICAM and VCAM receptors, as well as, selectin receptors on the cell surface of an endothelial cell. P-selectin and E-selectin are examples of selectin receptors exposed which are transiently expressed on the cell surface due to damage and inflammation, and chronically expressed in dysfunctional endothelium. An example of a disease state with chronic dysfunctional endothelial cells is diabetes.

Dysfunction of the endothelium plays an important role in the pathogenesis of a broad spectrum of diseases as endothelial cells participate in the maintenance of functional capillaries.

For instance, the endothelium is directly involved in peripheral vascular disease, stroke, heart disease, diabetes, insulin resistance, chronic kidney failure, tumor growth, metastasis, venous thrombosis, and severe viral infectious diseases (Rajendran et al., Int. J. Biol. Sci., 9:1057-1069, 2013).

A "disease associated with endothelial dysfunction," as used herein, refers to a human disease or condition that is at least in part caused by endothelial dysfunction or that induces endothelial dysfunction. Treating a disease associated with endothelial dysfunction, accordingly, refers to the treatment of the disease, recovering the dysfunctional endothelium, or preventing or ameliorating conditions or symptoms arising from dysfunctional endothelium, such as inflammation, intimal hyperplasia, and thrombosis.

The present inventors have demonstrated that collagen binding bioconjugates can be effectively delivered to any organ of a human patient. Therefore, the collagen binding bioconjugates can be used to treat endothelial dysfunction that occurs at any of the organs and associated with any of the following diseases or conditions.

*Vascular diseases.* Vascular diseases that can be suitably treated with collagen binding bioconjugates include, without limitation, atherosclerotic diseases (peripheral artery disease, coronary artery disease, stroke, carotid artery disease, renal arterial stenosis), venous thrombotic diseases (deep or superficial vein thrombosis), and iatrogenic large vessel injury (angioplasty, angioplasty with stent placement, atherectomy, thrombectomy, dialysis access creation, vein

harvesting for bypass, treatment of brain or aortic aneurysms).

*Renal diseases.* Renal diseases that can be suitably treated with collagen binding bioconjugates include, without limitation, acute tubular necrosis, diabetic chronic renal failure, lupus nephritis, renal fibrosis, and acute glomerulonephritis.

*Pulmonary diseases.* Pulmonary diseases that can be suitably treated with collagen binding bioconjugates include, without limitation, idiopathic pulmonary fibrosis (IPF), chronic obstructive pulmonary disease, asthma, and emphysema.

*Hematological diseases.* Hematological diseases that can be suitably treated with collagen binding bioconjugates include, without limitation, thrombotic thrombocytopenic purpura (TTP), disseminated intravascular coagulation (DIC), and hemolytic uremic syndrome (HUS).

Additionally, dermal diseases such as systemic sclerosis, rheumatologic diseases including vasculitic disorders (lupus), rheumatoid arthritis and other inflammatory arthritis (gout), gastrointestinal diseases including inflammatory bowel disease, hepatitis, and hepatic fibrosis, tumor growth, tumor metastasis, infectious diseases including viral and bacterial sepsis, neurologic diseases including multiple sclerosis, dementia, and amyotrophic lateral sclerosis, ophthalmologic diseases including macular degeneration, glaucoma, and uveitis, endocrinological diseases such as diabetes, and complex regional pain syndrome (CRPS) can also be treated with collagen binding bioconjugates of the present disclosure.

It is contemplated that the bioconjugates can be tailored with respect to the peptide identity, the number of peptides attached to the glycan, and the GAG backbone identity for optimized treatment depending on the disease to be treated and location of the affected dysfunctional endothelium. Thus, a number of molecular design parameters can be engineered to optimize the target effect.

The bioconjugate may comprise dermatan sulfate (DS) with attached collagen binding peptide(s). DS may be useful because of its ability to promote epithelial cell migration and proliferation.

It is contemplated that other variants of bioconjugate provided herein are also capable of inhibiting inflammation at dysfunctional endothelium. The bioconjugates may include a collagen binding peptide such as RRANAALKAGELYKSILY (SEQ ID NO: 1), referred to as "SILY".

***e. Tissue Adhesion***

The methods described herin are useful in a variety of applications related to tissue adhesions, such as cardiac, abdominal or pelvic adhesion. It is contemplated that the methods using the bioconjugates described would be useful in treating and/or preventing these persistent defects or recurrent injury.

The bioconjugates may be used in a method of treating and/or preventing abdominal or pelvic adhesion in a patient in need thereof, comprising applying a pharmaceutical composition on an unnaturally exposed tissue of an organ. The composition comprises a bioconjugate comprising a glycan having from about 1 to about 80 collagen binding peptide(s) bonded to the glycan. "Exposed tissue" can refer to tissue or a surface that is exposed to a new environment that is seen under normal, healthy conditions, or to tissue that is not exposed to cells or tissue of a different organ under normal, healthy conditions, but is exposed due to disease, or injury, or during a medical procedure (i.e., unnaturally exposed tissue").

An adhesion is a band of fibrous scar tissue that abnormally binds tissues and/or organs that are not normally connected. Adhesions develop in response to various types of injury or tissue disturbances, for example, such as surgery, trauma, infection, chemotherapy, radiation, foreign body, or cancer.

Abdominal and pelvic adhesions are a common complication of abdominal surgical procedures. Abdominal adhesions can cause severe clinical problems and/or pain. For example, abdominal adhesion-related clinical problems may include small-intestinal obstruction, secondary female infertility, ectopic gestation, chronic abdominal and pelvic pain, and difficult and hazardous re-operations (Diamond, M. P., Freeman, M. L. Eur. Soc. Human. Repro. Embryo. 2001; 7(6): 567-576). Abdominal adhesions may cause pain by tethering tissues and/or organs not normally connected and causing traction of nerves. If the bowel becomes obstructed then distention will causes pain. Accordingly, abdominal adhesions may cause intestinal disturbances and bowel obstruction or blockage. In extreme cases, abdominal adhesions may form fibrous bands around a segment of an intestine which constricts blood flow and leads to tissue death.

Standard treatment of abdominal and pelvic adhesions that cause the above clinical problems and/or pain is surgical intervention. However, surgical intervention carries the risk of additional abdominal adhesions and further complications. Therefore, alternative treatment and/or prevention options for abdominal adhesions would be beneficial in treating and/or preventing abdominal adhesions in patients in need thereof.

Trauma to the abdominal tissue or organs can result in fibrous tissue band formation between abdominal tissues and/or organs. It is contemplated that the use of the bioconjugates in the methods described herein would be useful in treating and/or preventing said abdominal adhesion.

It is contemplated that the synthetic bioconjugates provided herein will provide a protective hydrating layer to

minimize pain, protect abdominal tissue and/or organ collagen from degradation, and promote epithelial migration and epithelial proliferation.

The bioconjugates described herein may be used in a method of treating and/or preventing abdominal adhesion in a patient in need thereof, comprising applying a pharmaceutical composition on exposed tissue of an abdominal organ. The composition may comprise a bioconjugate comprising a glycan having from about 1 to about 80 collagen binding peptide(s) bonded to the glycan.

The bioconjugates described herein may be used in a method of treating and/or preventing tendon - tendon sheath adhesion in a patient in need thereof, comprising applying a bioconjugate or composition as described herein to an unnaturally exposed tendon and/or tendon sheath.

The bioconjugates described herein may be used in a method of treating and/or preventing cardiac adhesion in a patient in need thereof, comprising applying a bioconjugate or composition as described herein to an unnaturally exposed cardiac tissue.

The tissue may be exposed due to surgery, trauma, infection, chemotherapy, radiation, foreign body, or cancer. The tissue may be surgically exposed.

The composition may be applied as a spray. The tissue may be a peritoneal membrane tissue.

The compositions are also contemplated to be useful in methods for reducing or preventing orthopedic adhesions, such as during hand or finger surgeries.

The bioconjugates may also be useful in methods which can further include other methods known in the art in reducing or preventing adhesion, such as the use of a mesh surrounding a tissue.

The bioconjugates provided herein can be used in methods to treat and/or prevent abdominal adhesion in a patient in need thereof by administering to the patient a synthetic bioconjugate that targets extracellular matrix components of the abdominal tissues and/or organs. It is contemplated that the synthetic bioconjugates provided herein can be tailored with respect to the peptide identity, the number of peptides attached to the glycosaminoglycan (GAG) backbone, and the GAG backbone identity to promote abdominal tissue vascularization. Thus, a number of molecular design parameters can be engineered to optimize the target effect.

The bioconjugates provided herein can be used as an adjunct in surgery to prevent or reduce tissue adhesion. During surgery, the synthetic bioconjugates can be delivered to the tissues or organs that are potentially adhesiogenic. It is contemplated that such an administration will help in preventing and/or reducing the post-operative adhesions. The bioconjugates described herein may be used in a method for decreasing or preventing post-surgical adhesions, wherein the method comprises delivering the synthetic bioconjugates provided herein to a surgical site. The bioconjugates provided herein can be useful in abdominal procedures such as laparoscopic abdominal surgery. The bioconjugates provided herein can be delivered through a laparoscope to the tissues or organs that are potentially adhesiogenic. It is contemplated that the treatment with the synthetic bioconjugate DS-SILY will treat and/or prevent abdominal adhesion by binding to the area of injury, providing a protective hydrating layer to minimize pain, protecting abdominal tissue and/or organ collagen from degradation, and promoting epithelial migration and epithelial proliferation. It is further contemplated that the DS-SILY will persist in the injured area so that multiple treatments per day are not necessary.

The peptidoglycan may comprise dermatan sulfate (DS) with attached collagen binding peptide(s). DS may be useful in abdominal adhesion applications because of its ability to promote epithelial cell migration and proliferation.

It is contemplated that other variants of bioconjugate provided herein are also capable of inhibiting platelet activation through binding to type I collagen. The synthetic bioconjugates provided may be used to treat and/or prevent abdominal adhesion by enabling the glycan portion of the peptidoglycan to be tethered to the site of injury through the collagen binding peptide(s) (e.g., RRANAALKAGELYKSILY (SEQ ID NO: 1), referred to as "SILY").

The bioconjugate may comprise collagen binding peptide(s) (e.g., SII,Y) conjugated to glycan comprising heparin (Hep-SII,Y), dermatan sulfate (DS-SII,Y), or dextran (Dex-SILY).

The compositions of the present disclosure can be administered during open surgery or via a Laparoscope or via any instrument that allows for access to the surgical site.

### *f. Gastro-Esophageal Injury*

The present disclosure describes a new approach to address the unmet need of treating or preventing a gastro-esophageal injury in a patient. In general, the new approach entails applying a pharmaceutical composition that includes a synthetic collagen-binding bioconjugate of the present disclosure on the injured gastro-esophageal tissue or cell.

Such application of the composition can generate a coating of the synthetic collagen-binding bioconjugate. The synthetic collagen-binding bioconjugate can bind to collagen exposed on the esophageal tissue through physical peptide-collagen interactions. When bound to collagen, the bioconjugate has a number of functions including 1) acting as a barrier to platelet attachment/activation, 2) protecting collagen from degradation by inhibiting MMP access, and 3) sequestering growth factors FGF-2, FGF-7, and FGF-10, thus promoting endothelial and epithelial cell proliferation and migration, leading to tissue repair and recovery.

The collagen-binding bioconjugateincludes a polysaccharide backbone with covalently attached collagen-binding peptides. The synthetic bioconjugates can compete for platelet binding sites on collagen and prevent platelet binding and activation. The glycan backbone can be negatively charged and bind water molecules, creating a hydrophilic barrier over the collagen surface that prevents platelet and protein adhesion. By masking the exposed collagen, rather than inhibiting normal platelet function, the bioconjugate can provide a local treatment that addresses the initial steps in the cascade to inflammation and intimal hyperplasia.

This new approach is contemplated to be useful for treating gastro-esophageal injuries, including but not limited to those caused by GERD or iatrogenic interventions. It is further contemplated that patients of the following categories can benefit from this approach:

- GERD associated esophageal lesion requiring esophagogastroduodenoscopic (EGD) ablation
- Esophageal stricture requiring EGD dilation
- Peptic Ulcer Disease (PUD) requiring EGD treatment.

The pharmaceutically composition can be topically applied to one or more lesions of the injured gastro-esophageal tissue. Given the limited accessibility of the tissue, it is contemplated that use of a delivery device is beneficial. For instance, the composition can be delivered during an esophagogastroduodenoscopy (EGD) procedure or using an esophagogastroduodenoscope.

Palifermin is a keratinocyte growth factor useful for oral mucositis treatment. The bioconjugate of present disclosure binds to collagen and also binds to endogenous or exogenous growth factors such as Palifermin. Therefore, such a formulation provides targeted delivery of Palifermin. This disclosure provides bioconjugates which can be used in a method for delivering Palifermin. The method comprises applying a composition comprising bioconjugate and Palifermin to a patient in need thereof. This disclosure provides bioconjugates which can be used in a method for treating oral mucositis in a patient wherein the method comprises applying a composition comprising a bioconjugate and Palifermin to the patient in need thereof.

It is contemplated that the bioconjugates provided in the solution can be tailored with respect to the peptide identity, the number of peptides attached to the glycan, and the GAG backbone identity to promote recovery of an injured gastro-esophageal tissue. Thus, a number of molecular design parameters can be engineered to optimize the target effect.

The bioconjugate may comprise dermatan sulfate (DS) with attached collagen binding peptide(s). DS may be useful because of its ability to promote epithelial cell migration and proliferation.

It is contemplated that other variants of bioconjugate provided herein are also capable of inhibiting platelet activation through binding to type I collagen. The bioconjugates may include a collagen binding peptide, such as RRANAALKAGELYKSILY (SEQ ID NO: 1), referred to as "SILY". The bioconjugate may comprise collagen binding peptide(s) (SILY) conjugated to glycans such as heparin (Hep-SII,Y), dermatan sulfate (DS-SII,Y), or dextran (Dex-SILY).

The bioconjugate may comprise heparin and from 1 to 5 branched collagen binding peptides, such as (GQLYKSILY)$_4$-(KRR)$_2$-KGSG (SEQ ID NO: 391).

### *g. Wound Healing*

The compositions described herein can be used in methods to treat any condition where the integrity of tissue is damaged, including chronic wounds and acute wounds, wounds in connective tissue, and wounds in muscle, bone and nerve tissue. A "wound", as used herein includes surgical incisions, burns, acid and alkali burns, cold burn (frostbite), sun burn, ulcers, pressure sores, cuts, abrasions, lacerations, wounds caused by physical trauma, wounds caused by congenital disorders, wounds caused by periodontal disease or following dental surgery, and wounds associated with cancerous tissue or tumors. As described herein, wounds can include either an acute or a chronic wound.

Acute wounds are caused by external damage to intact skin and include surgical wounds, bites, burns, cuts, lacerations, abrasions, etc. Chronic wounds include, for example, those wounds caused by endogenous mechanisms that compromise the integrity of dermal or epithelial tissue, e.g., leg ulcers, foot ulcers, and pressure sores. The compositions for promoting wound healing or decreasing scar formation may be used at any time to treat chronic or acute wounds. For example, acute wounds associated with surgical incisions can be treated prior to surgery, during surgery, or after surgery to promote wound healing and/or decrease scar formation in a patient. The compositions as herein described can be administered to the patient in one dose or multiple doses, as necessary to promote wound healing and/or to decrease scar formation.

As used herein, "decreasing scar formation" includes an increase in the ultimate tensile strength of the scar and/or a decrease in the visible scar length. As used herein, a decrease in scar formation also includes complete inhibition of scar formation or complete elimination of visible scarring in a patient.

As used herein, "promoting wound healing" means causing a partial or complete healing of a chronic or an acute wound, or reducing any of the symptoms caused by an acute or a chronic wound. Such symptoms include pain, bleeding, tissue necrosis, tissue ulceration, scar formation, and any other symptom known to result from an acute or a chronic wound.

The use of the bioconjugates in a method of promoting wound healing is described. The method comprises the step of administering to the patient a collagen-binding synthetic bioconjugate, wherein the collagen -binding synthetic bioconjugate promotes healing of a wound in the patient. The collagen-binding synthetic bioconjugate can be an aberrant collagen-binding synthetic bioconjugate or a fibrillogenic collagen-binding synthetic bioconjugate with amino acid homology to a portion of the amino acid sequence of a bioconjugate that normally regulates collagen fibrillogenesis.

The use of the bioconjugates in a method of decreasing scar formation is described. The method comprises the steps of administering to the patient a collagen-binding synthetic bioconjugate, wherein the collagen-binding synthetic bioconjugate decreases scar formation in the patient. The collagen-binding synthetic bioconjugate can be an aberrant collagen-binding synthetic bioconjugate or a fibrillogenic collagen-binding synthetic bioconjugate with amino acid homology to a portion of the amino acid sequence of a bioconjugate that normally regulates collagen fibrillogenesis.

The compositions for promoting wound healing and/or decreasing scar formation can be impregnated into any materials suitable for delivery of the composition to the wound, including cotton, paper, non-woven fabrics, woven fabrics, and knitted fabrics, monofilaments, films, gels, sponges, etc. For example, surgical sutures (monofilaments, twisted yarns or knitting yarns), absorbent pads, transdermal patches, bandages, burn dressings and packings in the form of cotton, paper, non-woven fabrics, woven fabrics, knitted fabrics, films and sponges can be used.

## 4.3 Methods of Using Hyaluronic Acid-Binding Bioconjugates

### *a. Cartilage Replacement*

The use of the hyaluronic acid binding conjugates as an additive for a biomaterial cartilage replacement composition is described . The additive comprises a hyaluronic acid-binding synthetic bioconjugate for addition to an existing biomaterial cartilage replacement material. The previously described embodiments of the hyaluronic acid-binding synthetic bioconjugate are applicable to the additive described herein.

As used herein, the phrase "existing biomaterial cartilage replacement material" means a biologically compatible composition that can be utilized for replacement of damaged, defective, or missing cartilage in the body. Various types of existing biomaterial cartilage replacement compositions are well-known in the art and are contemplated. For example, existing biomaterial cartilage or bone replacement compositions include the DeNovo® NT Natural Tissue Graft (Zimmer), MaioRegen™ (JRI Limited), or the collection of cryopreserved osteoarticular tissues produced by Biomet.

The use of the hyaluronic acid binding conjugates in a method of preparing a biomaterial or bone cartilage replacement is described. The method comprises the step of combining the synthetic bioconjugate and an existing biomaterial or bone cartilage replacement material. The previously described embodiments of the hyaluronic acid-binding synthetic bioconjugate are applicable to the method described herein.

The use of the hyaluronic acid binding conjugates in a method of treatment for arthritis in a patient is provided. The method comprises the step of administering to the patient a hyaluronic acid-binding synthetic bioconjugate, wherein the synthetic bioconjugate reduces one or more symptoms associated with arthritis. The previously described embodiments of the hyaluronic acid-binding synthetic bioconjugate are applicable to the method described herein.

The synthetic bioconjugate used in the method of treatment for arthritis reduces one or more symptoms associated with arthritis. Various symptoms are known in the art to be associated with arthritis, including but not limited to pain, stiffness, tenderness, inflammation, swelling, redness, warmth, and decreased mobility. The symptoms of arthritis may be present in a joint, a tendon, or other parts of the body. As used herein, "reducing" means preventing or completely or partially alleviating a symptom of arthritis.

The arthritis may be osteoarthritis or rheumatoid arthritis. The pathogenesis and clinical symptoms of osteoarthritis and rheumatoid arthritis are well- known in the art. In one embodiment, the synthetic bioconjugate acts as a lubricant following administration or prevents loss of cartilage. In another embodiment, the synthetic bioconjugate prevents articulation of bones in the patient. For example, the synthetic bioconjugate inhibits bone on bone articulation in a patient with reduced or damaged cartilage.

The use of the hyaluronic acid binding conjugates in a method of reducing or preventing degradation of ECM components in a patient is provided. For example, the use in a method of reducing or preventing degradation of ECM components in the cartilage of a patient is provided. The method comprises administering to the patient a hyaluronic acid-binding synthetic bioconjugate. The previously described embodiments of the hyaluronic acid-binding synthetic bioconjugate are applicable to the method described herein. In one embodiment, the synthetic bioconjugate is resistant to matrix metallo proteases, e.g., an aggrecanase.

In another embodiment, the use of the hyaluronic acid binding conjugates in a method of reducing or preventing

hyaluronic acid degradation in a patient is described. The method comprises administering to the patient a hyaluronic acid-binding synthetic bioconjugate. The previously described embodiments of the hyaluronic acid-binding synthetic bioconjugate are applicable to the method described herein.

In another embodiment, the use of the hyaluronic acid binding conjugates in a method of reducing or preventing collagen degradation is described. The method comprises the steps of contacting a hyaluronic acid-binding synthetic bioconjugate with hyaluronic acid in the presence of collagen, and reducing or preventing collagen degradation. The previously described embodiments of the hyaluronic acid-binding synthetic bioconjugate are applicable to the method described herein.

In another embodiment, the use of the hyaluronic acid binding conjugates in a method of reducing or preventing chondroitin sulfate degradation is described. The method comprises the steps of contacting a hyaluronic acid- binding synthetic bioconjugate with hyaluronic acid in the presence of collagen, and reducing or preventing chondroitin sulfate degradation. The previously described embodiments of the hyaluronic acid-binding synthetic bioconjugate are applicable to the method described herein.

"Reducing ECM component degradation", e.g., hyaluronic acid, collagen, or chondroitin sulfate degradation, means completely or partially reducing degradation of hyaluronic acid, collagen, or chondroitin sulfate, respectively.

"Reducing hyaluronic acid degradation" in a patient means reducing the rate of hyaluronic acid degradation. "Reducing collagen degradation" means reducing the rate of collagen degradation. "Reducing chondroitin sulfate" degradation means reducing the rate of chondroitin sulfate degradation.

The use of the hyaluronic acid binding conjugates in a method for correcting or modifying a tissue defect in a patient is described. The method comprises administering into the tissue defect hyaluronic acid and a hyaluronic acid-binding synthetic bioconjugate wherein the defect is corrected or modified. The previously described embodiments of the hyaluronic acid-binding synthetic bioconjugate are applicable to the method described herein. The tissue defect may be a cosmetic defect.

the bioconjugate used in the methods described above may comprise chondroitin sulfate and about 5 to about 10 peptides, wherein the peptides comprise at least one sequence of GAHWQFNALTVR (SEQ ID NO: 58) or GAHWQFNALTVRGSG (SEQ ID NO: 357), and are bound to the chondroitin sulfate via a hydrazide-carbonyl linkage.

### 4.4 Other uses in methods of treatment which do not form part of the invention

#### *a. Corneal Wounds*

The bioconjugates described herein can be used in methods related to corneal wound healing. The corneal wound condition in need of treatment may be as a result of traumatic injury to the cornea (Chiapella, A. P., Rosenthal, A. R. British Journal of Ophthalmology, 1985; 69: 865-870). In another embodiment, the wound condition in need of treatment is caused by an ophthalmologic procedure such as Epi-Lasik induce corneal injury (Tuft, S.J., et al. Br J Ophthalmol. 1993; 77: 243-247). In some cases persistent defects or recurrent injury can occur due to lack of or incomplete healing (Kenyon, K. R. Cornea and Refractive Atlas of Clinical Wisdom. Eds. S.A. Melki and M. A. Fava. SLACK, Inc.: New Jersey, US, 2011; pp. 39). It is contemplated that the bioconjugates described herein may be used in methods for treating these persistent defects or recurrent injury.

The injury to the corneal epithelium may result in a breach in the corneal barrier function and it is contemplated that the methods described herein would be useful in treating said injury.

The bioconjugates provided herein can be used to promote corneal wound healing in a patient in need thereof by administering to the patient a bioconjugate that targets specific extracellular matrix components implicated in corneal wound healing. It is contemplated that the bioconjugates provided herein can be tailored with respect to the peptide identity, the number of peptides attached to the glycan, and the glycan identity to promote corneal wound healing. Thus, a number of molecular design parameters can be engineered to optimize the target effect. It is contemplated that the treatment with a bioconjugate comprising dermatan sulfate and collagen binding peptide(s) (e.g., RRANAALKAGELYKSILY (SEQ ID NO: 1), referred to as "SILY") will enhance corneal would healing by binding to the area of injury, providing a protective hydrating layer to minimize pain, protecting corneal collagen from degradation, and/or promoting epithelial migration and/or epithelial proliferation. It is further contemplated that the bioconjugate will persist in the injured area so that multiple treatments per day are not necessary.

#### *b. Lubricin Mimetic*

The synthetic bioconjugates described herein may be useful in replacing, rejuvenating, or supplementing tissues that have both collagen and hyaluronic acid, such as cartilage, synovial fluid, and the vitreous humor. In particular, peptidoglycans having both collagen-binding and hyaluronic acid binding peptides may be especially useful as a lubricin mimetic and in the methods and uses described below.

*Cartilage degeneration.* A well-lubricated surface on articular cartilage leads to optimal functionality of synovial joints. As occurs in osteoarthritis, however, a reduced lubrication results in cartilage degradation and fibrillation, which in turn contribute to joint dysfunction and pain. Reduced lubrication also leads to joint dysfunction and pain in other forms of arthritis, including rheumatoid arthritis.

The synthetic bioconjugates provided herein can be used to mimic some of the functions of lubricin, a mucinous glycoprotein secreted by tissues lining the interior surfaces of animal joints. The synthetic bioconjugate thus has the potential to enhance lubrication at an articular cartilage surface, thereby reducing wear-induced erosion of the cartilage. The synthetic bioconjugate also has the potential to protect macromolecules, like hyaluronic acid and type II collagen, from enzyme-induced degradation.

Accordingly, is the bioconjugates described here can be used in a method of treating and/or preventing cartilage degeneration in a patient comprising administering to a patient in need thereof a pharmaceutical composition comprising the extracellular matrix-binding synthetic bioconjugate described herein. The patient may be treated by injecting the pharmaceutical composition comprising the extracellular matrix-binding synthetic bioconjugate into a synovial cavity.

It is also contemplated that the synthetic bioconjugates can be used to treat and/or prevent articular cartilage disease by protecting the articular cartilage matrix from traumatic and cytokine-induced enzymatic degradation.

*Vitreous humor degeneration.* The vitreous humor is a viscoelastic, gel-like substance that fills the posterior cavity of the eye. Vitreous replacements have been used to replace a dysfunctional vitreous humor, for example, in cases where opacification or the physical collapse and liquefaction of the vitreous has occurred, and as a temporary or permanent vitreous replacement during retinal surgery. A suitable vitreous replacement should be transparent, biocompatible, and have a density and refractive index close to the natural vitreous.

The bioconjugates described here can be used in a method of treating and/or preventing vitreous humor degeneration in a patient comprising administering to a patient in need thereof a pharmaceutical composition comprising the extracellular matrix-binding synthetic bioconjugate described herein.

*Nucleus pulposus degeneration.* The nucleus pulposus is a gel-like substance present in spinal discs, and functions to distribute hydraulic pressure in all directions within each disc under compressive loads and is comprised of chondrocyte-like cells, collagen fibrils, and bioconjugate aggrecans that aggregate through hyaluronic chains. Degeneration of the nucleus pulposus results in reduced ability of the disc to transmit loads evenly and efficiently between vertebral bodies, and leads to damage in the annular region of the disc, known as the annulus fibrosis. Fissures or tears in the annulus can translate into a disc that herniates or ruptures, resulting in impingement of the nerves in the region of the disc and finally lower back or leg pain.

Attempts have also been made to replace only the nucleus pulposus. Replacement of the nucleus pulposus is expected to arrest the initial dehydration of the degenerated nucleus and return the disc to a fully hydrated state so that the degenerative process, including the associated pain, is postponed or prevented and the mechanical function is restored to the vertebral segment.

It is contemplated that the synthetic bioconjugates described herein will bind to and protect the annulus fibrosis. Accordingly, the bioconjugates described here can be used in a method of treating and/or preventing annulus fibrosis degeneration in a patient comprising administering to a patient in need thereof a pharmaceutical composition comprising the extracellular matrix-binding synthetic bioconjugate described herein. Also the bioconjugates described here can be used in a method of treating and/or preventing nucleus pulposus degeneration in a patient comprising administering to a patient in need thereof a pharmaceutical composition comprising the extracellular matrix-binding synthetic bioconjugate described herein.

The bioconjugate used in the methods described above may comprise chondroitin sulfate and about 1 to about 20 peptides, wherein the peptides comprise at least one sequence of GAHWQFNALTVR (SEQ ID NO: 58) or GAHWQFNALTVRGSG (SEQ ID NO: 357), and at least one sequence of WYRGRL (SEQ ID NO: 29) or WYRGRLGSG (SEQ ID NO: 392).

The bioconjugate used in the methods described above may comprise chondroitin sulfate and about 1 to about 20 peptides, wherein the peptides comprise at least one sequence of GAHWQFNALTVR (SEQ ID NO: 58) or GAHWQFNALTVRGSG (SEQ ID NO: 357), and at least one sequence of RRANAALKAGELYKSILY (SEQ ID NO: 1) or RRANAALKAGEL YKSIL YGSG (SEQ ID NO: 287).

**5. Compositions**

In one embodiment, the bioconjugate is administered in a composition. The present disclosure provides compositions comprising a bioconjugate and a pharmaceutically acceptable carrier. Pharmaceutically acceptable carriers are known to one having ordinary skill in the art may be used, including water or saline. As is known in the art, the components as well as their relative amounts are determined by the intended use and method of delivery. Diluent or carriers employed in the compositions can be selected so that they do not diminish the desired effects of the bioconjugate. Examples of suitable compositions include aqueous solutions, for example, a solution in isotonic saline, 5% glucose.

Other well-known pharmaceutically acceptable liquid carriers such as alcohols, glycols, esters and amides, may be employed. In certain embodiments, the composition further comprises one or more excipients, such as, but not limited to ionic strength modifying agents, solubility enhancing agents, sugars such as mannitol or sorbitol, pH buffering agent, surfactants, stabilizing polymer, preservatives, and/or co-solvents.

In certain embodiments, the composition is an aqueous solution. Aqueous solutions are suitable for use in composition formulations based on ease of formulation, as well as an ability to easily administer such compositions by means of instilling the solution in. In certain embodiments, the compositions are suspensions, viscous or semi-viscous gels, or other types of solid or semi-solid compositions. In some embodiments, the composition is in the form of foams, ointments, liquid wash, gels, sprays and liposomes, which are very well known in the art. Alternatively, the topical administration is an infusion of the provided bioconjugate to the treatment site via a device selected from a pump-catheter system, a continuous or selective release device, or an adhesion barrier. In certain embodiments, the composition is a solution that is directly applied to or contacts the internal wall of a vein or artery. In some embodiments, the composition comprises a polymer matrix. In other embodiments, the composition is absorbable. In certain embodiments, the composition comprises a pH buffering agent. In some embodiments, the composition contains a lubricity enhancing agent.

In certain embodiments, a polymer matrix or polymeric material is employed as a pharmaceutically acceptable carrier or support for the composition. The polymeric material described herein may comprise natural or unnatural polymers, for example, such as sugars, peptides, protein, laminin, collagen, hyaluronic acid, ionic and non-ionic water soluble polymers; acrylic acid polymers; hydrophilic polymers such as polyethylene oxides, polyoxyethylene-polyoxypropylene copolymers, and polyvinylalcohol; cellulosic polymers and cellulosic polymer derivatives such as hydroxypropyl cellulose, hydroxyethyl cellulose, hydroxypropyl methylcellulose, hydroxypropyl methylcellulose phthalate, methyl cellulose, carboxymethyl cellulose, and etherified cellulose; poly(lactic acid), poly(glycolic acid), copolymers of lactic and glycolic acids, or other polymeric agents both natural and synthetic. In certain embodiments, the compositions provided herein is formulated as films, gels, foams, or and other dosage forms.

Suitable ionic strength modifying agents include, for example, glycerin, propylene glycol, mannitol, glucose, dextrose, sorbitol, sodium chloride, potassium chloride, and other electrolytes.

In certain embodiments, the solubility of the bioconjugate may need to be enhanced. In such cases, the solubility may be increased by the use of appropriate formulation techniques, such as the incorporation of solubility-enhancing compositions such as mannitol, ethanol, glycerin, polyethylene glycols, propylene glycol, poloxomers, and others known in the art.

In certain embodiments, the composition contains a lubricity enhancing agent. As used herein, lubricity enhancing agents refer to one or more pharmaceutically acceptable polymeric materials capable of modifying the viscosity of the pharmaceutically acceptable carrier. Suitable polymeric materials include, but are not limited to: ionic and non-ionic water soluble polymers; hyaluronic acid and its salts, chondroitin sulfate and its salts, dextrans, gelatin, chitosans, gellans, other bioconjugates or polysaccharides, or any combination thereof; cellulosic polymers and cellulosic polymer derivatives such as hydroxypropyl cellulose, hydroxyethyl cellulose, hydroxypropyl methylcellulose, hydroxypropyl methylcellulose phthalate, methyl cellulose, carboxymethyl cellulose, and etherified cellulose; collagen and modified collagens; galactomannans, such as guar gum, locust bean gum and tara gum, as well as polysaccharides derived from the foregoing natural gums and similar natural or synthetic gums containing mannose and/or galactose moieties as the main structural components (e.g., hydroxypropyl guar); gums such as tragacanth and xanthan gum; gellan gums; alginate and sodium alginate; chitosans; vinyl polymers; hydrophilic polymers such as polyethylene oxides, polyoxyethylene-polyoxypropylene copolymers, and polyvinylalcohol; carboxyvinyl polymers or crosslinked acrylic acid polymers such as the "carbomer" family of polymers, e.g., carboxypolyalkylenes that may be obtained commercially under the Carbopol™ trademark; and various other viscous or viscoelastomeric substances. In one embodiment, a lubricity enhancing agent is selected from the group consisting of hyaluronic acid, dermatan, chondroitin, heparin, heparan, keratin, dextran, chitosan, alginate, agarose, gelatin, hydroxypropyl cellulose, hydroxyethyl cellulose, hydroxypropyl methylcellulose, hydroxypropyl methylcellulose phthalate, methyl cellulose, carboxymethyl cellulose, and etherified cellulose, polyvinyl alcohol, polyvinylpyrrolidinone, povidone, carbomer 941, carbomer 940, carbomer 971P, carbomer 974P, or a pharmaceutically acceptable salt thereof. In one embodiment, a lubricity enhancing agent is applied concurrently with the bioconjugate. Alternatively, in one embodiment, a lubricity enhancing agent is applied sequentially to the bioconjugate. In one embodiment, the lubricity enhancing agent is chondroitin sulfate. In one embodiment, the lubricity enhancing agent is hyaluronic acid. The lubricity enhancing agent can change the viscosity of the composition.

For further details pertaining to the structures, chemical properties and physical properties of the above lubricity enhancing agents, see e.g., U.S. 5,409,904, U.S. 4,861,760 (gellan gums), U.S. 4,255,415, U.S. 4,271,143 (carboxyvinyl polymers), WO 94/10976 (polyvinyl alcohol), WO 99/51273 (xanthan gum), and WO 99/06023 (galactomannans). Typically, non-acidic lubricity enhancing agents, such as a neutral or basic agent are employed in order to facilitate achieving the desired pH of the formulation.

In some embodiments, the bioconjugates can be combined with minerals, amino acids, sugars, peptides, proteins, vitamins (such as ascorbic acid), or laminin, collagen, fibronectin, hyaluronic acid, fibrin, elastin, or aggrecan,

or growth factors such as epidermal growth factor, platelet-derived growth factor, transforming growth factor beta, or fibroblast growth factor, and glucocorticoids such as dexamethasone or viscoelastic altering agents, such as ionic and non-ionic water soluble polymers; acrylic acid polymers; hydrophilic polymers such as polyethylene oxides, polyoxyethylene-polyoxypropylene copolymers, and polyvinylalcohol; cellulosic polymers and cellulosic polymer derivatives such as hydroxypropyl cellulose, hydroxyethyl cellulose, hydroxypropyl methylcellulose, hydroxypropyl methylcellulose phthalate, methyl cellulose, carboxymethyl cellulose, and etherified cellulose; poly(lactic acid), poly(glycolic acid), copolymers of lactic and glycolic acids, or other polymeric agents both natural and synthetic.

Suitable pH buffering agents for use in the compositions herein include, for example, acetate, borate, carbonate, citrate, and phosphate buffers, as well as hydrochloric acid, sodium hydroxide, magnesium oxide, monopotassium phosphate, bicarbonate, ammonia, carbonic acid, hydrochloric acid, sodium citrate, citric acid, acetic acid, disodium hydrogen phosphate, borax, boric acid, sodium hydroxide, diethyl barbituric acid, and proteins, as well as various biological buffers, for example, TAPS, Bicine, Tris, Tricine, HEPES, TES, MOPS, PIPES, cacodylate, or MES. In certain embodiments, an appropriate buffer system (e.g., sodium phosphate, sodium acetate, sodium citrate, sodium borate or boric acid) is added to the composition to prevent pH drift under storage conditions. In some embodiments, the buffer is a phosphate buffered saline (PBS) solution (i.e., containing sodium phosphate, sodium chloride and in some formulations, potassium chloride and potassium phosphate). The particular concentration will vary, depending on the agent employed. In certain embodiments, the pH buffer system (e.g., sodium phosphate, sodium acetate, sodium citrate, sodium borate or boric acid) is added to maintain a pH within the range of from about pH 4 to about pH 8, or about pH 5 to about pH 8, or about pH 6 to about pH 8, or about pH 7 to about pH 8. In some embodiments, the buffer is chosen to maintain a pH within the range of from about pH 4 to about pH 8. In some embodiments, the pH is from about pH 5 to about pH 8. In some embodiments, the buffer is a saline buffer. In certain embodiments, the pH is from about pH 4 and about pH 8, or from about pH 3 to about pH 8, or from about pH 4 to about pH 7. In some embodiments, the composition is in the form of a film, gel, patch, or liquid solution which comprises a polymeric matrix, pH buffering agent, a lubricity enhancing agent and a bioconjugate wherein the composition optionally contains a preservative; and wherein the pH of said composition is within the range of about pH 4 to about pH 8.

Surfactants are employed in the composition to deliver higher concentrations of bioconjugate. The surfactants function to solubilize the inhibitor and stabilize colloid dispersion, such as micellar solution, microemulsion, emulsion and suspension. Suitable surfactants comprise c polysorbate, poloxamer, polyosyl 40 stearate, polyoxyl castor oil, tyloxapol, triton, and sorbitan monolaurate. In one embodiment, the surfactants have hydrophile/lipophile/balance (HLB) in the range of 12.4 to 13.2 and are acceptable for ophthalmic use, such as TritonX1 14 and tyloxapol.

In certain embodiments, stabilizing polymers, i.e., demulcents, are added to the composition. The stabilizing polymer should be an ionic/charged example, more specifically a polymer that carries negative charge on its surface that can exhibit a zeta-potential of (-)10-50 mV for physical stability and capable of making a dispersion in water (i.e. water soluble). In one embodiment, the stabilizing polymer comprises a polyelectrolyte or polyectrolytes if more than one, from the family of cross-linked polyacrylates, such as carbomers and Pemulen®, specifically Carbomer 974p (polyacrylic acid), at a range of about 0.1% to about 0.5% w/w.

In one embodiment, the composition comprises an agent which increases the permeability of the bioconjugate to the extracellular matrix of blood vessels. Preferably the agent which increases the permeability is selected from benzalkonium chloride, saponins, fatty acids, polyoxyethylene fatty ethers, alkyl esters of fatty acids, pyrrolidones, polyvinylpyrrolidone, pyruvic acids, pyroglutamic acids or mixtures thereof.

The bioconjugate may be sterilized to remove unwanted contaminants including, but not limited to, endotoxins and infectious agents. Sterilization techniques which do not adversely affect the structure and biotropic properties of the bioconjugate can be used. In certain embodiments, the bioconjugate can be disinfected and/or sterilized using conventional sterilization techniques including propylene oxide or ethylene oxide treatment, sterile filtration, gas plasma sterilization, gamma radiation, electron beam, and/or sterilization with a peracid, such as peracetic acid. In one embodiment, the bioconjugate can be subjected to one or more sterilization processes. Alternatively, the bioconjugate may be wrapped in any type of container including a plastic wrap or a foil wrap, and may be further sterilized.

In some embodiments, preservatives are added to the composition to prevent microbial contamination during use. Suitable preservatives added to the compositions comprise benzalkonium chloride, benzoic acid, alkyl parabens, alkyl benzoates, chlorobutanol, chlorocresol, cetyl alcohols, fatty alcohols such as hexadecyl alcohol, organometallic compounds of mercury such as acetate, phenyl mercury nitrate or borate, diazolidinyl urea, diisopropyl adipate, dimethyl polysiloxane, salts of EDTA, vitamin E and its mixtures. In certain embodiments, the preservative is selected from benzalkonium chloride, chlorobutanol, benzododecinium bromide, methyl paraben, propyl paraben, phenylethyl alcohol, edentate disodium, sorbic acid, or polyquarternium-1. In certain embodiments, the compositions comprise a preservative. In some embodiments, the preservatives are employed at a level of from about 0.001% to about 1.0% w/v. In certain embodiments, the compositions do not contain a preservative and are referred to as "unpreserved". In some embodiments, the unit dose compositions are sterile, but unpreserved.

In some embodiments, separate or sequential administration of the bioconjugate and other agent is necessary

to facilitate delivery of the composition. In certain embodiments, the bioconjugate and the other agent can be administered at different dosing frequencies or intervals. For example, the bioconjugate can be administered daily, while the other agent can be administered less frequently. Additionally, as will be apparent to those skilled in the art, the bioconjugate and the other agent can be administered using the same route of administration or different routes of administration.

Any effective regimen for administering the bioconjugate can be used. For example, the bioconjugate can be administered as a single dose, or as a multiple-dose daily regimen. Further, a staggered regimen, for example, one to five days per week can be used as an alternative to daily treatment.

In various embodiments, the bioconjugate can be administered topically, such as by film, gel, patch, or liquid solution. In some of the embodiments, the compositions provided are in a buffered, sterile aqueous solution. In certain embodiments, the solutions have a viscosity of from about 1 to about 100 centipoises (cps), or from about 1 to about 200 cps, or from about 1 to about 300 cps, or from about 1 to about 400 cps. In some embodiments, the solutions have a viscosity of from about 1 to about 100 cps. In certain embodiments, the solutions have a viscosity of from about 1 to about 200 cps. In certain embodiments, the solutions have a viscosity of from about 1 to about 300 cps. In certain embodiments, the solutions have a viscosity of from about 1 to about 400 cps. In certain embodiments, the solution is in the form of an injectable liquid solution. In other embodiments, the compositions are formulated as viscous liquids, i.e., viscosities from several hundred to several thousand cps, gels or ointments. In these embodiments, the bioconjugate is dispersed or dissolved in an appropriate pharmaceutically acceptable carrier.

Exemplary compositions for use with the bioconjugates for catheter-based delivery may comprise: a) a synthetic bioconjugate as described herein; b) a pharmaceutically acceptable carrier; c) a polymer matrix; d) a pH buffering agent to provide a pH in the range of about pH 4 to about pH 8; and e) a water soluble lubricity enhancing agent in the concentration range of about 0.25% to about 10% total formula weight or any individual component a), b), c), d) or e), or any combinations of a), b), c), d) or e).

Exemplary formulations may comprise: a) bioconjugate as described herein; b) pharmaceutically acceptable carrier; c) polymer matrix; and d) pH buffering agent to provide a pH in the range of about pH 4 to about pH 8, wherein said solution has a viscosity of from about 3 to about 30 cps for a liquid solution.

Exemplary compositions contemplated by the present disclosure may also be for administration by injection include aqueous or oil suspensions, or emulsions, with sesame oil, corn oil, cottonseed oil, or peanut oil, as well as elixirs, mannitol, dextrose, or a sterile aqueous solution, and similar pharmaceutical vehicles. Aqueous solutions in saline are also conventionally used for injection, but less preferred in the context of the present disclosure. Ethanol, glycerol, propylene glycol, liquid polyethylene glycol, and the like (and suitable mixtures thereof), cyclodextrin derivatives, and vegetable oils may also be employed. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like.

Sterile injectable solutions are prepared by incorporating the component in the required amount in the appropriate solvent with various other ingredients as enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

In making pharmaceutical compositions that include bioconjugates described herein, the active ingredient is usually diluted by an excipient or carrier and/or enclosed within such a carrier that can be in the form of a capsule, sachet, paper or other container. When the excipient serves as a diluent, it can be a solid, semi-solid, or liquid material (as above), which acts as a vehicle, carrier or medium for the active ingredient. Thus, the compositions can be in the form of films, gels, patches, powders, lozenges, sachets, cachets, elixirs, suspensions, emulsions, solutions, syrups, aerosols (as a solid or in a liquid medium), ointments containing, for example, up to 10% by weight of the active compounds, soft and hard gelatin films, gels, patches, sterile injectable solutions, and sterile packaged powders.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents.

Films used for drug delivery are well known in the art and comprise non-toxic, non-irritant polymers devoid of leachable impurities, such as polysaccharides (e.g., cellulose, maltodextrin, etc.). In some embodiments, the polymers are hydrophilic. In other embodiments, the polymers are hydrophobic. The film adheres to tissues to which it is applied, and is slowly absorbed into the body over a period of about a week. Polymers used in the thin-film dosage forms described herein are absorbable and exhibit sufficient peel, shear and tensile strengths as is well known in the art. In some

embodiments, the film is injectable. In certain embodiments, the film is administered to the patient prior to, during or after surgical intervention.

Gels are used herein refer to a solid, jelly-like material that can have properties ranging from soft and weak to hard and tough. As is well known in the art, a gel is a non-fluid colloidal network or polymer network that is expanded throughout its whole volume by a fluid. A hydrogel is a type of gel which comprises a network of polymer chains that are hydrophilic, sometimes found as a colloidal gel in which water is the dispersion medium. Hydrogels are highly absorbent and can contain a high degree of water, such as, for example greater than 90% water. In some embodiments, the gel described herein comprises a natural or synthetic polymeric network. In some embodiments, the gel comprises a hydrophilic polymer matrix. In other embodiments, the gel comprises a hydrophobic polymer matrix. In some embodiments, the gel possesses a degree of flexibility very similar to natural tissue. In certain embodiments, the gel is biocompatible and absorbable. In certain embodiments, the gel is administered to the patient prior to, during or after surgical intervention.

Liquid solution as used herein refers to solutions, suspensions, emulsions, drops, ointments, liquid wash, sprays, liposomes which are well known in the art. In some embodiments, the liquid solution contains an aqueous pH buffer agent which resists changes in pH when small quantities of acid or base are added. In certain embodiments, the liquid solution is administered to the patient prior to, during or after surgical intervention.

Exemplary formulations may comprise: a) one or more bioconjugate as described herein; b) pharmaceutically acceptable carrier; and c) hydrophilic polymer as matrix network, wherein said compositions are formulated as viscous liquids, i.e., viscosities from several hundred to several thousand cps, gels or ointments. In these embodiments, the bioconjugate is dispersed or dissolved in an appropriate pharmaceutically acceptable carrier.

In certain embodiments, the bioconjugate, or a composition comprising the same, is lyophilized prior to, during, or after, formulation. Accordingly, also provided herein is a lyophilized composition comprising a bioconjugate or composition comprising the same as described herein.

### 6. Dosing

Suitable dosages of the bioconjugate can be determined by standard methods, for example by establishing dose-response curves in laboratory animal models or in clinical trials and can vary significantly depending on the patient condition, the disease state being treated, the route of administration and tissue distribution, and the possibility of co-usage of other therapeutic treatments. The effective amount to be administered to a patient is based on body surface area, patient weight or mass, and physician assessment of patient condition. In various exemplary embodiments, a dose ranges from about 0.01 μg to about 10 g. For example, for systemic delivery, the dose can be about 10 g, or about 5 g, or about 1 g. In other illustrative embodiments, effective doses ranges from about 100 μg to about 10 g per dose, or from about 100 μg to about 1 g per dose, or from about 100 μg to about 500 mg per dose, from about 0.01 μg to about 100 mg per dose, or from about 100 μg to about 50 mg per dose, or from about 500 μg to about 10 mg per dose, or from about 1 mg to 10 mg per dose, or from about 1 to about 100 mg per dose, or from about 1 mg to 500 mg per dose, or from about 1 mg to 200 mg per dose, or from about 10 mg to 100 mg per dose, or from about 10 mg to 75 mg per dose, or from about 10 mg to 50 mg per dose, or about 10 mg per dose, or about 20 mg per dose, or about 30 mg per dose, or about 40 mg per dose, or about 50 mg per dose, or about 60 mg per dose, or about 70 mg per dose, or about 80 mg per dose, or about 90 mg per dose, or about 100 mg per dose. In any of the various embodiments described herein, effective doses ranges from about 0.01 μg to about 1000 mg per dose, 1 μg to about 100 mg per dose, about 100 μg to about 1.0 mg, about 50 μg to about 600 μg, about 50 μg to about 700 μg, about 100 μg to about 200 μg, about 100 μg to about 600 μg, about 100 μg to about 500 μg, about 200 μg to about 600 μg, or from about 100 μg to about 50 mg per dose, or from about 500 μg to about 10 mg per dose or from about 1 mg to about 10 mg per dose.

In some embodiments, the compositions are packaged in multidose form. Preservatives are thus required to prevent microbial contamination during use. In certain embodiments, suitable preservatives as described above can be added to the compositions. In some embodiments, the composition contains a preservative. In certain embodiments the preservatives are employed at a level of from about 0.001% to about 1.0% w/v. In some embodiments, the unit dose compositions are sterile, but unpreserved.

## Examples

### Example 1. EDC Functionalization Protocol (Hep-SILY)

***Materials***

A suitable reaction buffer is prepared (e.g., 2-(N-morpholino)ethanesulfonic acid (MES)) with an appropriate concentration of a chaotropic agent, such as butanol, ethanol, guanidinium chloride, lithium perchlorate, lithium acetate, magnesium chloride, phenol, propanol, sodium dodecyl sulfate, thiourea, or urea (e.g., from about 5 M to about 10 M

urea). The final pH is adjusted to a pH of from about 4.5 to about 6 with 1 N HCl.

Peptide (e.g., RRANAALKAGELYKSILYGSG-$NHNH_2$ (SEQ ID NO: 397) (MW = 2253 Da, structure shown below)) is dissolved in reaction buffer to 3 mg/mL. The peptide solution is typically freshly prepared prior to the coupling reaction.

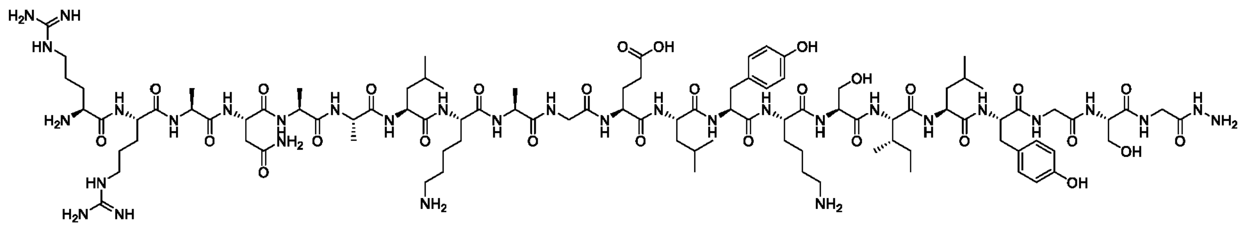

Biotinylated peptide (e.g., biotinRRANAALKAGELYKSILYGSG-$NHNH_2$ (SEQ ID NO: 398) (MW = 2479 Da)) is dissolved in reaction buffer to 3 mg/mL. The resulting labeled peptide solution is typically freshly prepared prior to the coupling reaction.

Glycan (e.g., heparin ($MW_{avg}$ = 16 kDa)) is dissolved in reaction buffer to 20 mg/mL and either stored at -20 °C or prepared freshly prior to the coupling reaction.

EDC (1-Ethyl-3-(3-dimethylaminopropyl)carbodiimide) is dissolved to 75 mg/mL in reaction buffer immediately before adding to the glycan.

***Conjugation - Heparin Containing Bioconjugate (100 mg)***

Heparin was activated by adding 1-ethyl-3-(3-dimethylaminopropyl)carbodiimide (EDC) (59.3 mg or 0.79 mL dissolved at 75 mg/mL in water) in a 50 molar excess to heparin. The starting materials were reacted at room temperature for about 5 minutes. The labeled peptide was then added to the activated heparin in a 1:1 molar ratio (heparin:labeled peptide) (15.3 mg or 5.1 mL at 3 mg/mL in reaction buffer). The reaction mixture was then shaken for about 5 minutes at room temperature. While shaking, the peptide was added in a 1:7 molar ratio (Hep:peptide) (97.3 mg or 32.4 mL at 3 mg/mL in reaction buffer). The components were then allowed to react for about 2 hours at room temperature while shaking. After the allotted time, the reaction was quenched by raising the pH to 8 with 0.5 M NaOH (approximately 4.5 mL) for about 30 minutes at room temperature while shaking.

The resulting Hep-SILY bioconjugate was purified via diafilter (Spectrum - MidiKros mPES 10 K hollow tube filter) using 5 column volumes (CVs) of reaction buffer (approximately 250 mL), followed by 10 CVs of water (approximately 500 mL) at a flow rate of 35 mL/min with TMP at approximately 15 psi. The retentate (i.e., final product) was then frozen at -80 °C. Optionally, the product is lyophilized to dryness.

**Example 2. Platelet Inhibition as Measured by Platelet-Factor-4 (PF4)**

The following method is used to assess the effect of the bioconjugates disclosed herein on platelet inhibition. Microplates were coated with fibrillar collagen and then blocked with 1% milk. The bioconjugate was diluted in 1X PBS starting at 1 mg/mL and diluted with 1X PBS using a 10X concentration factor, and 50 μL solution was added to the collagen coated wells. Treatments were incubated at room temperature for 15 min. Wells were then rinsed of unbound treatment by removing the treatment solution and rinsing with 1X PBS three times.

Human whole blood was collected from healthy volunteers by venipuncture. The first 5 mL of blood was discarded and approximately 15 mL was then collected into citrated glass vacutainers (BD Bioscience). Blood was centrifuged in the glass tube for 20 min at 200 g at 25 °C. The top layer of the centrifuged blood, the platelet rich plasma, was used for platelet experiments.

Platelet rich plasma (50 μL/well) was added to the microplate for 1 h at room temperature. After 1 h of incubation, 45 mL of platelet rich plasma was removed from each well and added to a microcentrifuge tube containing 5 mL ETP (107 mM EDTA, 12 mM theophylline, and 2.8 mM prostaglandin E1) to inhibit further platelet activation. These tubes were spun at 4 °C for 30 min at 2000 g to pellet the platelets. The supernatant was collected for ELISA studies to test for the presence of platelet activation markers. Sandwich ELISAs were utilized in order to detect each protein. The components for both sandwich ELISAs were purchased from R&D Systems and the provided protocols were followed. It was necessary to dilute the platelet serum in 1% BSA in 1X PBS in order for values to fall within a linear range of the assay. Platelet activation was measured through release of platelet factor 4 (PF-4).

### Example 3. Fibrillogenesis Assay

The following method is used to assess the effect of the bioconjugates disclosed herein on fibrillogenesis. Collagen solutions were prepared by diluting 2 mg/mL collagen in HCl to 1 mg/mL in 2X TES buffer (60 mM TES, 60mM $Na_2HPO_4$, 300 mM NaCl) and kept on ice. Test samples containing bioconjugate were dissolved to a final concentration of 0.6 mg/mL in 1X phosphate buffered saline (PBS) solutions and also kept on ice. Test samples were added to collagen in a ratio of 1:1 (volume:volume) such that the final collagen concentration was 0.5 mg/mL. Collagen test solutions were then thoroughly mixed by vortexing.

Fibrillogenesis was measured by monitoring the turbidity (absorbance at 313 nm) of the collagen test solutions during incubation at 37 °C. Samples were pipetted into a 96-well plate at 100 μL/well. A microplate reader was held at 37 °C, and turbidity was monitored every minute for up to 60 minutes.

### Example 4. Collagen-Binding Plate Assay

The following method is used to assess the binding affinity of bioconjugates disclosed herein for collagen. Similar assays are employed to assess binding affinity of other targets (e.g., hyaluronic acid).

Collagen-binding of bioconjugate variants was compared by a plate-assay, in which collagen was coated on 96-well plates. Collagen was coated on high-bind plates at 50 μg/mL in 0.02 N acetic acid for 1 hour at room temperature. Unbound collagen was rinsed with 1X PBS pH 7.4. Plates were then blocked in 1% milk in 1X PBS solution for 1 hour at room temperature.

Bioconjugate variants containing biotinlyated peptides were dissolved to a final concentration of 1 mg/mL in 1% milk in 1X PBS pH 7.4. From this solution, a 10X serial dilution was performed. Molecules were then incubated on the blocked collagen-coated plates and incubated for 15 minutes at room temperature. Plates were then rinsed 3 times with 1X PBS in 1% BSA and 0.2% Tween20.

Bound molecules were detected by streptavidin-HRP, which was diluted 1:500 in 1X PBS with 1% BSA and 0.2% Tween20 and incubated 200 μL/well for 20 minutes at room temperature. Streptavidin solution was rinsed from the plates 3 times with 1X PBS with 0.2% Tween20. TMB Substrate solution was then added to each well 100 μL/well for 15 minutes at room temperature, and the color evolution was stopped with 100 μL sulfuric acid solution (0.16 M). Absorbance in the well was then measured at 450 nm and binding affinity was plotted in a dose-response by absorbance vs. concentration.

### Example 5. Comparison of Hyaluronic acid-binding Bioconjugates

This example shows that bioconjugate comprising peptides with a hyaluronic acid-binding unit as described herein exhibit an unexpectedly enhanced binding affinity to hyaluronic acid when compared to a hyaluronic acid-binding bioconjugate as described in U.S. 2014/0301983.

#### *Bioconjugate*

Hyaluronic acid-binding bioconjugates having the hydrazide-carbonyl linkage as described herein were prepared according to Example 1, above, using chondroitin sulfate and GAH peptide (i.e., GAHWQFNALTVRGSG-$NHNH_2$ (SEQ ID NO: 399)) and STM peptide (i.e., STMMSRSHKTRSHHVGSG-$NHNH_2$ (SEQ ID NO: 400)). Biotinylated versions of the hyaluronic acid-binding bioconjugates having the hydrazide-carbonyl linkage were also prepared according to Example 1, above, using chondroitin sulfate and labeled GAH peptide (i.e., biotinGAHWQFNALTVRGSG-$NHNH_2$ (SEQ ID NO: 401)) and labeled STM peptide (i.e., biotin STMMSRSHKTRSHHVGSG-$NHNH_2$ (SEQ ID NO: 402)).

A hyaluronic acid-binding bioconjugate using oxidized chondroitin sulfate and the GAH peptide with a GGGC spacer (i.e., GAHWQFNALTVRGGGC (SEQ ID NO: 402)) via a BMPH linker as described in U.S. 2014/0301983.Biotinylated chondroitin sulfate was used as a control (**FIG. 1A**).

#### *Incorporation Assay*

To evaluate the binding of the aggrecan mimic to hyaluronic acid, HyStem hydrogels were synthesized as per the manufacturer's protocol (ESI BIO). Briefly, the Glycosil (thiol-modified hyaluronic acid), Extralink-lite (polyethylene glycol diacrylate, PEGDA), and degassed, deionized water (DG) were allowed to come to room temperature. Under aseptic conditions using a syringe and needle 1.0 mL of DG water was added to the Glycosil vial with shaking on a horizontal shaker for 40 minutes. Similarly, 0.5 mL of DG water was added to the Extralink-Lite vial. The Extralink-lite and the Glycosil were then mixed in a 1:4 volume ratio (0.25 mL Extralink-Lite to 1.0 mL Glycosil). 50 μl of the mixture was pipetted into 0.5 mL tube caps from Eppendorf and the solution was allowed to gel for 2 hours. These hydrogels

were then incubated with 500 μl of aggrecan mimic solution in a 24 well plate for 3 hours with shaking on a horizontal shaker. Subsequently, the gels were washed by incubating in 1X phosphate buffered saline (1X PBS) overnight, followed by staining using a Streptavidin DyLight fluorescence probe (Lifetechnologies). Streptavidin DyLight was diluted in a 1:200 volume ratio in 1X PBS and added to the gels for 1 hour with shaking on a horizontal shaker. Non-specifically bound fluorescence probe was washed away by incubating the gels in 1X PBS for 20 minutes with shaking on a horizontal shaker (repeated twice). The gels were then imaged using a spectral confocal microscope (Nikon, A1+).

Figure 1 shows that the bioconjugate having GAH peptide bound to CS via a hydrazide-carbonyl linkage (**FIG. 1C**) and bioconjugate having STM peptide bound to CS via a hydrazide-carbonyl linkage (**FIG. 1D**) exhibit a greater hyaluronic acid-binding affinity as compared to the bioconjugate having GAH peptide bound to CS via oxidative saccharide ring-opening chemistry and BMPH linker (**FIG. 1B**).

**Example 6. Delivery of bioconjugate to the fistula vessels without altering standard of care**

This example demonstrates that the bioconjugates disclosed herein could be used as a luminal vascular coating to prevent intimal hyperplasia in a native AVF. The fistulas are created in the femoral arteries and veins of pigs, either treatment with the bioconjugate disclosed herein or with saline as a control. It is contemplated that the bioconjugate will be effectively delivered to the fistula vessels without altering standard of care, and will result in significantly less stenosis than untreated fistulas and enlarged vessel diameter.

Three delivery methods may be tested via this example: (1) delivery immediately prior to blood flow, (2) delivery following intentional denudation of the fistula prior to blood flow, and (3) delivery to the formed fistula following restoration of blood flow (e.g., after 5 minutes).

Method 1 is considered the primary method of delivery, and methods 2 and 3 can be used alone or in addition to method 1 in case the method 1 does not adequately result in coverage of bioconjugate to the vessel lumen. In method 2, intentional denudation of the vessel is performed by rubbing the vein with the handle of forceps, similarly to clinical application of a dilator during AVF creation. Method 3 can be examined to determine if the high blood flow in the vein immediately following fistula creation results in damage to the endothelial cell layer that would not be addressed by initial delivery of the therapeutic. In method 3, blood flow is restored to the fistula, and then stopped by clamping the proximal vein and artery. The fistula is then flushed with bioconjugate and blood flow restored.

In all cases, a single suture is removed in the newly created anastomosis and the bioconjugate is flushed through the fistula using a feeding needle with a smooth ball tip. Bioconjugate (e.g., approximately 5 mL) is flushed through the fistula. Animals are sacrificed following bioconjugate delivery (e.g., about 2 hours). The fistulas are removed, rinsed in saline, and fixed with 10% formalin for (e.g., about 10 minutes).

Cells are stained using phalloidin. When the bioconjugate has a biotin tag, bioconjugate is detected using a fluorescently labeled streptavidin marker. The vein is separated from the artery, and both vessels are opened so that their luminal surfaces can be examined using confocal microscopy.

It is contemplated that delivery of bioconjugate will be effective in all three methods. Non-specific binding is accounted for by testing arteries not exposed to bioconjugate, i.e., no evident staining. Level of stenosis is determined by injecting a contrast dye into the animal during a CT scan so that the vessels can be visualized and the diameter of the vessel measured through the images.

**Example 7. Hep-SILY for treatment of stenosis or occlusion within the femoropopliteal artery**

The following is a clinical test designed to evaluate and compare the safety and efficacy of balloon angioplasty plus intraluminal Hep-SILY against plain old balloon angioplasty (POBA) plus saline (control) for treatment of stenosis or occlusion within the femoropopliteal artery. Hep-SILY with approximately 6-8 SILY peptides per heparin is prepared according to Example 1.

This test proposes a multicenter, 2-arm, parallel, blinded, randomized comparison of the safety and efficacy of balloon angioplasty plus intraluminal Hep-SILY to balloon angioplasty alone in de novo or restenotic lesions in native femoropopliteal arteries. Approximately 66 subjects presenting with claudication or ischemic rest pain and an angiographically significant lesion in the femoropopliteal artery with patient outflow artery to the foot will be randomized and treated for this study.

Key inclusion criteria will be age over 40 and willing to provide consent. Key exclusion criteria will be pregnancy, life expectancy of less than 5 years, history of haemorrhagic stroke within 3 months of screening, history of myocardial infarction, thrombolysis or angina within 2 weeks of screening, and known contraindication to heparin.

Subjects will receive a baseline angiogram to confirm an angiographically significant lesion in the femoropopliteal artery. After angiographic confirmation and successful crossing of the lesion(s) by the guidewire, subjects will be randomized 2:1 to POBA plus SBCV treatment (Group 1, N = 44) versus POBA treatment plus saline (control) (Group 2, N = 22). Group 1 will receive balloon angioplasty and up to 10 ml of Hep-SILY flush, whereas Group 2 will receive balloon

angioplasty and up to 10 ml of saline flush.

Total duration of the study is approximately 10 months (from first subject first visit to last subject last visit) including the enrolment period of 4 months. Individual study participation is approximately 24 weeks after the initial procedure and the screening visit will be up to one month before study procedure.

A variety of data will be collected pre-discharge as well as at 4, 8, 12, 18 and 24 week follow-up visits. The primary endpoint is efficacy as measured by late lumen loss (LLL) at 24 weeks following treatment in the analysis segment (entire length of the injury segment plus 5-mm proximal and distal margins) as assessed by an independent, blinded angiographic core lab. LLL is defined as the difference between the minimum lumen diameter (MLD) immediately post-primary procedure and the MLD at follow-up.

LLL will be measured using the 24-week follow up angiogram compared to the post-procedure angiogram (for subjects with chronic renal insufficiency, a duplex Doppler ultrasound will be used to measure LLL).

The secondary endpoint is safety as measured by the incidence of treatment-emergent adverse events (AEs), clinical laboratory evaluations, vital signs, and physical examination findings. Specific safety events to be measured include all-cause death, amputation (above the ankle)-free survival (AFS) and target vessel revascularization (TVR).

**Example 8: Hep-SILY for treatment or prevention of neointimal hyperplasia or peripheral artery disease (PAD)**

Neointimal hyperplasia is evaluated in a rabbit angioplasty model in which a bioconjugate as described herein (e.g., the Hep-SILY of Example 1) is delivered. Multiple (e.g., six) rabbits are enrolled in the study. Each animal receives a balloon angioplasty-mediated injury to both the right and left iliac artery. Animals are divided into test group (Heparin-SII,Y) or vehicle control (1xPBS). In each group, both iliac arteries are injured and treated with test article or control immediately following balloon injury.

After a given time (e.g., 28 days) following injury, animals are euthanized and the artery segments evaluated histologically. Several (e.g., three) histological sections with the most severe neointimal response from each vessel are typically selected for morphometry. Cross-sectional areas of the extranal elastic lamina (EEL), internal elastic lamina (IEL), and lumen are measured with digital morphometry (IPLab software, Rockville, MD) from Movat stained slides. Neointimal thickness is measured as the distance from the IEL to the lumen, at minimal and maximal sites, and then averaged. The cross-sectional areas are used to calculated the following:

- $$\text{Medial area} = \text{EEL area} - \text{IEL area}$$
- $$\text{Neointimal area} = \text{IEL area} - \text{Lumen area}$$
- $$\text{Medial-Intimal Area} = \text{EEL area} - \text{lumen area}$$
- $$\%\ \text{Stenosis} = [1 - (\text{Lumen area}/\text{IEL Area})] * 100$$

The means of the variables are compared using analysis of variance (ANOVA). A p-value of less than 0.05 is typically considered statistically significant.

It is contemplated that the bioconjugate as described herein (e.g., the Hep-SILY of Example 1) will be effective in inhibiting neointimal hyperplasia, and thus can be used to treat or prevent peripheral artery disease (PAD).

**Example 9: Bioconjugate/peptide aggregate formation**

Hep-SILY was synthesized as described in Example 1 with the following modification during the purification procedure. Prior to purifying in 5 CVs of reaction buffer, the reaction was diluted into water, such that the chaotropic agent (e.g., urea) concentration was reduced to approximately 3 M. Subsequently, the reaction was purified into 16 CVs of water.

The resulting product was evaluated by size-exclusion chromatography, demonstrating the formation of high molecular weight species, indicating aggregate formation. The performance of the Hep-SILY complex was compared to reference Hep-SILY not containing ionically interacting SILY peptide. Hep-SILY containing aggregates bind collagen with a higher affinity (lower $EC_{50}$ values) compared to Hep-SILY that does not contain aggregates.

**Example 10. Vein grafts treated with Bioconjugate**

The method below can be used to confirm that vein grafts treated with a bioconjugate as described herein can result in reduced vein graft failure when the grafts are used in a bypass surgery. The example will also test for conditions for preparing the vein grafts.

*A. Optimize the concentration of bioconjugate*

Arteries from *ex vivo* studies on excised rabbit blood vessel tissue will be performed to optimize the drug substance concentration and soak duration prior to starting animal model studies. Information from the *ex vivo* binding studies will be used to define the soak time, drug substance concentration and formulation buffer to generate a vein graft preservation solution.

First, bioconjugate binding to veins will be quantified to examine effect of bioconjugate concentration and soak time. Excised veins from one rabbit will be cut into approximately 1$cm^2$ segments and placed in a 24-well plate. Varying concentrations of bioconjugate in buffered saline and varying times of treatment will be tested. Tissue pieces will be homogenized in extraction medium containing detergents and centrifuged to pellet debris. The supernatant will be assayed for protein by bicinchoninic acid assay (BCA) reagent and for drug substance by ELISA or ECL (electrochemiluminescent technology by MSD, Meso Scale Discovery).

Additionally, to determine how extensive vessel wall damage can enhance the binding, a second set of experiments will be included in which the vessels are scraped gently with a rubber policeman before cutting them into pieces. This procedure will simulate the process in which surgeons remove valves from the vein prior to implantation.

In addition to quantification of bioconjugate as described herein, immunohistochemistry (IHC) will be performed to confirm that the drug substance binds to the lumen of the vein. Two conditions (concentration and soak time) will be chosen based on the above experiments for testing. The jugular veins will be excised from a rabbit and flushed and soaked with bioconjugate solution. The veins will then be rinsed in 3 changes of buffered saline. The tissue will be cut into 3 segments. One segment will be fixed in neutral buffered formalin (NBF), a second segment will be cryopreserved in optimal cutting temperature (OCT) and a third will be snap frozen. Tissue sections from cryostat or formalin-fixed, paraffin-embedded (FFPE) specimens will be stained with H&E and immunostained using antibody specific for drug substance that has already been prepared. While the bioconjugate will bind to any exposed collagen on the vessel, this example expects to see the drug substance coating the inner surface of the blood vessel.

Further, the IHC procedure will be performed using human cadaver vein to ensure translation of procedures to human tissue. Vein tissue will be obtained from cadavers within 24 hours of death.

*B. Evaluate the bioconjugate solution in vein bypass animal procedure*

A vein graft model will be performed in male New Zealand White rabbits. The vein bypass graft will be constructed with an anastomotic cuff technique. The external jugular vein will be harvested and placed in a solution of either heparinized saline or a vein graft preservation solution at the appropriate time and concentration of bioconjugate. Following the storage period, the vein ends will be passed through a polyurethane cuff fashioned from a 4F vascular introducer sheath and then everted over the outside of the cuffs and secured with sutures. The carotid artery lumen will then be exposed with a 1-2 cm arteriotomy. The cuffed and reversed vein ends will be inserted into the carotid arterial lumen and the artery will be secured around the cuff with sutures. Once flow is restored, the interposed segment of the artery will be completely divided to allow full vein graft extension. Graft patency will be confirmed by visualization of pulsatile flow within the graft.

A total of 20 animals will be used. Ten animals will have the vein soaked in heparinized saline prior to grafting into the carotid, and ten animals will have the vein soaked in the vein graft preservation solution. The animals will be survived for 28 days. Heparinized saline is chosen as the control arm because it is commonly used in a clinical setting.

At day 28, anesthesia will be induced and the patency of the vessel will be confirmed. An intravenous heparin bolus will be given, and animals will be sacrificed. Vein grafts will undergo in situ perfusion fixation from the ascending aorta with 10% neutral-buffered formalin (NBF). The vein graft will then be excised and submersion fixed in NBF, prior to paraffin embedding for sectioning and morphometry. At least three different sections of the vein graft will be analyzed along the length of the vein graft, avoiding the tissue immediately adjacent to the foreign body cuffs.

Paraffin embedded 6 $\mu$m sections will be stained with Movat's pentachrome stain and imaged with an Aperio microscope. The circumference of the lumen, internal elastic lamina (IEL) and external elastic lamina (EEL) will be outlined and the areas within each perimeter will be calculated. The neointimal area will be calculated as the IEL area - Lumen area.

In each group there are 10 vein grafts, and a minimum of three areas will be examined within each graft. The three measurements within each graft will be averaged to give a mean neointimal area value per graft. The sample size

of 10 results in sufficient power (0.8, $\alpha$ = 0.05) for detecting a 25% reduction in neointimal hyperplasia with 20% standard deviation in the measurement. The criteria for success in this aim include a 25% reduction in neointimal hyperplasia in vein grafts treated with bioconjugate.

*C. Liquid stability study.*

The design and synthesis of the bioconjugate compound is in the process of being developed with well-established controls. A stability program will be completed to determine the stability of bioconjugate stored as a liquid at room temperature and at 4°C. The bioconjugate solution has previously been stored in a lyophilized form, and reconstituted prior to use. Such a solution was found to be stable for 3 months (time tested to date). Thus, a formal stability protocol for bioconjugate stored as a liquid at 4°C and at room temperature will be initiated. A liquid formulation would be convenient form of bioconjugate for clinical delivery in this setting.

*D. Toxicity studies in rats*

The dose range finding studies will be performed. First, rats will be given a single IV injection at four dose levels to ascertain acute toxicity after two days. Satellite groups will be similarly dosed and blood samples taken at time intervals to determine pharmacokinetic parameters. Next, a 7-day repeat dose study will be performed in a non-GLP setting in rats with no recovery period. The results from this study will be used to choose a highest tolerable dose level. Finally, a chronic 28-day repeat dose study will be performed with a 28-day recovery period using the highest tolerable dose level.

Endpoints for the studies will include mortality, clinical observations, body weights prior to dosing and at necropsy, food consumption prior to dosing and at necropsy, toxicokinetic observations from blood collection and clinical pathology. Standard histopathology will be performed on standard organs.

It is anticipated that no toxicity will be observed. It is expected that solubility limits will be reached prior to finding any toxic effects.

**Example 11. Stability comparison of oxDS-SILY vs. eDS-SILY**

Stability studies were conducted to compare stability of DS-SILY synthesized by oxidation chemistry (i.e., ring-opening; oxDS-SILY) or according to Example 1, above, using dermatan sulfate in place of heparin (i.e., non-ring-opening; eDS-SII,Y).

Briefly, oxDS-SILY was prepared as follows. Dermatan sulfate (DS) was dissolved in 0.1 M sodium phosphate buffer at pH 5.5 to make a solution of a concentration of 20 mg/mL. The degree of functionalization is controlled by the concentration of the periodate. Periodate solutions of various concentrations were prepared by dissolving it in 0.1 M sodium phosphate buffer at pH 5.5 according to the following table.

| Target (SILY/DS) | Peridodate Concentration (mg/mL) |
|---|---|
| 20 | 2.3 |

The DS solution was mixed with the periodate solution in a ratio of 1:1 (V:V) for two hours at room temperature to provide the oxidized DS, which was purified using Biogel P6 column with phosphate buffer saline. SILY peptide having a terminal GSG-$NHNH_2$ bound thereto (i.e., RRANAALKAGELYKSILYGSG-$NHNH_2$ (SEQ ID NO: 397)) was dissolved in water to provide a concentration of 1 mg/mL using sonication if needed. The SILY peptide was slowly added to the oxidized DS at room temperature and stirred for about 2 hours protecting it from light. The pH of the reaction mixture was maintained above 6. Optionally, one mole of similarly functionalized $SILY_{biotin}$ (biotin-labeled peptide) can be reacted with one mole of DS and then unlabeled SILY peptide can be added up (molar equivalent -1) to the number of aldehydes expected. DS-$SILY_{20}$ was also prepared by adding 20 moles of SII,Y-unlabeled to one mole of DS. The product was purified with water to provide the desired DS-SIIY.

Samples were synthesized and stored frozen for up to 8 weeks. HPLC-SEC was measured at time 0, 4 weeks, and 8 weeks. Peak areas were compared, where a decrease in peak area indicates degradation.

Results indicate that eDS-SILY remains stable over the 8-week test period, whereas oxDS-SILY degrades over the time period. Main peak and high molecular weight related peak (solid bars and shaded bars, respectively) decrease over 8-weeks with oxDS-SILY, whereas eDS-SILY values remain relatively constant and do not decrease over time (Figure 2).

**Example 12. Clinical Trial Protocol for Treating Gastro-Esophageal Injury**

This example proposes a clinical trial to test the ability of bioconjugates selected from those described herein, such as Hep-SILY, other heparin-containing bioconjugates including the branched peptides as discussed above, or DS-SILY in treating gastro-esophageal injuries. The text agent will be bioconjugates prepared in a solution or gel. Gastro-esophageal injuries constitute significant morbidity and costs to the patient and healthcare system. Maintaining esophageal patency consumes significant resources.

The disease target is any gastro-esophageal injury, either spontaneous from GERD or iatrogenic from interventions. This is a randomized, multi-center, safety and effectiveness study of topical bioconjugates administered via esophagogastroduodenoscopy (EGD) for treatment of gastro-esophageal injuries.

The objectives of this trial include, 1) to assess the overall safety profile of EGD-delivered bioconjugate treatment dosed at the time of EGD intervention, and 2) to determine the effectiveness of EGD-delivered bioconjugate in reducing restenosis rates in gastro-esophageal injuries.

Fifty (50) patients will be enrolled for the trial, 25 of which will receive bioconjugate (EGD-delivered) and 25 vehicle (EGD-delivered).

The key inclusion criteria include (a) GERD associated esophageal lesion requiring EGD ablation, (b) esophageal stricture requiring EGD dilation, and (c) peptic ulcer disease (PUD) requiring EGD treatment. The key exclusion criteria include H/O severe allergic diseases.

The visit schedule will be one, six, and 12 weeks. The safety assessment include (1) ascertainment of adverse events (AEs) and Serious AEs (SAEs), (2) physical examination/vitals: with special attention given to local findings, and (3) labs including bioconjugate antibodies (baseline, week 1, 12 and 24). The EGD delivery can be repeated at 6 and 12 weeks.

To measure the patency of the esophagus, quantitative barium swallow will be conducted pre-treatment and at end of the study.

The primary endpoint of this trial is reduced rate of recurrent stricture, and the secondary endpoints are time to advancement of oral diet, time to recurrent stricture and improved PUD score or symptoms. It is contemplated that the trial will succeed on all of these endpoints.

## Claims

1. A bioconjugate comprising a glycan selected from the group consisting of chondroitin sulfate, dermatan sulfate, and heparin, or a derivative thereof and at least one peptide(s), wherein the peptide(s) comprises a collagen-binding unit or a hyaluronic acid-binding unit, and are bound to the glycan via a hydrazide-carbonyl linkage, and wherein the glycan does not contain oxidatively cleaved saccharide units.

2. The bioconjugate of claim 1, wherein the glycan is chondroitin sulfate, dermatan sulfate or heparin.

3. The bioconjugate of any preceding claim, wherein the peptide(s) comprise up to about 25 amino acids.

4. The bioconjugate of any preceding claim, comprising from 1 to about 25 peptide(s), or from about 5 to about 25 peptides, or from about 11 to about 14 peptides, or from 1 to about 10 peptide(s), or about 7-8 peptides.

5. The bioconjugate of any preceding claim, wherein the glycan comprises from about 5 to about 30 percent (%) functionalization, or from about 10 to about 40 percent (%) functionalization, or about 25 percent (%) functionalization, or about 30 percent (%) functionalization.

6. The bioconjugate of any one of claims 1 or 3-5, wherein the glycan is a derivatized glycan, optionally wherein the derivatized glycan is a partially N-desulfated derivative, partially O-desulfated derivative, partially O-carboxymethylated derivative, or any combination thereof.

7. The bioconjugate of claim 1 or claim 2, wherein the peptide(s) comprise:

   i) an amino acid sequence selected from the group consisting of YKSILY (SEQ ID NO: 179), LYKSILY (SEQ ID NO: 180), ELYKSILY (SEQ ID NO: 181), GELYKSILY (SEQ ID NO: 2), AGELYKSILY (SEQ ID NO: 182), KAGELYKSILY (SEQ ID NO: 183), LKAGELYKSILY (SEQ ID NO: 184), ALKAGELYKSILY (SEQ ID NO: 185), AALKAGELYKSILY (SEQ ID NO: 186), NAALKAGELYKSILY (SEQ ID NO: 187), ANAALKAGELYKSILY (SEQ ID NO: 188), RANAALKAGELYKSILY (SEQ ID NO: 189), RRANAALKAGELYKSILY (SEQ ID NO: 1), QLYKSILY

(SEQ ID NO: 190), GQLYKSILY (SEQ ID NO: 16), AGQLYKSILY (SEQ ID NO: 191), KAGQLYKSILY (SEQ ID NO: 192), LKAGQLYKSILY (SEQ ID NO: 193), ALKAGQLYKSILY (SEQ ID NO: 194), AALKAGQLYKSILY (SEQ ID NO: 195), NAALKAGQLYKSILY (SEQ ID NO: 196), ANAALKAGQLYKSILY (SEQ ID NO: 197), RANAALKAGQLYKSILY (SEQ ID NO: 198), and RRANAALKAGQLYKSILY (SEQ ID NO: 17), or a sequence having at least about 80% sequence identity thereto, provided that the sequence comprises at least one YKS sequence;

ii) an amino acid sequence selected from the group consisting of YKCILY (SEQ ID NO: 199), LYKCILY (SEQ ID NO: 200), ELYKCILY (SEQ ID NO: 201), GELYKCILY (SEQ ID NO: 4), AGELYKCILY (SEQ ID NO: 202), KAGELYKCILY (SEQ ID NO: 203), LKAGELYKCILY (SEQ ID NO: 204), ALKAGELYKCILY (SEQ ID NO: 205), AALKAGELYKCILY (SEQ ID NO: 206), NAALKAGELYKCILY (SEQ ID NO: 207), ANAALKAGELYKCILY (SEQ ID NO: 208), RANAALKAGELYKCILY (SEQ ID NO: 209), RRANAALKAGELYKCILY (SEQ ID NO: 3), QLYKCILY (SEQ ID NO: 210), GQLYKCILY (SEQ ID NO: 211), AGQLYKCILY (SEQ ID NO: 212), KAGQLYKCILY (SEQ ID NO: 213), LKAGQLYKCILY (SEQ ID NO: 214), ALKAGQLYKCILY (SEQ ID NO: 215), AALKAGQLYKCILY (SEQ ID NO: 216), NAALKAGQLYKCILY (SEQ ID NO: 217), ANAALKAGQLYKCILY (SEQ ID NO: 218), RANAALKAGQLYKCILY (SEQ ID NO: 219), and RRANAALKAGQLYKCILY (SEQ ID NO: 220), or a sequence having at least about 80% sequence identity thereto, provided that the sequence comprises at least one YKS sequence;

iii) the amino acid sequence GAHWQFNALTVR (SEQ ID NO: 58), or a sequence having at least about 80% sequence identity thereto, provided that the sequence is capable of binding to hyaluronic acid;

iv) the amino acid sequence STMMSRSHKTRSHHV (SEQ ID NO: 59), or a sequence having at least about 80% sequence identity thereto, provided that the sequence is capable of binding to hyaluronic acid;

v) at least one sequence of GAHWQFNALTVR (SEQ ID NO: 58) or GAHWQFNALTVRGSG (SEQ ID NO: 357) or a sequence having at least about 80% sequence identity thereto, provided that the sequence is capable of binding to hyaluronic acid, and at least one sequence of WYRGRL (SEQ ID NO: 29) or WYRGRLGSG (SEQ ID NO: 392) or a sequence having at least about 80% sequence identity thereto, provided that the sequence is capable of binding to collagen; or

vi) at least one sequence of GAHWQFNALTVR (SEQ ID NO: 58) or GAHWQFNALTVRGSG (SEQ ID NO: 357) or a sequence having at least about 80% sequence identity thereto, provided that the sequence is capable of binding to hyaluronic acid, and at least one sequence of RRANAALKAGELYKSILY (SEQ ID NO: 1) or RRANAALKAGELYKSILYGSG (SEQ ID NO: 287) or a sequence having at least about 80% sequence identity thereto, provided that the sequence is capable of binding to collagen.

**8.** The bioconjugate of any preceding claim, wherein the hydrazide group is bonded to the peptide(s) C-terminus, optionally via a spacer.

**9.** The bioconjugate of any preceding claim, wherein the hydrazide group is bonded to the C-terminus via a spacer comprising one or more amino acids selected from the group consisting of glycine, alanine, arginine, lysine and serine, or a spacer selected from the group consisting of glycine, glycine-glycine, serine-glycine, lysine-arginine, arginine-arginine, and glycine-serine-glycine.

**10.** A composition comprising the bioconjugate of any preceding claim, wherein the average number of peptide(s) per glycan is:

i) less than about 30,
ii) from about 5 to about 30, or
iii) about 7.

**11.** A bioconjugate of any one of claims 1-9 or the composition of claim 10 for use in a method of treatment for arthritis in a patient, said method comprising of administering to the patient an effective amount of the bioconjugate or the composition.

**12.** The bioconjugate or composition for use of claim 11, wherein the arthritis is selected from the group consisting of osteoarthritis and rheumatoid arthritis.

**13.** A method for making a bioconjugate comprising a glycan selected from the group consisting of chondroitin sulfate, or a derivative thereof, dermatan sulfate, or a derivative thereof, and heparin, or a derivative thereof, and from 1 to 50 peptide(s), said method comprising contacting the glycan with a sufficient amount of peptide, optionally in the presence of an activating agent, wherein the peptide comprises a hydrazide group, under coupling reaction conditions to provide the bioconjugate, wherein the peptide(s) comprise a collagen-binding unit or a hyaluronic acid-binding unit and are bound to the glycan via a hydrazide-carbonyl linkage between a terminal hydrazide group on the peptides

and a carbonyl group on the glycan, and wherein the glycan does not contain oxidatively cleaved saccharide units.

**14.** The method of claim 13, wherein:

i) the bioconjugate comprises at least one sequence of YKS or YKC,
ii) the activating agent is a carbodiimide reagent,
iii) the activating agent is selected from the group consisting of N,N'-dicyclohexylcarbodiimide, N,N'-diisopropylcarbodiimide, and 1-ethyl-3-(3-dimethylaminopropyl)carbodiimide, or
iv) the hydrazide group is bonded to the peptide(s) C-terminus, optionally via a spacer.

**15.** The method of claim 14, wherein the spacer comprises one or more amino acids selected from the group consisting of glycine, alanine, arginine, lysine and serine, and/or a spacer selected from the group consisting of glycine, glycine-glycine, serine-glycine, arginine-arginine, lysine-arginine, lysine-arginine-arginine and glycine-serine-glycine.

## Patentansprüche

**1.** Biokonjugat, das ein Glykan, das aus der Gruppe ausgewählt ist, die aus Chondroitinsulfat, Dermatansulfat und Heparin oder einem Derivat davon besteht, und zumindest ein oder mehrere Peptide umfasst, wobei das eine oder die mehreren Peptide eine Kollagenbindungseinheit oder eine Hyaluronsäurebindungseinheit umfassen und über eine Hydrazid-Carbonyl-Verknüpfung an das Glykan gebunden sind, und wobei das Glykan keine oxidativ gespaltene Saccharideinheiten enthält.

**2.** Biokonjugat nach Anspruch 1, wobei das Glykan Chondroitinsulfat, Dermatansulfat oder Heparin ist.

**3.** Biokonjugat nach einem vorstehenden Anspruch, wobei das eine oder die mehreren Peptide bis zu ungefähr 25 Aminosäuren umfassen.

**4.** Biokonjugat nach einem vorstehenden Anspruch, das 1 bis ungefähr 25 Peptide oder ungefähr 5 bis ungefähr 25 Peptide oder ungefähr 11 bis ungefähr 14 Peptide oder 1 bis ungefähr 10 Peptide oder ungefähr 7 bis 8 Peptide umfasst.

**5.** Biokonjugat nach einem vorstehenden Anspruch, wobei das Glykan ungefähr 5 bis ungefähr 30 Prozent (%) Funktionalisierung oder ungefähr 10 bis ungefähr 40 Prozent (%) Funktionalisierung oder ungefähr 25 Prozent (%) Funktionalisierung oder ungefähr 30 Prozent (%) Funktionalisierung umfasst.

**6.** Biokonjugat nach einem der Ansprüche 1 oder 3 bis 5, wobei das Glykan ein derivatisiertes Glykan ist, wahlweise wobei das derivatisierte Glykan ein teilweise N-desulfatiertes Derivat, teilweise O-desulfatiertes Derivat, teilweise O-carboxymethyliertes Derivat oder eine beliebige Kombination davon ist.

**7.** Biokonjugat nach Anspruch 1 oder 2, wobei das eine oder die mehreren Peptide umfassen:

i) eine Aminosäuresequenz, die aus der Gruppe ausgewählt ist, die aus YKSILY (SEQ ID NO: 179), LYKSILY (SEQ ID NO: 180), EL YKSILY (SEQ ID NO: 181), GEL YKSILY (SEQ ID NO: 2), AGELYKSILY (SEQ ID NO: 182), KAGELYKSILY (SEQ ID NO: 183), LKAGELYKSILY (SEQ ID NO: 184), ALKAGELYKSILY (SEQ ID NO: 185), AALKAGELYKSILY (SEQ ID NO: 186), NAALKAGELYKSILY (SEQ ID NO: 187), ANAALKAGELYKSILY (SEQ ID NO: 188), RANAALKA GELYKSILY (SEQ ID NO: 189), RRANAALKAGELYKSILY (SEQ ID NO: 1), QLYKSILY (SEQ ID NO: 190), GQLYKSILY (SEQ ID NO: 16), AGQLYKSILY (SEQ ID NO: 191), KAGQLYKSILY (SEQ ID NO: 192), LKAGQLYKSILY (SEQ ID NO: 193), ALKAGQLYKSILY (SEQ ID NO: 194), AALKAGQLYKSILY (SEQ ID NO: 195), NAALKAGQLYKSILY (SEQ ID NO: 196), ANAALKAGQLYKSILY (SEQ ID NO: 197), RANAALKAGQLYKSILY (SEQ ID NO: 198) und RRANAALKAGQLYKSILY (SEQ ID NO: 17) besteht, oder eine Sequenz mit einer Sequenzidentität dazu von zumindest ungefähr 80 %, mit der Maßgabe, dass die Sequenz zumindest eine YKS-Sequenz umfasst;
ii) eine Aminosäuresequenz, die aus der Gruppe ausgewählt ist, die aus YKCILY (SEQ ID NO: 199), LYKCILY (SEQ ID NO: 200), ELYKCILY (SEQ ID NO: 201), GELYKCILY (SEQ ID NO: 4), AGELYKCILY (SEQ ID NO: 202), KAGELYKCILY (SEQ ID NO: 203), LKAGELYKCILY (SEQ ID NO: 204), ALKAGELYKCILY (SEQ ID NO: 205), AALKAGELYKCILY (SEQ ID NO: 206), NAALKAGELYKCILY (SEQ ID NO: 207), ANAALKAGELYKCILY (SEQ ID NO: 208), RANAALKAGELYKCILY (SEQ ID NO: 209), RRANAALKAGELYKCILY (SEQ ID NO: 3),

QLYKCILY (SEQ ID NO: 210), GQLYKCILY (SEQ ID NO: 211), AGQLYKCILY (SEQ ID NO: 212), KAGQLYKCILY (SEQ ID NO: 213), LKAGQLYKCILY (SEQ ID NO: 214), ALKAGQLYKCILY (SEQ ID NO: 215), AALKAGQLYKCILY (SEQ ID NO: 216), NAALKAGQLYKCILY (SEQ ID NO: 217), ANAALKAGQLYKCILY (SEQ ID NO: 218), RANAALKAGQLYKCILY (SEQ ID NO: 219) und RRANAALKAGQLYKCILY (SEQ ID NO: 220) besteht, oder eine Sequenz mit einer Sequenzidentität dazu von zumindest ungefähr 80 %, mit der Maßgabe, dass die Sequenz zumindest eine YKS-Sequenz umfasst;

iii) die Aminosäuresequenz GAHWQFNALTVR (SEQ ID NO: 58) oder eine Sequenz mit einer Sequenzidentität dazu von zumindest ungefähr 80 %, mit der Maßgabe, dass die Sequenz zur Bindung an Hyaluronsäure in der Lage ist;

iv) die Aminosäuresequenz STMMSRSHKTRSHHV (SEQ ID NO: 59) oder eine Sequenz mit einer Sequenzidentität dazu von zumindest ungefähr 80 %, mit der Maßgabe, dass die Sequenz zur Bindung an Hyaluronsäure in der Lage ist;

v) zumindest eine Sequenz von GAHWQFNALTVR (SEQ ID NO: 58) oder GAHWQFNALTVRGSG (SEQ ID NO: 357) oder eine Sequenz mit einer Sequenzidentität dazu von zumindest ungefähr 80 %, mit der Maßgabe, dass die Sequenz zur Bindung an Hyaluronsäure in der Lage ist, und zumindest eine Sequenz von WYRGRL (SEQ ID NO: 29) oder WYRGRLGSG (SEQ ID NO: 392) oder eine Sequenz mit einer Sequenzidentität dazu von zumindest ungefähr 80 %, mit der Maßgabe, dass die Sequenz zur Bindung an Kollagen in der Lage ist; oder

vi) zumindest eine Sequenz von GAHWQFNALTVR (SEQ ID NO: 58) oder GAHWQFNALTVRGSG (SEQ ID NO: 357) oder eine Sequenz mit einer Sequenzidentität dazu von zumindest ungefähr 80 %, mit der Maßgabe, dass die Sequenz zur Bindung an Hyaluronsäure in der Lage ist, und zumindest eine Sequenz von RRANAALKAGELYKSILY (SEQ ID NO: 1) oder RRANAALKAGELYKSILYGSG (SEQ ID NO: 287) oder eine Sequenz mit einer Sequenzidentität dazu von zumindest ungefähr 80 %, mit der Maßgabe, dass die Sequenz zur Bindung an Kollagen in der Lage ist.

**8.** Biokonjugat nach einem vorstehenden Anspruch, wobei die Hydrazidgruppe an den C-Terminus des einen oder der mehreren Peptide gebunden ist, wahlweise über einen Abstandshalter.

**9.** Biokonjugat nach einem vorstehenden Anspruch, wobei die Hydrazidgruppe über einen Abstandshalter, der eine oder mehrere Aminosäuren umfasst, die aus der Gruppe ausgewählt sind, die aus Glycin, Alanin, Arginin, Lysin und Serin besteht, oder über einen Abstandshalter, der aus der Gruppe ausgewählt ist, die aus Glycin, Glycin-Glycin, Serin-Glycin, Lysin-Arginin, Arginin-Arginin und Glycin-Serin-Glycin besteht, an den C-Terminus gebunden ist.

**10.** Zusammensetzung, die das Biokonjugat nach einem vorstehenden Anspruch umfasst, wobei die durchschnittliche Anzahl von Peptid(en) pro Glykan ist:

i) weniger als ungefähr 30,
ii) ungefähr 5 bis ungefähr 30 oder
iii) ungefähr 7.

**11.** Biokonjugat nach einem der Ansprüche 1 bis 9 oder Zusammensetzung nach Anspruch 10 zur Verwendung in einem Verfahren zum Behandeln von Arthritis bei einem Patienten, wobei das Verfahren das Verabreichen einer wirksamen Menge des Biokonjugats oder der Zusammensetzung an den Patienten umfasst.

**12.** Biokonjugat oder Zusammensetzung zur Verwendung nach Anspruch 11, wobei die Arthritis aus der Gruppe ausgewählt ist, die aus Osteoarthrose und rheumatoider Arthritis besteht.

**13.** Verfahren zum Herstellen eines Biokonjugats, das ein Glykan, das aus der Gruppe ausgewählt ist, die aus Chondroitinsulfat oder einem Derivat davon, Dermatansulfat oder einem Derivat davon und Heparin oder einem Derivat davon besteht, und 1 bis 50 Peptide umfasst, wobei das Verfahren das Inkontaktbringen des Glykans mit einer ausreichenden Menge an Peptid, wahlweise in Gegenwart eines Aktivators, wobei das Peptid eine Hydrazidgruppe umfasst, unter Kupplungsreaktionsbedingungen umfasst, um das Biokonjugat bereitzustellen, wobei das eine oder die mehreren Peptide eine Kollagenbindungseinheit oder eine Hyaluronsäurebindungseinheit umfassen und über eine Hydrazid-Carbonyl-Verknüpfung zwischen einer terminalen Hydrazidgruppe an den Peptiden und einer Carbonylgruppe an dem Glykan an das Glykan gebunden sind, und wobei das Glykan keine oxidativ gespaltene Saccharideinheiten enthält.

**14.** Verfahren nach Anspruch 13, wobei:

i) das Biokonjugat zumindest eine Sequenz von YKS oder YKC umfasst,
ii) der Aktivator ein Carbodiimidreagens ist,
iii) der Aktivator aus der Gruppe ausgewählt ist, die aus N,N'-Dicyclohexylcarbodiimid, N,N'-Diisopropylcarbodiimid und 1-Ethyl-3-(3-dimethylaminopropyl)carbodiimid besteht, oder
iv) die Hydrazidgruppe an den C-Terminus des einen oder der mehreren Peptide gebunden ist, wahlweise über einen Abstandshalter.

**15.** Verfahren nach Anspruch 14, wobei der Abstandshalter eine oder mehrere Aminosäuren umfasst, die aus der Gruppe ausgewählt sind, die aus Glycin, Alanin, Arginin, Lysin und Serin besteht, und/oder einen Abstandshalter, der aus der Gruppe ausgewählt ist, die aus Glycin, Glycin-Glycin, Serin-Glycin, Arginin-Arginin, Lysin-Arginin, Lysin-Arginin-Arginin und Glycin-Serin-Glycin besteht.

## Revendications

**1.** Bioconjugué comprenant un glycane choisi dans le groupe consistant en sulfate de chondroïtine, sulfate de dermatane et héparine, ou un de leurs dérivés, et au moins un peptide, dans lequel le ou les peptides comprennent une unité de liaison au collagène ou une unité de liaison à l'acide hyaluronique, et sont liés au glycane via une liaison hydrazide-carbonyle, et dans lequel le glycane ne contient pas d'unités saccharidiques clivées par oxydation.

**2.** Bioconjugué selon la revendication 1, dans lequel le glycane est le sulfate de chondroïtine, le sulfate de dermatane ou l'héparine.

**3.** Bioconjugué selon une quelconque revendication précédente, dans lequel le ou les peptides comprennent jusqu'à environ 25 acides aminés.

**4.** Bioconjugué selon une quelconque revendication précédente, comprenant 1 à environ 25 peptides, ou environ 5 à environ 25 peptides, ou environ 11 à environ 14 peptides, ou 1 à environ 10 peptides, ou environ 7 à 8 peptides.

**5.** Bioconjugué selon une quelconque revendication précédente, dans lequel le glycane comprend environ 5 à environ 30 pour cent (%) de fonctionnalisation, ou environ 10 à environ 40 pour cent (%) de fonctionnalisation, ou environ 25 pour cent (%) de fonctionnalisation, ou environ 30 pour cent (%) de fonctionnalisation.

**6.** Bioconjugué selon l'une quelconque des revendications 1 ou 3 à 5, dans lequel le glycane est un glycane dérivé, facultativement dans lequel le glycane dérivé est un dérivé partiellement N-désulfaté, un dérivé partiellement O-désulfaté, un dérivé partiellement O-carboxyméthylé, ou l'une quelconque de leurs combinaisons.

**7.** Bioconjugué selon la revendication 1 ou la revendication 2, dans lequel le ou les peptides comprennent :

i) une séquence d'acides aminés choisie dans le groupe consistant en YKSILY (SEQ ID N° : 179), LYKSILY (SEQ ID N° : 180), ELYKSILY (SEQ ID N° : 181), GELYKSILY (SEQ ID N° : 2), AGELYKSILY (SEQ ID N° : 182), KAGELYKSILY (SEQ ID N° : 183), LKAGELYKSILY (SEQ ID N° : 184), ALKAGELYKSILY (SEQ ID N° : 185), AALKAGELYKSILY (SEQ ID N° : 186), NAALKAGELYKSILY (SEQ ID N° : 187), ANAALKAGELYKSILY (SEQ ID N° : 188), RANAALKAGELYKSILY (SEQ ID N° : 189), RRANAALKAGELYKSILY (SEQ ID N° : 1), QLYKSILY (SEQ ID N° : 190), GQLYKSILY (SEQ ID N° : 16), AGQLYKSILY (SEQ ID N° : 191), KAGQLYKSILY (SEQ ID N° : 192), LKAGQLYKSILY (SEQ ID N° : 193), ALKAGQLYKSILY (SEQ ID N° : 194), AALKAGQLYKSILY (SEQ ID N° : 195), NAALKAGQLYKSILY (SEQ ID N° : 196), ANAALKAGQLYKSILY (SEQ ID N° : 197), RANAALKAGQLYKSILY (SEQ ID N° : 198), et RRANAALKAGQLYKSILY (SEQ ID N° : 17), ou une séquence ayant au moins environ 80 % d'identité de séquence avec celle-ci, à condition que la séquence comprenne au moins une séquence YKS ;
ii) une séquence d'acides aminés choisie dans le groupe consistant en YKCILY (SEQ ID N° : 199), LYKCILY (SEQ ID N° : 200), ELYKCILY (SEQ ID N° : 201), GELYKCILY (SEQ ID N° : 4), AGELYKCILY (SEQ ID N° : 202), KAGELYKCILY (SEQ ID N° : 203), LKAGELYKCILY (SEQ ID N° : 204), ALKAGELYKCILY (SEQ ID N° : 205), AALKAGELYKCILY (SEQ ID N° : 206), NAALKAGELYKCILY (SEQ ID N° : 207), ANAALKAGELYKCILY (SEQ ID N° : 208), RANAALKAGELYKCILY (SEQ ID N° : 209), RRANAALKAGELYKCILY (SEQ ID N° : 3), QLYKCILY (SEQ ID N° : 210), GQLYKCILY (SEQ ID N° : 211), AGQLYKCILY (SEQ ID N° : 212), KAGQLYKCILY (SEQ ID N° : 213), LKAGQLYKCILY (SEQ ID N° : 214), ALKAGQLYKCILY (SEQ ID N° : 215), AALKAGQLYKCILY (SEQ ID N° : 216), NAALKAGQLYKCILY (SEQ ID N° : 217), ANAALKAGQLYKCILY (SEQ ID N° : 218),

RANAALKAGQLYKCILY (SEQ ID N° : 219), et RRANAALKAGQLYKCILY (SEQ ID N° : 220), ou une séquence ayant au moins environ 80 % d'identité de séquence avec celle-ci, à condition que la séquence comprenne au moins une séquence YKS ;
iii) la séquence d'acides aminés GAHWQFNALTVR (SEQ ID N° : 58), ou une séquence ayant au moins environ 80 % d'identité de séquence avec celle-ci, à condition que la séquence soit capable de se lier à l'acide hyaluronique ;
iv) la séquence d'acides aminés STMMSRSHKTRSHHV (SEQ ID N° : 59), ou une séquence ayant au moins environ 80 % d'identité de séquence avec celle-ci, à condition que la séquence soit capable de se lier à l'acide hyaluronique ;
v) au moins une séquence GAHWQFNALTVR (SEQ ID N° : 58) ou GAHWQFNALTVRGSG (SEQ ID N° : 357), ou une séquence ayant au moins environ 80 % d'identité de séquence avec celle-ci, à condition que la séquence soit capable de se lier à l'acide hyaluronique, et au moins une séquence WYRGRL (SEQ ID N° : 29) ou WYRGRLGSG (SEQ ID N° : 392), ou une séquence ayant au moins environ 80 % d'identité de séquence avec celle-ci, à condition que la séquence soit capable de se lier au collagène ; ou
vi) au moins une séquence GAHWQFNALTVR (SEQ ID N° : 58) ou GAHWQFNALTVRGSG (SEQ ID N° : 357), ou une séquence ayant au moins environ 80 % d'identité de séquence avec celle-ci, à condition que la séquence soit capable de se lier à l'acide hyaluronique, et au moins une séquence RRANAALKAGELYKSILY (SEQ ID N° : 1) ou RRANAALKAGELYKSILYGSG (SEQ ID N° : 287), ou une séquence ayant au moins environ 80 % d'identité de séquence avec celle-ci, à condition que la séquence soit capable de se lier au collagène.

**8.** Bioconjugué selon une quelconque revendication précédente, dans lequel le groupe hydrazide est lié à l'extrémité C-terminale du ou des peptides, facultativement via un espaceur.

**9.** Bioconjugué selon une quelconque revendication précédente, dans lequel le groupe hydrazide est lié à l'extrémité C-terminale via un espaceur comprenant un ou plusieurs acides aminés choisis dans le groupe consistant en glycine, alanine, arginine, lysine et sérine, ou un espaceur choisi dans le groupe consistant en glycine, glycine-glycine, sérine-glycine, lysine-arginine, arginine-arginine et glycine-sérine-glycine.

**10.** Composition comprenant le bioconjugué selon une quelconque revendication précédente, dans laquelle le nombre moyen de peptide(s) par glycane est :

i) inférieur à environ 30,
ii) d'environ 5 à environ 30, ou
iii) d'environ 7.

**11.** Bioconjugué selon l'une quelconque des revendications 1 à 9 ou composition selon la revendication 10 pour une utilisation dans un procédé de traitement de l'arthrite chez un patient, ledit procédé comprenant l'administration au patient d'une quantité efficace du bioconjugué ou de la composition.

**12.** Bioconjugué ou composition pour une utilisation selon la revendication 11, l'arthrite étant choisie dans le groupe consistant en l'arthrose et la polyarthrite rhumatoïde.

**13.** Procédé de préparation d'un bioconjugué comprenant un glycane choisi dans le groupe consistant en sulfate de chondroïtine, ou un de ses dérivés, sulfate de dermatane, ou un de ses dérivés, et héparine, ou un de ses dérivés, et 1 à 50 peptide(s), ledit procédé comprenant la mise en contact du glycane avec une quantité suffisante de peptide, facultativement en présence d'un agent activateur, dans lequel le peptide comprend un groupe hydrazide, dans des conditions de réaction de couplage pour fournir le bioconjugué, dans lequel le ou les peptides comprennent une unité de liaison au collagène ou une unité de liaison à l'acide hyaluronique, et sont liés au glycane via une liaison hydrazide-carbonyle entre un groupe hydrazide terminal sur les peptides et un groupe carbonyle sur le glycane, et dans lequel le glycane ne contient pas d'unités saccharidiques clivées par oxydation.

**14.** Procédé selon la revendication 13, dans lequel :

i) le bioconjugué comprend au moins une séquence YKS ou YKC,
ii) l'agent activateur est un réactif carbodiimide,
iii) l'agent activateur est choisi dans le groupe consistant en N,N'-dicyclohexyl-carbodiimide, N,N'-diisopropylcarbodiimide, et 1-éthyl-3-(3-diméthylaminopropyl)carbodiimide, ou
iv) le groupe hydrazide est lié à l'extrémité C-terminale du ou des peptides, facultativement via un espaceur.

**15.** Procédé selon la revendication 14, dans lequel l'espaceur comprend un ou plusieurs acides aminés choisis dans le groupe consistant en glycine, alanine, arginine, lysine et sérine, et/ou un espaceur choisi dans le groupe consistant en glycine, glycine-glycine, sérine-glycine, arginine-arginine, lysine-arginine, lysine-arginine-arginine et glycine-sérine-glycine.

FIG. 1A

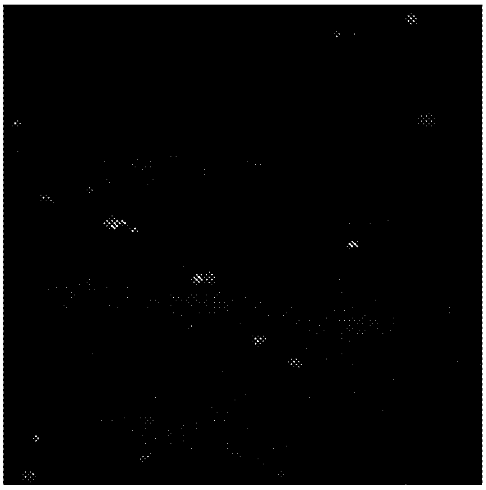

FIG. 1C

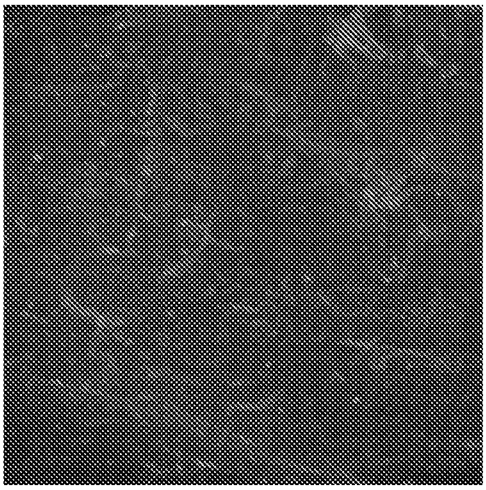

FIG. 1B

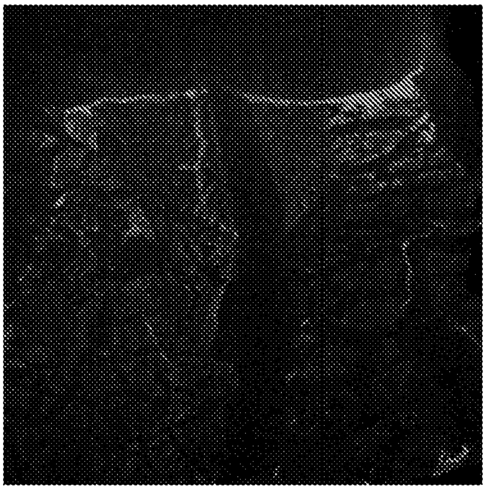

FIG. 1D

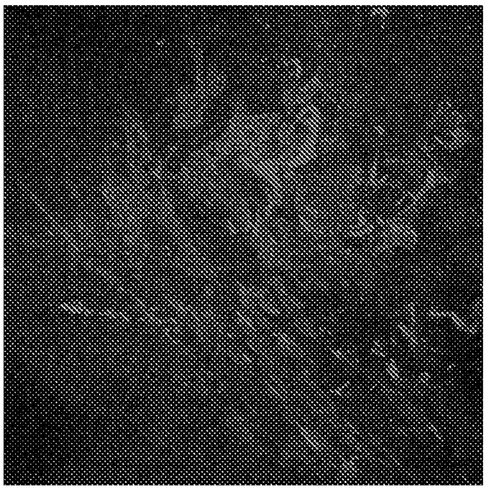

FIG. 2

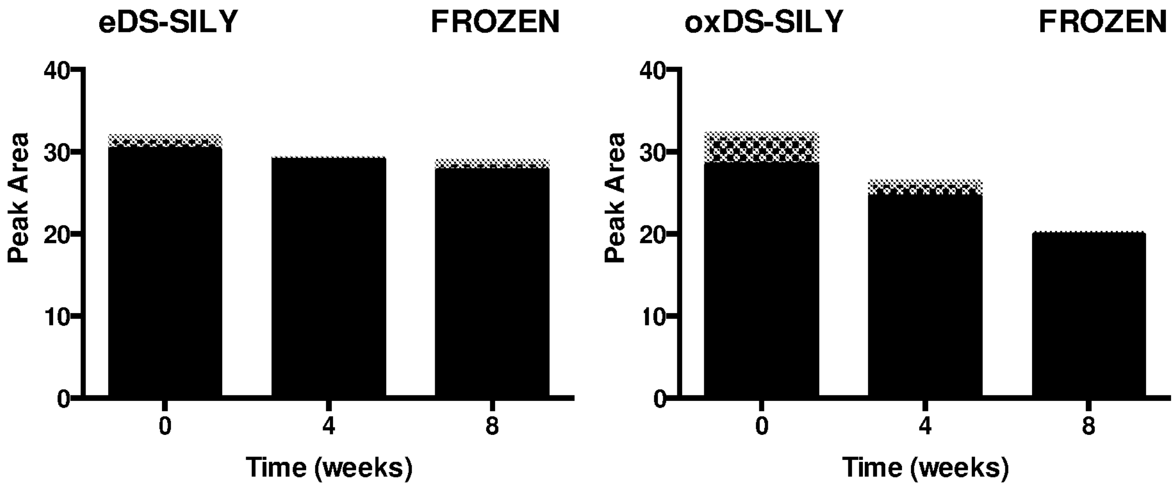
eDS-SILY
FROZEN
Peak Area
40
30
20
10
0
0
4
8
Time (weeks)
oxDS-SILY
FROZEN
Peak Area
40
30
20
10
0
0
4
8
Time (weeks)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014144969 A
- US 20070293656 A
- WO 2014059530 A
- US 20130243700 A
- US 20120142907 A, Prestwich,
- US 20100330143 A
- US 7300454 B
- US 5409904 A
- US 4861760 A
- US 4255415 A
- US 4271143 A
- WO 9410976 A
- WO 9951273 A
- WO 9906023 A
- US 20140301983 A


### Non-patent literature cited in the description


- **RADEK, K. A. et al.** *Wound Repair Regen.,* 2009, vol. 17, 118-126
- **TAYLOR, K. R. et al.** *J. Biol. Chem.,* 2005, vol. 280, 5300-5306
- **FRANSSON, L. A. et al.** *Eur. J. Biochem.,* 1980, vol. 106, 59-69
- **LI, Y. et al.** *Current Opinion in Chemical Biology,* 2013, vol. 17, 968-975
- **HELMES, B.A. et al.** *J. Am. Chem. Soc.,* 2009, vol. 131, 11683-11685
- **PETSALAKI, E. et al.** *PLoS Comput Biol,* 2009, vol. 5 (3), e1000335
- **CHIANG, T.M. et al.** *J. Biol. Chem.,* 2002, vol. 277, 34896-34901
- **HUIZINGA, E.G. et al.** *Structure,* 1997, vol. 5, 1147-1156
- **ROMIJN, R.A. et al.** *J. Biol. Chem.,* 2003, vol. 278, 15035-15039
- **CHIANG et al.** *Cardio. & Haemato. Disorders-Drug Targets,* 2007, vol. 7, 71-75
- **ABD-ELGALIEL, W.R. et al.** *Biopolymers,* 2013, vol. 100 (2), 167-173
- **RAYNAL, N. et al.** *J. Biol. Chem.,* 2006, vol. 281 (7), 3821-3831
- **HELMS, B.A. et al.** *J. Am. Chem. Soc.,* 2009, vol. 131, 11683-11685
- **TAKAGI, J. et al.** *Biochemistry,* 1992, vol. 31, 8530-8534
- **ROTHENFLUH D.A. et al.** *Nat Mater.,* 2008, vol. 7 (3), 248-54
- **DEPRAETERE H. et al.** *Blood.,* 1998, vol. 92, 4207-4211
- **DUNCAN R.** *Nat Rev Drug Discov,* 2003, vol. 2 (5), 347-360
- **VANHOORELBEKE, K. et al.** *J. Biol. Chem.,* 2003, vol. 278, 37815-37821
- **MUZZARD, J. et al.** *PLoS one.,* vol. 4 (5585), I- 10
- **CHAN, J. M. et al.** *Proc Natl Acad Sci U.S.A.,* 2010, vol. 107, 2213-2218
- **HELMS, B.A. et al.** *J. Am. Chem. Soc.,* 2009, vol. 131 (33), 11683-11685
- **FREDRICO, S.** *Angew. Chem. Int. Ed.,* 2015, vol. 37, 10980-10984
- **BECERRA, S.P. et al.** *J. Biol. Chem.,* 2008, vol. 283, 33310-33320
- **AMEMIYA et al.** *Biochem. Biophys. Acta,* 2005, vol. 1724, 94-99
- **YANG, B et al.** *EMBO Journal,* 1994, vol. 13, 286-296
- **GOETINCK, P.F. et al.** *J. Cell. Biol,* 1987, vol. 105, 2403-2408
- **KARIYA et al.** *J. Biol. Chem.,* 2000, vol. 275, 25949-5958
- **LAPIERRE et al.** *Glycobiology,* 1996, vol. 6 (3), 355-366
- **WANG et al.** *Biomacromolecules,* 2013, vol. 14 (7), 2427-2432
- **SAMBROOK et al.** Molecular Cloning: A Laboratory Manual. Cold Spring Harbor Laboratory Press, 2001
- **EL-FAHAM et al.** *Chem. Rev.,* 2011, vol. 111 (11), 6557-6602
- **HAN et al.** *Tetrahedron,* 2004, vol. 60, 2447-2467
- **RAJENDRAN et al.** *, Int. J. Biol. Sci.,* 2013, vol. 9, 1057-1069
- **DIAMOND, M. P. ; FREEMAN, M. L.** *Eur. Soc. Human. Repro. Embryo.,* 2001, vol. 7 (6), 567-576
- **CHIAPELLA, A. P. ; ROSENTHAL, A. R.** *British Journal of Ophthalmology,* 1985, vol. 69, 865-870

- **TUFT, S.J. et al.** *Br J Ophthalmol.,* 1993, vol. 77, 243-247
- **KENYON, K. R.** Cornea and Refractive Atlas of Clinical Wisdom. SLACK, Inc, 2011, 39